(19) Europäisches Patentamt
European Patent Office
Office européen des brevets

(11) **EP 2 277 595 A2**

(12) **EUROPEAN PATENT APPLICATION**

(43) Date of publication:
**26.01.2011 Bulletin 2011/04**

(21) Application number: **10179748.8**

(22) Date of filing: **24.06.2005**

(51) Int Cl.:
***A61P 37/04*** *(2006.01)*
***A61K 31/505*** *(2006.01)*
***A61K 31/52*** *(2006.01)*
***A61K 31/7068*** *(2006.01)*
***A61K 31/708*** *(2006.01)*

(84) Designated Contracting States:
**AT BE BG CH CY CZ DE DK EE ES FI FR GB GR HU IE IS IT LI LT LU MC NL PL PT RO SE SI SK TR**

(30) Priority:
**24.06.2004 US 582654 P**
**14.07.2004 US 588096 P**
**22.07.2004 US 590459 P**
**05.08.2004 US 599717 P**
**05.08.2004 US 599592 P**
**11.08.2004 US 600850 P**
**19.08.2004 US 603001 P**
**23.08.2004 US 603867 P**
**21.09.2004 US 612070 P**
**30.09.2004 US 614963 P**

(62) Document number(s) of the earlier application(s) in accordance with Art. 76 EPC:
**05791104.2 / 1 765 313**

(71) Applicant: **Novartis Vaccines and Diagnostics, Inc.**
**Emeryville, CA 94608-2916 (US)**

(72) Inventors:
- **Valiante, Nicholas**
  **Emeryville, CA 94608-2916 (US)**
- **Xu, Feng**
  **Emeryville, CA 94608-2916 (US)**
- **Silver, Joel B.**
  **Emeryville, CA 94608-2916 (US)**

(74) Representative: **Marshall, Cameron John**
**Carpmaels & Ransford**
**One Southampton Row**
**London**
**WC1B 5HA (GB)**

Remarks:
This application was filed on 25-09-2010 as a divisional application to the application mentioned under INID code 62.

# (54) Compounds for immunopotentiation

(57) Compounds of formula I:

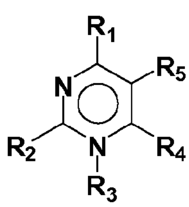

**I**

wherein $R_1$ to $R_5$ are defined herein, for use in modulating an immune response in a subject, wherein said use comprises administering said compound to said subject, are disclosed.

EP 2 277 595 A2

## Description

### FIELD OF THE INVENTION

This invention pertains generally to compounds capable of stimulating or modulating an immune response in a subject. In some embodiments, the compounds are 3,4-di(1H-indol-3-yl)-1H-pyrrole-2,5-diones, staurosporine analogs, derivatized pyridazines, chromen-4-ones, indolinones, quinazolines, nucleoside analogs, or other small molecules as described herein. In some further embodiments, the invention provides novel combinations of antigens with immune potentiators that may be used in vaccine therapies. The compounds in one embodiment can be used as immunotherapeutics for proliferative diseases, infectious diseases, autoimmune diseases and/or allergies/asthma.

### BACKGROUND OF THE INVENTION

With the number and diversity of diseases burgeoning and respective therapeutic treatments receding, a new therapeutic approach is needed. Such an approach should be focused less on targeting specific substrates in the disease state and more on bolstering the immune response to the disease. Since the discovery of penicillin, which targets bacteria-specific cell walls conveniently absent in man, the model of modem medicine has been to eliminate substrates in the disease state, while leaving the host system unaffected. Unfortunately, few therapies have ever reached that pinnacle and fewer still remain effective in the face of resistance mutations. Applied to cancer, upregulated kinases have been the targets of therapeutic development. Unfortunately, the only recent therapeutic agent to hit the bull's-eye is Gleevec®, and likely not solely because of its kinase inhibitory activity. Borg et al. J. Clin. Invest. 114:379-388 (2004).

There are numerous benefits to (or detriments to not) potentiating an immune response instead of, or in addition to, disease substrate inhibition. One advantage is that substrates in the disease and host are commonly shared, although possibly upregulated in the disease state. For example, cancer drugs targeting kinases may be cytotoxic and may destroy cellular machinery in the host in addition to the cancer cells. Subsequently, the maximum tolerated doses (MTDs) necessary for treatment efficacy may result in undesirable side effects and even weaken the immune response in the patient. Such side effects may require cessation of treatment. Conversely, as seen with Gleevec®, the dual action of inhibiting bcr-abl, while stimulating an immune response, likely contributes to its efficacy and tolerability, particularly because NK cells, which are stimulated by administration of Gleevec®, independently play a role in tumor recession. This synergistic approach to cancer regression is extremely effective. Alternatively, cytotoxics that suppress the immune system may independently contribute to the disease state since they may inhibit separate pathways that are involved in recovery.

Another advantage to immune potentiation is that it provides a platform less easily bypassed by resistance mutations. Where therapeutic targets are so polarized and specific (which may be necessary in order to avoid targeting host cells), such as a particular substrate in a viral replicon or a kinase in a cancer cell line, a single point mutation in the disease state may render it unaffected by a drug resulting in even harsher strains of the disease in future generations.

Novel methods and mechanisms for treating patients having diseases resistant to, or inadequately treated by, conventional approaches utilizing agents targeting specific immune response mechanisms in the body is needed.

Substances that stimulate immune cells *in vitro* exhibit similar immunostimulatory effects *in vivo.* These compounds, such as recombinant cytokines, pathogen products (e.g. toxins, lipids, proteins/peptides, carbohydrates and nucleic acids) and other mammalian-derived immunostimulatory molecules (e.g. heat shock proteins, immune complexes and proteoglycans) all induce a measurable pro-inflammatory response both *in vitro* and *in vivo.*

Immune response to certain agents that are otherwise less potent can be enhanced through the use of adjuvants. Such adjuvants potentiate the immune response to the specific agents or antigens and are therefore the subject of considerable interest and study within the medical community. Historically, the classic adjuvants have been Freund's complete or incomplete (i.e., without mycobacteria) adjuvants. Edmund Coley described the potential of Coley's toxin for cancer immuno-therapy. Other materials, such as mineral oil and aluminum hydroxide, have also been used as adjuvants, but they invariably suffer from disadvantages. For example, mineral oil is known to produce tissue irritation and to be potentially oncogenic. Alum, the only approved adjuvant in the United States, also induces granulomas at the inoculation site and furthermore it does not effectively induce cell-mediated immunity. Moreover, many of the adjuvants currently available have limited utility because they contain components that are not metabolizable in humans. Additionally, most adjuvants are difficult to prepare in that they may require time consuming procedures and the use, in some cases, of elaborate and expensive equipment to formulate a vaccine and adjuvant system.

Immunological adjuvants are described in "Current Status of Immunological Adjuvants", Ann. Rev. Immunol., 1986, 4, pp. 369-388, and "Recent Advances in Vaccine Adjuvants and Delivery Systems" by Derek T O'Hagan and Nicholas M. Valiante. See also U.S. Pat. Nos. 4,806,352; 5,026,543; and 5,026,546 for disclosures of various vaccine adjuvants appearing in the patent literature.

Compounds are described in issued U.S. Patent Nos. 4,547,511 and 4,738,971 with the general structure (a):

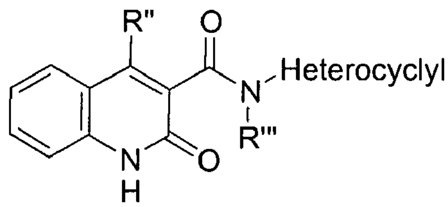

a

for the treatment of disorders responsive to agents that enhance cell-mediated immunity.

Immunostimulatory oligonucleotides and polynucleotides are described in PCT WO 98/55495 and PCT WO 98/16247. U.S. Patent Application No. 2002/0164341 describes adjuvants including an unmethylated CpG dinucleotide (CpG ODN) and a non-nucleic acid adjuvant. U.S. Patent Application No. 2002/0197269 describes compositions comprising an antigen, an antigenic CpG-ODN and a polycationic polymer.

Additionally, issued U.S. Patent Nos. 4,689,338, 5,389,640, 5,268,376, 4,929,624, 5,266,575, 5,352,784, 5,494,916, 5,482,936, 5,346,905, 5,395,937, 5,238,944, 5,525,612, WO99/29693 and U.S. Ser. No. 09/361,544 disclose compounds of the general structure (b):

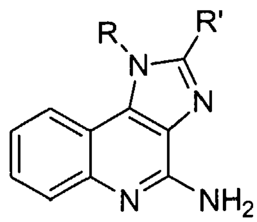

b

wherein R' is focused around H and $C_{1-8}$ alkyl for the use as "immune response modifiers." Currently at the forefront of that genus is resiquimod. Although a potent cytokine stimulator, resiquimod has not been shown to directly inhibit mechanisms in the virus. As a result, it's efficacy in treating viral infections may be greatly reduced. Nevertheless, the fact that without direct antiviral activity resiquimod is still capable of ameliorating HSV infections indicates the effectiveness of treating by immune potentiation.

Many of existing cancer therapeutics focus on targeted cytotoxicity, ideally eradicating cancer cell lines, while leaving normal cells intact. Unfortunately, the cancer cell targets for a particular drug, although upregulated in cancer cells, typically exist in normal non-carcinogenic cells as well. Subsequently, with high drug concentrations necessary for treatment, damage to healthy cells results, thereby effecting general health and immune response in the patient as well as causing toxicities such as mucositis, stomatitis, anorexia, vomiting, hypersensitivity, neurotoxicity, oncogenesis and/or mutagenesis. Furthermore, cytotoxic drugs are typically only effective against cancers with rapid growth rates, limiting their applicability and effective patient populations.

Compounding the problem of toxicity and weakened immune response is the potential for an increased fraction of treatment resistant carcinogenic cells as therapy progresses. In order for treatment to be successful (beyond palliative therapy), it is necessary that all of the cancer cells be eliminated from the patient. Where the drug is so selective in its targeted mechanism of action, the likelihood of drug resistance increases since cancer cell survival depends solely on modifying a single targeted point in the cellular machinery.

Drug regimens have been designed to kill as many tumor cells as possible by treating with "maximum tolerated doses" (MTDs) of these cytotoxic agents. In an effort to balance toxicity with efficacy, a conventional dosing schedule calls for episodic application of a cytotoxic drug at or near the MTD, followed by periods of rest to allow normal tissues to recover. Many such chemotherapy regimens are initially efficacious, resulting in tumor regression, but eventually lead relapses often marked by aggressive cancers that are resistant to the cytotoxic drug.

Subsequently, an altered regimen with multiple mechanisms of action, functioning at doses low enough to avoid toxicity, while stimulating an immune response, would be beneficial. Such a regimen could be used in patients with both rapidly growing tumor cell lines as well as those with low growth rates and/or those in remission to prevent a relapse, since toxicities would be low and immune system functionality would be enhanced as opposed to being suppressed.

It is therefore an object of the invention to provide a new treatment regimen involving a small molecule immune

potentiator, either alone or in combination with another agent, for treatment against a disease characterized by decreased immune capacity, particularly cancer, whereby the regimen targets both substrates integral in cancer cell line survival as well as host immune potentiation, allowing the patient to benefit from both palliative and curative therapies at decreased compound doses.

The current invention also seeks to provide individual therapeutic and prophylactic agents for treatment of disease states characterized by other immune deficiencies or abnormalities, including autoimmune diseases responsive to compounds with the capacity to modulate cytokines and/or TNF-a, such as multiple sclerosis and Crohn's disease. This need could be met by small molecule immune potentiators (SMIPs) because the small molecule platform provides diverse compounds for the selective manipulation of the immune response, necessary for increasing the therapeutic index immune modulators.

Furthermore, it would be desirable to provide novel compounds with a varied capacity to alter levels and/or profiles of cytokine production in human immune cells. Compounds with structural disparities will often times elicit a desired response through a different mechanism of action, or with greater specificity to a target, such as a dendritic cell, modulating potency and lowering side effects when administered to a patient.

Furthermore, it would be desirable to provide novel sole acting agents with a varied capacity to alter levels and/or profiles of cytokine production in human immune cells. Compounds with structural disparities will often times elicit a desired response through a different mechanism of action, or with greater specificity to a target, such as a dendritic cell, modulating potency and lowering side effects when administered to a patient.

The immunosuppressive effect of cytostatic substances has rendered them useful in the therapy of autoimmune diseases such as multiple sclerosis, psoriasis and certain rheumatic diseases. Unfortunately, their beneficial effect has to be weighed against the serious side effects that necessitate too low dosages and/or interruption of the treatment.

It is one object of the present invention to provide an agent or combination of active substances that results in a significantly improved cytostatic or cytotoxic effect as compared to conventional cytostatics e.g. vincristin, methotrexate, cisplatin etc. Thereby, chemotherapies may be offered that combine increasing efficiency with a large reduction of side effects and therapeutic doses. Thus, the therapeutic efficiency of known cytostatic drugs is increased. Also, certain cell lines that are insensitive to chemotherapeutic treatment may become susceptible to chemotherapy by applying the combination of active substances.

The current invention also seeks to provide individual therapeutic and prophylactic agents for treatment of disease states characterized by other immune deficiencies, abnormalities, or infections including autoimmune diseases and viral and bacterial infections responsive to compounds with the capacity to modulate cytokines and/or TNF-a, such as multiple sclerosis, Crohn's disease, HIV, HSV, and HCV, among others.

Therapeutics that could serve to augment natural host defenses against viral infections with reduced toxicity would be very beneficial. The present invention provides such therapeutic agents, and further provides other related advantages.

## SUMMARY OF THE INVENTION

In one aspect, the present invention provides immunogenic compositions and novel methods of administering a vaccine by administering 3,4-di(1H-indol-3-yl)-1H-pyrrole-2,5-diones in combination with antigens or other agents. In accordance with the first aspect, the invention further provides 3,4-di(1H-indol-3-yl)-1H-pyrrole-2,5-diones as pharmaceutical compositions, for use in the treatment of cancer, infectious diseases, allergies, and asthma. More particularly the invention provides 3,4-di(1H-indol-3-yl)-1H-pyrrole-2,5-diones for treatment of diseases in patients with suppressed immune systems. For the treatment of cancer at lowered doses, 3,4-di(1H-indol-3-yl)-1H-pyrrole-2,5-diones surpass existing therapies in that they function by independently targeting substrates involved in cancer pathways, while separately inducing an immune response in the subject thereby attacking different modes of the disease state.

As adjuvants, the 3,4-di(1H-indol-3-yl)-1H-pyrrole-2,5-dione compounds are combined with numerous antigens and delivery systems to form a final vaccine product.

As immuno-therapeutics, the 3,4-di(1H-indol-3-yl)-1H-pyrrole-2,5-dione compounds are used alone or in combination with other therapies (e.g. anti-virals, anti-bacterials, other immune modulators or in therapeutic vaccine antigens) for treatment of chronic infections such as HIV, HCV, HBV, HSV, and H. pylori, as well as medicaments for the reduction of tumor growth.

As immunotherapeutics, the 3,4-di(1H-indol-3-yl)-1H-pyrrole-2,5-dione compounds also may be used for the treatment of cancer either alone or in combination with other anti-cancer therapies (e.g. chemotherapeutic agents, mAbs or other immune potentiators). In addition, certain 3,4-di(1H-indol-3-yl)-1H-pyrrole-2,5-diones with the capacity to induce Type 1 cytokines (e.g. IL-12, TNF or IFN's) could be useful for the treatment of allergies or asthma due to their capacity to steer the immune response towards more benign sequelae. The 3,4-di(1H-indol-3-yl)-1H-pyrrole-2,5-dione compounds may be used for example for the treatment of BCG, cholera, plague, typhoid, hepatitis B infection, influenza, inactivated polio, rabies, measles, mumps, rubella, oral polio, yellow fever, tetanus, diphtheria, hemophilus influenzae

b, meningococcus infection, and pneumococcus infection. The 3,4-di(1H-indol-3-yl)-1H-pyrrole-2,5-dione compounds may be used in an anti cell proliferative effective amount for the treatment of cancer. The 3,4-di(1H-indol-3-yl)-1H-pyrrole-2,5-dione compounds may also be used in anti-Th2/Type2 cytokine amount for the deviation of allergic/asthmatic immune responses.

In preferred embodiments of the first aspect of the invention, methods of treating cancer are provided wherein known anticancer agents are combined with 3,4-di(1H-indol-3-yl)-1H-pyrrole-2,5-dione compounds to reduce tumor growth in a subject. A method of inhibiting tumor cell growth is provided, comprising administering to a subject an effective dose of a combination containing at least one 3,4-di(1H-indol-3-yl)-1H-pyrrole-2,5-dione and a mAb or antigen, wherein said combination is more effective to inhibit such cell growth than when said mAb or antigen is administered individually.

Additional embodiments, methods and compositions contemplated to be useful in the first aspect of the instant invention are disclosed in USSN 10/814,480, 10/762,873, 60/582,654 and 10/748,071 which are incorporated by reference as if set forth fully herein.

In a second aspect, the present invention provides immunogenic compositions and novel methods of administering a vaccine by administering indolinones in combination with antigens or other agents. In accordance with the second aspect of the invention, the invention further provides indolinones as pharmaceutical compositions, for use in the treatment of cancer, infectious diseases, allergies, and asthma. More particularly the invention provides indolinones for treatment of diseases in patients with suppressed immune systems. For the treatment of cancer at lowered doses, indolinones surpass existing therapies in that they function by independently targeting substrates involved in cancer pathways, while separately inducing an immune response in the subject thereby attacking different modes of the disease state.

As adjuvants, the indolinone compounds are combined with numerous antigens and delivery systems to form a final vaccine product.

As immuno-therapeutics, the indolinone compounds are used alone or in combination with other therapies (e.g. anti-virals, anti-bacterials, other immune modulators or in therapeutic vaccine antigens) for treatment of chronic infections such as HIV, HCV, HBV, HSV, and H. pylori, as well as medicaments for the reduction of tumor growth.

As immunotherapeutics, the indolinone compounds also may be used for the treatment of cancer either alone or in combination with other anti-cancer therapies (e.g. chemotherapeutic agents, mAbs or other immune potentiators). In addition, certain indolinones with the capacity to induce Type 1 cytokines (e.g. IL-12, TNF or IFN's) could be useful for the treatment of allergies or asthma due to their capacity to steer the immune response towards more benign sequelae. The indolinone compounds may be used for example for the treatment of BCG, cholera, plague, typhoid, hepatitis B infection, influenza, inactivated polio, rabies, measles, mumps, rubella, oral polio, yellow fever, tetanus, diphtheria, hemophilus influenzae b, meningococcus infection, and pneumococcus infection. The indolinone compounds may be used in an anti cell proliferative effective amount for the treatment of cancer. The indolinone compounds may also be used in anti-Th2/Type2 cytokine amount for the deviation of allergic/asthmatic immune responses.

In preferred embodiments of the second aspect of the invention, methods of treating cancer are provided wherein known anticancer agents are combined with indolinone compounds to reduce tumor growth in a subject. A method of inhibiting tumor cell growth is provided, comprising administering to a subject an effective dose of a combination containing at least one indolinone and a mAb or antigen, wherein said combination is more effective to inhibit such cell growth than when said mAb or antigen is administered individually.

Preferred SMIPs of the second aspect of the present invention include indolinone compositions, specifically N-(2-(dimethylamino) ethyl)- 5-((5- fluoro- 2- oxoindolin- 3- ylidene) methyl)- 2,4- dimethyl- 1H- pyrrole- 3- carboxamide; 3-((3,5-dimethyl-1H-pyrrol-2-yl)methylene)indolin-2-one; 3-(2,4-dimethyl-5-((2-oxoindolin-3-ylidene)methyl)-1H-pyrrol-3-yl)propanoic acid; and 3-(3,5-dibromo-4-hydroxybenzylidene)-5-iodoindolin-2-one.

Additional embodiments, methods and compositions contemplated to be useful in the second aspect of the invention are disclosed in USSN 10/814,480, 10/762,873, and 10/748,071 which are incorporated by reference as if set forth fully herein.

In a third aspect, the present invention provides immunogenic compositions and novel methods of administering a vaccine by administering chromen-4-ones in combination with antigens or other agents. The third aspect of the invention further provides chromen-4-ones as pharmaceutical compositions, for use in the treatment of cancer, infectious diseases, allergies, and asthma. More particularly the invention provides chromen-4-ones for treatment of diseases in patients with suppressed immune systems. For the treatment of cancer at lowered doses, chromen-4-ones surpass existing therapies in that they function by independently targeting substrates involved in cancer pathways, while separately inducing an immune response in the subject thereby attacking different modes of the disease state.

As adjuvants, the chromen-4-one compounds are combined with numerous antigens and delivery systems to form a final vaccine product.

As immuno-therapeutics, the chromen-4-one compounds are used alone or in combination with other therapies (e.g. anti-virals, anti-bacterials, other immune modulators or in therapeutic vaccine antigens) for treatment of chronic infections such as HIV, HCV, HBV, HSV, and H. pylori, as well as medicaments for the reduction of tumor growth.

As immunotherapeutics, the chromen-4-one compounds also may be used for the treatment of cancer either alone or in combination with other anti-cancer therapies (e.g. chemotherapeutic agents, mAbs or other immune potentiators). In addition, certain chromen-4-ones with the capacity to induce Type 1 cytokines (e.g. IL-12, TNF or IFN's) could be useful for the treatment of allergies or asthma due to their capacity to steer the immune response towards more benign sequelae. The chromen-4-one compounds may be used for example for the treatment of BCG, cholera, plague, typhoid, hepatitis B infection, influenza, inactivated polio, rabies, measles, mumps, rubella, oral polio, yellow fever, tetanus, diphtheria, hemophilus influenzae b, meningococcus infection, and pneumococcus infection. The chromen-4-one compounds may be used in an anti cell proliferative effective amount for the treatment of cancer. The chromen-4-one compounds may also be used in anti-Th2/Type2 cytokine amount for the deviation of allergic/asthmatic immune responses.

In preferred embodiments of the third aspect of the invention, methods of treating cancer are provided wherein known anticancer agents are combined with chromen-4-one compounds to reduce tumor growth in a subject. A method of inhibiting tumor cell growth is provided, comprising administering to a subject an effective dose of a combination containing at least one chromen-4-one and a mAb or antigen, wherein said combination is more effective to inhibit such cell growth than when said mAb or antigen is administered individually.

Preferred SMIPs of the third aspect of the present invention include chromen-4-one compositions, specifically 2-(2-chlorophenyl)-2,3-dihydro-5,7-dihydroxy-8-(3-hydroxy-1-methylpiperidin-4-yl)chromen-4-one; 5,7-dihydroxy-3-(4-hydroxyphenyl)-4H-chromen-4-one; and 2,3-dihydro-5,7-dihydroxy-6-methoxy-2-(3,4-dimethoxyphenyl)chromen-4-one.

Additional embodiments, methods and compositions contemplated to be useful in the third aspect instant invention are disclosed in USSN 10/814,480, 10/762,873, 60/582,654 and 10/748,071 which are incorporated by reference as if set forth fully herein.

In a fourth aspect, the present invention provides immunogenic compositions and novel methods of administering a vaccine by administering derivatized pyridazines in combination with antigens or other agents. In accordance with the fourth aspect, the invention further provides derivatized pyridazines as pharmaceutical compositions, for use in the treatment of cancer, infectious diseases, allergies, and asthma. More particularly the invention provides derivatized pyridazines for treatment of diseases in patients with suppressed immune systems. For the treatment of cancer at lowered doses, derivatized pyridazines surpass existing therapies in that they function by independently targeting substrates involved in cancer pathways, while separately inducing an immune response in the subject thereby attacking different modes of the disease state.

As adjuvants, the derivatized pyridazine compounds are combined with numerous antigens and delivery systems to form a final vaccine product.

As immuno-therapeutics, the derivatized pyridazine compounds are used alone or in combination with other therapies (e.g. anti-virals, anti-bacterials, other immune modulators or in therapeutic vaccine antigens) for treatment of chronic infections such as HIV, HCV, HBV, HSV, and H. pylori, as well as medicaments for the reduction of tumor growth.

As immunotherapeutics, the derivatized pyridazine compounds also may be used for the treatment of cancer either alone or in combination with other anti-cancer therapies (e.g. chemotherapeutic agents, mAbs or other immune potentiators). In addition, certain derivatized pyridazines with the capacity to induce Type 1 cytokines (e.g. IL-12, TNF or IFN's) could be useful for the treatment of allergies or asthma due to their capacity to steer the immune response towards more benign sequelae. The derivatized pyridazine compounds may be used for example for the treatment of BCG, cholera, plague, typhoid, hepatitis B infection, influenza, inactivated polio, rabies, measles, mumps, rubella, oral polio, yellow fever, tetanus, diphtheria, hemophilus influenzae b, meningococcus infection, and pneumococcus infection. The derivatized pyridazine compounds may be used in an anti cell proliferative effective amount for the treatment of cancer. The derivatized pyridazine compounds may also be used in anti-Th2/Type2 cytokine amount for the deviation of allergic/asthmatic immune responses.

In preferred embodiments of the fourth aspect of the invention, methods of treating cancer are provided wherein known anticancer agents are combined with derivatized pyridazine compounds to reduce tumor growth in a subject. A method of inhibiting tumor cell growth is provided, comprising administering to a subject an effective dose of a combination containing at least one derivatized pyridazine and a mAb or antigen, wherein said combination is more effective to inhibit such cell growth than when said mAb or antigen is administered individually.

Preferred SMIPs of the fourth aspect of the present invention include derivatized pyridazine compositions, specifically 1-(4-Chloroanilino)-4-(4-pyridylmethyl)phthalazine.

Additional embodiments, methods and compositions contemplated to be useful in the fourth aspect of the instant invention are disclosed in USSN 10/814,480, 10/762,873, 60/582,654, US 6,258,812 and 10/748,071 which are incorporated by reference as if set forth fully herein.

In a fifth aspect, the present invention provides immunogenic compositions and novel methods of administering a vaccine by administering staurosporine analogs in combination with antigens or other agents. The fifth aspect of the invention further provides staurosporine analogs as pharmaceutical compositions, for use in the treatment of cancer,

infectious diseases, allergies, and asthma. More particularly the invention provides staurosporine analogs for treatment of diseases in patients with suppressed immune systems. For the treatment of cancer at lowered doses, staurosporine analogs surpass existing therapies in that they function by independently targeting substrates involved in cancer pathways, while separately inducing an immune response in the subject thereby attacking different modes of the disease state.

As adjuvants, the staurosporine analog compounds are combined with numerous antigens and delivery systems to form a final vaccine product.

As immuno-therapeutics, the staurosporine analog compounds are used alone or in combination with other therapies (e.g. anti-virals, anti-bacterials, other immune modulators or in therapeutic vaccine antigens) for treatment of chronic infections such as HIV, HCV, HBV, HSV, and H. pylori, as well as medicaments for the reduction of tumor growth.

As immunotherapeutics, the staurosporine analogs also may be used for the treatment of cancer either alone or in combination with other anti-cancer therapies (e.g. chemotherapeutic agents, mAbs or other immune potentiators). In addition, certain staurosporine analogs with the capacity to induce Type 1 cytokines (e.g. IL-12, TNF or IFN's) could be useful for the treatment of allergies or asthma due to their capacity to steer the immune response towards more benign sequelae. The staurosporine analog compounds may be used for example for the treatment of BCG, cholera, plague, typhoid, hepatitis B infection, influenza, inactivated polio, rabies, measles, mumps, rubella, oral polio, yellow fever, tetanus, diphtheria, hemophilus influenzae b, meningococcus infection, and pneumococcus infection. The staurosporine analog compounds may be used in an anti cell proliferative effective amount for the treatment of cancer. The staurosporine analog compounds may also be used in anti-Th2/Type2 cytokine amount for the deviation of allergic/asthmatic immune responses.

In preferred embodiments of the fifth aspect of the invention, methods of treating cancer are provided wherein known anticancer agents are combined with staurosporine analog compounds to reduce tumor growth in a subject. A method of inhibiting tumor cell growth is provided, comprising administering to a subject an effective dose of a combination containing at least one staurosporine analog and a mAb or antigen, wherein said combination is more effective to inhibit such cell growth than when said mAb or antigen is administered individually.

Preferred SMIPs of the fifth aspect of the present invention include staurosporine analog compositions, specifically 9,13-Epoxy-1H,9H-diindolo[1,2,3-gh:3',2',1'-lm]pyrrolo[3,4-j][1,7]benzodiazonin-1-one, 2,3,10,11,12,13-hexahydro-11,12-dihydroxy-10-methoxy-9-methyl; 9,13-Epoxy-1H,9H-diindolo[,2,3-gh:3',2',1'-lm]pyrrolo[3,4-j][1,7]benzodiazonin-1-one, 2,3,10,11,12,13-hexahydro-10-methoxy-9-methyl-11-(methylamino); 9,13-Epoxy-1H,9H-diindolo[1,2,3-gh:3',2',1'-lm]pyrrolo[3,4j][1,7]benzodiazonin-1-one, 2,3,10,11,12,13-hexahydro-3-hydroxy-10-methoxy-9-methyl-11-(methylamino); 9,12-Epoxy-1H-diindolo[1,2,3-fg:3',2',1'-kl]pyrrolo[3,4-i][1,6]benzodiazocin-1-one, 2,3,9,10,11,12-hexahydro-10-hydroxy-10-(hydroxymethyl)-9-methyl; and benzamide, N-[(9S,10R,11R,13R)-2,3,10,11,12,13-hexahydro-10-methoxy-9-methyl-1-oxo-9,13-epoxy-1H,9H-diindolo[1,2,3-gh:3',2',1'-lm]pyrrolo[3,4-j][1,7]benzodiazonin-11-yl]-N-methyl.

Additional embodiments, methods and compositions contemplated to be useful in the fifth aspect of the instant invention are disclosed in USSN 10/814,480, 10/762,873, 60/582,654 and 10/748,071 which are incorporated by reference as if set forth fully herein.

In a sixth aspect, the instant invention provides novel immune potentiators, immunogenic compositions, and novel methods of administering a vaccine, by administering nucleoside analogs alone or in combination with antigens/other agents. The sixth aspect of the invention further provides compositions, novel compounds and pharmaceutical compositions, for use in the treatment of infectious diseases.

The nucleoside analogs used in the methods and compositions of the sixth aspect of the invention are inexpensive to produce and easy to administer. They have potential for finer specificity compared to existing immunostimulants, thus providing improved efficacy and safety profiles.

As adjuvants, the nucleoside analogs are combined with numerous antigens and delivery systems to form a final vaccine product.

As immuno-therapeutics, the nucleoside analogs are used alone or in combination with other therapies (e.g. anti-virals, anti-bacterials, other immune modulators or in therapeutic vaccine antigens) for treatment of chronic infections such as Kaposi's sarcoma, anogenital warts, HIV, HCV, SARS, HBV, HPV, HSV, and H. pylori.

The nucleoside analogs of the present invention target substrates in the virus or disease state, such as, for example proteases, replicases, DNA polymerase, and/or RNA polymerase.

In addition, certain nucleoside analogs with the capacity to induce Type 1 cytokines (e.g. IL-12, TNF or IFN's) could be useful for the treatment of allergies or asthma due to their capacity to steer the immune response towards more benign sequelae. The nucleoside analogs may be used for example for the treatment of BCG, cholera, plague, typhoid, hepatitis B infection, influenza, inactivated polio, rabies, measles, mumps, rubella, oral polio, yellow fever, tetanus, diphtheria, hemophilus influenzae b, meningococcus infection, and pneumococcus infection. The nucleoside analogs may be used in an anti cell proliferative effective amount for the treatment of cancer. The nucleoside analogs may also be used in anti-Th2/Type2 cytokine amount for the deviation of allergic/asthmatic immune responses.

Additional embodiments, methods and compositions contemplated to be useful in the sixth aspect of instant

invention are disclosed in USSN 10/814,480, 10/762,873, 60/582,654 and 10/748,071 which are incorporated by reference as if set forth fully herein.

Methods of manufacturing compounds and compositions described herein are provided and contemplated to fall within the scope of the invention. Further provided are pharmaceutical compositions comprising any one of the compounds of Table 1, or compounds encompassed by Formulas I, II, or III, of Section VI of the detailed description.

In a seventh aspect, the present invention immunogenic compositions and novel methods of administering a vaccine, by administering fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, or tetrathiomolybdate in combination with antigens or other agents. The seventh aspect of the invention further provides one of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, or tetrathiomolybdate as a pharmaceutical composition, for use in the treatment of diseases associated or complicated with immune suppression, such as cancer, infectious diseases, allergies, and asthma. For the treatment of cancer at attenuated doses, fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate surpass existing therapies in that they function by independently targeting substrates involved in cancer pathways, while separately inducing an immune response in the subject thereby attacking different modes of the disease state.

As adjuvants, the SMIP compounds (namely fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, or tetrathiomolybdate) are combined with numerous antigens and delivery systems to form a final vaccine product.

As immuno-therapeutics, the SMIP compounds are used alone or in combination with other therapies (e.g. anti-virals, anti-bacterials, other immune modulators or in therapeutic vaccine antigens) for treatment of autoimmune diseases, chronic infections such as HIV, HCV, HBV, HSV, and H. pylori, as well as medicaments for the reduction of tumor growth.

More particularly, one embodiment of the seventh aspect of the invention provides administering a compound selected from the group consisting of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate to treat one of the following diseases: bacterial diseases autoimmune-, allergic- and viral-diseases, disturbances caused by the incidence of mental diseases, central nervous system depressants, habitual alcohols, and disorders of the respiratory system, CNS, urinary system, lymphatic system, reproductive system, or digestive system.

As immunotherapeutics, the SMIP compounds also may be used for the treatment of cancer either alone or in combination with other anti-cancer therapies (e.g. chemotherapeutic agents, mAbs or other immune potentiators). In addition, fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, or tetrathiomolybdate with the capacity to induce Type 1 cytokines (e.g. IL-12, TNF or IFN's) are useful for the treatment of allergies or asthma due to their capacity to steer the immune response towards more benign sequelae.

In preferred embodiments of the seventh aspect of the invention, methods of treating cancer are provided wherein known anticancer agents are combined with SMIP compounds to reduce tumor growth in a subject. A method of inhibiting tumor cell growth is provided, comprising administering to a subject an effective dose of a combination containing at least one SMIP and a Mab or antigen, wherein said combination is more effective to inhibit such cell growth than when said Mab or antigen is administered individually.

Preferable SMIPs of the seventh aspect of the present invention include fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, or tetrathiomolybdate compositions as well as analogs disclosed in the following patents and patent applications: US 4,323,581, US 6,258,812, WO 98/35958, WO 01/60814, US 5,883,113, WO 99/61422, US 5,883,113, WO 99/61422, WO 03/24978, WO 03/04505, US 5,780,454, US 2003134846, WO 97/21701, US 5,621,100, WO 01/32651, WO 02/16351, US 6,727,256, WO 02/02552, US 5,457,105, US 5,616,582, US 5,770,599, US 5,747,498, WO, 96/30347, US 2003171303, WO 97/20842, WO 99/02162, WO 95/17182, WO 01/79255, WO 89/07105, US 5,439,936, WO 94/18151, WO 97/42949, WO 98/13344, US 5,554,519, WO 98/04541, US 6,025,387, US 2004073044, WO 02/62826, WO 04/06834, US 6,331,555, and WO 01/60814, wherein they are administered in reduced dosing amounts from existing regimens. More particularly, preferred compounds of the seventh aspect include those encompassed by Formula I in the aforementioned references.

Additional embodiments, methods and compositions contemplated to be useful in the seventh aspect of the instant invention are disclosed in USSN 10/814,480, 10/762,873, and 10/748,071.

Further embodiments of the several aspects of the invention include those described in the detailed description.

## DETAILED DESCRIPTION OF THE INVENTION

In each of the aspects of the invention, the "method of modulating an immune response in a patient comprising administering a compound" of a particular formula, such as formula (I), can be replaced with: "use of a compound of formula (I) in the manufacture of a medicament for modulating an immune response in a patient." In other embodiments the compounds are used in the manufacture of a medicament for treating an infectious disease, autoimmune disease, allergies, or cancer. In other embodiments the compounds are used in the manufacture of a medicament for use as an adjuvant. Preferred compounds of the present invention include those having a particular formula as described in the aspects of the invention.

Other embodiments provide the use of a compound of formula (I) and another agent for simultaneous separate or sequential administration. In a more particular embodiment the other agent is an antigen. In another more particular embodiment the use is for modulating an immune response in a patient. In another embodiment the use is for treating an infectious disease, autoimmune disease, allergies, or cancer. In another embodiment the use is as an adjuvant.

Other embodiments provide a pharmaceutical preparation or system, comprising (a) a first pharmaceutical agent, which comprises a compound of formula (I); and (b) a second pharmaceutical agent, wherein said first and second agents are either in admixture or are separate compositions. In a more particular embodiment the second agent is an antigen. More specifically, the agents are for simultaneous separate or sequential administration. In another more particular embodiment the use is for modulating an immune response in a patient. In another embodiment the use is for treating an infectious disease, autoimmune disease, allergies, or cancer. In another embodiment the use is as an adjuvant.

A kit comprising (a) a first pharmaceutical agent, which comprises a SMIP of formula I-L; and (b) a second pharmaceutical agent. In a more particular embodiment the second agent is an antigen. In another more particular embodiment the use is for modulating an immune response in a patient. In another embodiment the use is for treating an infectious disease, autoimmune disease, allergies, or cancer. In another embodiment the use is as an adjuvant.

Another embodiment provides the use of a compound of formula (I) and another agent in the manufacture of a combination medicament. In a more particular embodiment the other agent is an antigen. In another more particular embodiment the use is for modulating an immune response in a patient. In another embodiment the use is for treating an infectious disease, autoimmune disease, allergies, or cancer. In another embodiment the use is as an adjuvant.

Another embodiment provides the use of a compound of formula (I) in the manufacture of a medicament, wherein the medicament is co-administered with another agent. In a more particular embodiment the second agent is an antigen. In another more particular embodiment the use is for modulating an immune response in a patient. In another embodiment the use is for treating an infectious disease, autoimmune disease, allergies, or cancer. In another embodiment the use is as an adjuvant.

Another embodiment provides the use of an antigen in the manufacture of a medicament, wherein the medicament is co-administered with a compound of formula (I).

The two agents are preferably administered within 4 hours of each other.

Another embodiment provides the use of a compound of formula (I) in the manufacture of a medicament, wherein the medicament is for administration to a patient who has been pre-treated with another agent. In a more particular embodiment the second agent is an antigen. In another more particular embodiment the use is for modulating an immune response in a patient. In another embodiment the use is for treating an infectious disease, autoimmune disease, allergies, or cancer. In another embodiment the use is as an adjuvant.

Another embodiment provides the use of an antigen in the manufacture of a medicament, wherein the medicament is for administration to a patient who has been pre-treated with a compound of formula (I). The pre-treatment may be recent (e.g. within the 24 hours preceding administration of said medicament), intermediate (e.g. more than 24 hours previous, but no longer than 4 weeks), more distant (e.g. at least 4 weeks previous), or very distant (e.g. at least 6 months previous), with these time periods referring to the most recent pre treatment dose. The patient may be refractory to treatment by the pharmaceutical agent that was administered in the pre-treatment. In another more particular embodiment the use is for modulating an immune response in a patient. In another embodiment the use is for treating an infectious disease, autoimmune disease, allergies, or cancer. In another embodiment the use is as an adjuvant.

Another embodiment provides, the use of a compound of formula (I) in the manufacture of a medicament, wherein the medicament is for administration to a patient who has a tumor or infection that is resistant to treatment with another agent.

**Section I - First Aspect of the Invention - Quinazolines for Immunopotentiation**

All definitions, descriptors of constituent variables for chemical formulas, and descriptions appearing in the

section shall be understood to apply to this section only.

In accordance with the first aspect of the invention, Applicants have discovered methods of stimulating cytokine activity in cells and immunotherapeutics and/or vaccine adjuvants, that will provide effective treatments for disorders described herein and those apparent to one skilled in the art.

One embodiment of the first aspect invention includes a method of modulating an immune response in a subject comprising administering a compound of formula I:

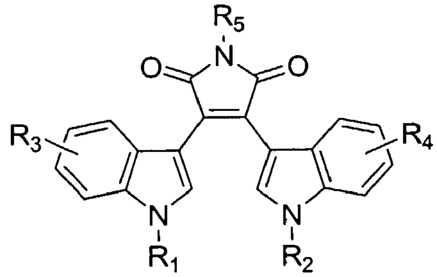

I

wherein,

$R_1$ and $R_2$ are each independently H, alkyl, aryl, alkoxy, -C(O)$R_c$, or heterocyclyl; or

$R_1$ and $R_2$ are taken together to form a bridge in subformula Ia:

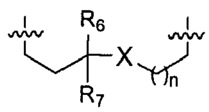

Ia

the binding being achieved at the bonds crossed with a 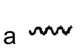 ;

$R_3$ and $R_4$ each independently H, -CN, -OH, halogen, alkyl, aryl, alkoxy, -N$R_a$$R_b$, -C(O)$R_c$, -S(O)$_p$$R_d$, or heterocyclyl;

$R_5$ is H or alkyl;

$R_6$ is H, -OH, -$NH_2$, halogen, or alkyl;

$R_7$ is H, -CN, -OH, halogen, alkyl, aryl, alkoxy, -N$R_a$$R_b$, -C(O)$R_c$, -S(O)$_p$$R_d$, or heterocyclyl;

X is -CH($R_e$)-, -O-, -S-, or -N($R_f$)-;

each $R_a$ and $R_b$ is independently H, alkyl, -C(O)$R_c$, aryl, heterocyclyl, or alkoxy; each $R_c$ is independently H, alkyl, alkoxy, -$NH_2$, -NH(alkyl), -N(alkyl)$_2$, aryl, or heterocyclyl;

each $R_d$ is independently H, alkyl, alkenyl, aryl, or -N$R_a$$R_b$;

$R_e$ is H, -OH, halogen, -C(O)$R_c$, aryl, heterocyclyl, or -N$R_a$$R_b$;

n is 0, 1, or 2;

each p is independently 0, 1, or 2;

each q is independently 0, 1, or 2.

Another embodiment is provided, wherein $R_5$ is H. In still another more particular embodiment $R_3$ and $R_4$ are both H. In still another more particular embodiment $R_1$ is methyl, ethyl, propyl, or isopropyl, and $R_2$ is heterocyclyl.

In another more particular embodiment, $R_2$ is defined as in subformula $R_{2a}$:

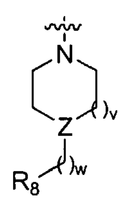

$R_{2a}$

the binding being achieved at the bond crossed with a ;
Z is N, O, S, or CH, provided that when Z is O or S, w is 0 and $R_8$ is absent;
$R_8$ is absent, aryl or heterocyclyl;
v is 0, 1, or 2; and
w is 0, 1, or 2.

In another embodiment thereof, Z is N. In still another more particular embodiment, w and v are both 1. In still another more particular embodiment $R_8$ is pyridyl.

In a separate embodiment thereof, $R_1$ and $R_2$ are taken together to form a bridge in subformula Ia:

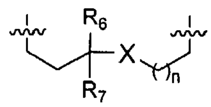

Ia

the binding being achieved at the bonds crossed with a .

In another more particular embodiment of substructure Ia, $R_6$ is H. In another more particular embodiment of substructure Ia, n is 1.

In another more particular embodiment of substructure Ia, X is O. In still another more particular embodiment of substructure Ia, $R_7$ is $-CH_2-NH(alkyl)$, $-CH_2-NH_2$, $-CH_2-CH_2-NH_2$, $-CH_2-CH_2-NH(alkyl)$, $-CH_2-CH_2-N(alkyl)_2$ or $-CH_2-N(alkyl)_2$. In a preferred embodiment thereof said alkyl with $R_7$ is methyl, ethyl, propyl, or isopropyl.

In another more particular embodiment of substructure Ia, X is $-CH_2-$. In still another more particular embodiment of substructure Ia, $R_7$ is $-CH_2-NH(alkyl)$, $-CH_2-NH_2$, $-CH_2-CH_2-NH_2$, $-CH_2-CH_2-NH(alkyl)$, $-CH_2-CH_2-N(alkyl)_2$ or $-CH_2-N(alkyl)_2$. In a preferred embodiment thereof said alkyl with $R_7$ is methyl, ethyl, propyl, or isopropyl.

Another embodiment of the invention provides a method of modulating an immune response in a subject comprising administering a compound selected from the group consisting of:

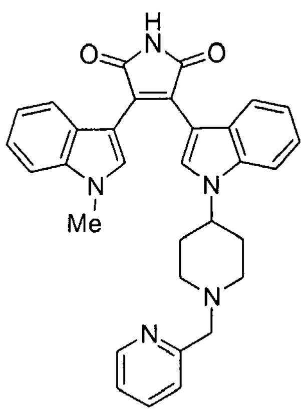
,
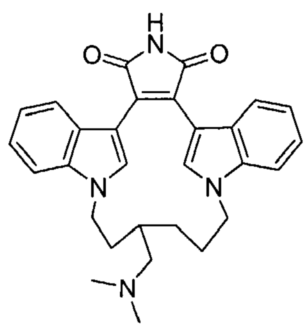
, and
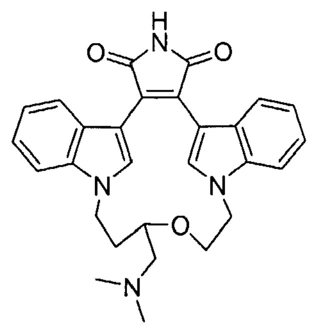
.

In a more particular embodiment of any of the above methods, said modulating is inducing. In another embodiment said inducing stimulates production of cytokines, chemokines, and/or growth factors.

In another embodiment said compound is administered in a subcytotoxic amount to said subject.

In another embodiment said subject is in remission from cancer.

In another embodiment said compound is administered for the treatment of refractory cancer cells.

In another embodiment said compound is administered metronomically.

In another embodiment the subject is not suffering from cancer.

In another embodiment said compound is co-administered with another agent.

In another embodiment said compound is administered in a dose capable of increasing TNF-$\alpha$ levels.

In another embodiment said compound has an average steady state drug concentration in the blood of less than 20 $\mu$M.

In another embodiment subject is suffering from an autoimmune disease. Further still, said autoimmune disease is multiple sclerosis or Crohn's disease.

In another embodiment said subject is suffering from a viral infection.

In another embodiment said viral infection is HCV, HIV, or HSV.

In another embodiment said subject is suffering from allergies.

In another embodiment said subject is suffering from asthma.

In another embodiment the subject is suffering from precancerous lesions, such as actinic keratosis, atypical or dysplastic nevi, or premalignant lentigos. In another embodiment the subject is suffering from a disease associated with abnormal cellular proliferation, such as, neuro-fibromatosis, atherosclerosis, pulmonary fibrosis, arthritis, psoriasis, glomerulonephritis, restenosis, proliferative diabetic retinopathy (PDR), hypertrophic scar formation, inflammatory bowel disease, transplantation rejection, angiogenesis, or endotoxic shock.

Another embodiment provides a pharmaceutical composition containing any of the aforementioned compounds or embodiments of formula I.

In another embodiment the subject is suffering from precancerous lesions, such as actinic keratosis, atypical or dysplastic nevi, or premalignant lentigos.

Furthermore, it is contemplated that the invention encompasses all possible combinations of the preceding embodiments.

The 3,4-di(1H-indol-3-yl)-1H-pyrrole-2,5-dione compounds can be used with or without an antigen in therapeutic applications, for example to treat cancer or infectious diseases. The 3,4-di(1H-indol-3-yl)-1H-pyrrole-2,5-dione compounds also may be used in combination with other therapeutic agents, such as anti-virals and monoclonal antibodies in different therapeutic applications.

One preferred embodiment of the method of inducing an immunostimulatory effect in a patient is directed to administering an immunogenic composition comprising a vaccine in an amount effective to stimulate an immune response such as a cell-mediated immune response and, as a vaccine adjuvant, an 3,4-di(1H-indol-3-yl)-1H-pyrrole-2,5-dione compound, in an amount effective to potentiate the immune response such as the cell-mediated immune response to the vaccine.

Agents combined with the 3,4-di(1H-indol-3-yl)-1H-pyrrole-2,5-dione compounds, contemplated to be useful in treating the aforementioned diseases include those well known in the art, such as, anesthetics, hypnotic sedatives, anti-anxieties, antiepileptics, antipyretic antiphlogistics, stimulants, wake amines, anti-parkinson drugs, agents for psychoneuroses, agents for central nervous system, skeletal muscle relaxants, agents for autonomic nervous system, antispastic agents, cytotoxic agents, monoclonal antibodies, drugs for eye, drugs for nose and ear, anti-vertiginous drugs, cardiotonics, antiarrhythmic drugs, diuretics, pressure reduction drugs, vasoconstrictors, coronary vaso-dilators, peripheral vasodilating drugs, hyper-lipemia drugs, breath stimulants, antitussive and expectorant drugs, bronchodilators, drugs for allergy, antidiarrheal drugs, drugs for intestinal disorders, peptic ulcer drugs, stomachic digestants, antacids, cholagogouses, pituitary hormone drugs, salivary gland hormones, thyroid hormone drugs, antithyroid drugs, anabolic steroids, corticosteroids, androgen drugs, estrogen drugs, corpus luteum hormone drugs, mixed hormones, urinary/genital organ drugs, anus drugs, surgical sterilizations/antiseptics, wound protectives, externals for purulent diseases, analgesics, antipruritics, astringents, antiphlogistics, externals for parasite skin diseases, skin-softening drugs, caustics, dental/oral drugs, vitamins, inorganic preparations, supplemental liquids, hemostatics, anticoagulation drugs, drugs for liver diseases, antidotes, habitual intoxication drugs, drugs for treatment of gout, enzyme preparations, diabetic drugs, antioncotics, antihistaminics, antibiotics (such as ketolides, aminoglycosides, sulphonamides, and/or beta lactams), chemotherapeutics, biological preparations, anthelmintics, anti-Protozoas, drugs for preparations, X-ray contrast media, and diagnostic drugs.

Further in accordance with the first aspect of the invention, methods of the invention are provided wherein compositions described herein are used for the treatment of cancer and reduction of tumor growth. In one embodiment an 3,4-di(1H-indol-3-yl)-1H-pyrrole-2,5-dione compound of the invention is combined with a known mAb for the treatment of cancer. In a presently preferred embodiment of this embodiment of the present invention, an antibody and a 3,4-di(1H-indol-3-yl)-1H-pyrrole-2,5-dione compound are administered. It may be particularly preferred that said antibody, individually, has an inhibiting effect upon tumor cell growth and that the 3,4-di(1H-indol-3-yl)-1H-pyrrole-2,5-dione compound induces the production of cytokines.

In accordance with another embodiment of the first aspect of the present invention, there is provided a therapeutic composition for inhibiting tumor cell growth in a subject, which composition comprises an effective amount of a combination of at least an 3,4-di(1H-indol-3-yl)-1H-pyrrole-2,5-dione compound and a mAb and a pharmaceutically acceptable carrier, wherein said combination is more effective to inhibit growth of certain mammalian tumor cells than are either of the agents when administered individually.

In another embodiment of the first aspect of the invention, methods of treating allergies are provided comprising administering an 3,4-di(1H-indol-3-yl)-1H-pyrrole-2,5-dione compound alone or in combination with another agent known to be effective against allergies, wherein said combination is more effective in treating an allergic condition than the known agent(s) are without the addition of said 3,4-di(1H-indol-3-yl)-1H-pyrrole-2,5-dione compound. In a more preferred embodiment the known agent is an antihistamine and/or a leukotriene inhibitor. In another preferred embodiment, the allergic condition is asthma. In another preferred embodiment, the allergic condition is selected from the group consisting of allergic rhinitis, dermatosis, and urticaria. In an even more preferred embodiment the combination is administered to

a subject enterally, parenterally, intranasally, subcutaneously, or intraarterially.

Preferred SMIPs in accordance with the first aspect of the invention are 3,4-di(1H-indol-3-yl)-1H-pyrrole-2,5-diones, as well as analogs disclosed in the following patents and patent applications: US 5,552,396, US 5,057,614, and WO 95/17182. More particularly, preferred SMIPs of the first aspect of the invention include flavones, isoflavones and those encompassed by Formula I as described in this section, or contained within the aforementioned references.

Reference to "3,4-di(1H-indol-3-yl)-1H-pyrrole-2,5-diones" indicates compounds having the general structure of Formula I as described herein. Preferred 3,4-di(1H-indol-3-yl)-1H-pyrrole-2,5-diones are

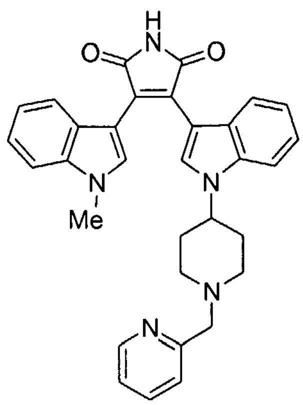

,

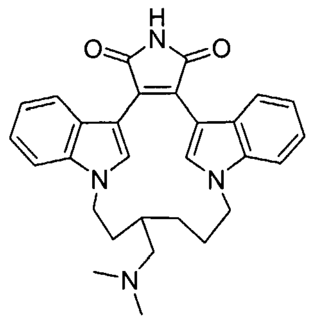

,

or

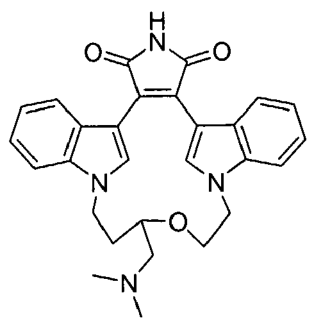

.

The foregoing may be better understood by reference to the Examples, *infra,* that are presented for illustration and not to limit the scope of the inventive concepts. The Example compounds and their analogs are either commercially available, or are easily synthesized by one skilled in the art from procedures described in patents/applications, such as US 5,552,396, US 5,057,614, WO 95/17182 and other patents/applications listed herein.

**Section II - Second Aspect of the Invention - Indolinones for Immunopotentiation**

All definitions, descriptors of constituent variables for chemical formulas, and descriptions appearing in the section shall be understood to apply to this section only.

In accordance with the second aspect of the invention, Applicants have discovered methods of stimulating cytokine activity in cells and immunotherapeutics and/or vaccine adjuvants, that will provide effective treatments for disorders described herein and those apparent to one skilled in the art.

In one embodiment of the second aspect of the invention, a method of modulating an immune response in a subject is provided, comprising administering a compound of formula I:

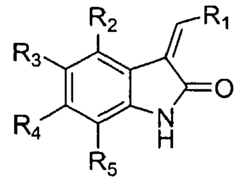

I

wherein,

$R_1$ is alkyl, aryl or heterocyclyl;

$R_2$, $R_3$, $R_4$, and $R_5$ are each independently H, -CN, -OH, halogen, alkyl, aryl, alkoxy, $-NR_aR_b$, $-C(O)R_c$, $-S(O)_nR_d$, or heterocyclyl;

each $R_a$ and $R_b$ is independently H, alkyl, $-C(O)R_c$, aryl, heterocyclyl, or alkoxy;

each $R_c$ is independently H, alkyl, alkoxy, $-NH_2$, -NH(alkyl), $-N(alkyl)_2$, aryl, or heterocyclyl;

each $R_d$ is independently H, alkyl, alkenyl, aryl, or $-NR_aR_b$;

each n is independently 0, 1, or 2; and

each q is independently 0, 1, or 2.

In a more particular embodiment, $R_1$ is heterocyclyl. In a more particular embodiment still, $R_1$ is $R_{1a}$:

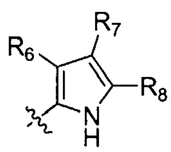

$R_{1a}$

wherein,

$R_6$, $R_7$ and $R_8$ are each independently H, -CN, -OH, halogen, alkyl, aryl, alkoxy, - $NR_aR_b$, $-C(O)R_c$, $-S(O)_nR_d$, or heterocyclyl. In yet another more particular embodiment, wherein $R_1$ is $R_{1a}$, $R_7$ is $-C(O)-(CH_2)_p-N(H)_{(2-r)}(alkyl)_r$, wherein p is 0, 1, 2, 3, 4, or 5 and r is 0, 1, or 2. In yet another more particular embodiment, wherein $R_1$ is $R_{1a}$, $R_3$ is F. In yet another more particular embodiment, wherein $R_1$ is $R_{1a}$, $R_7$ is - $(CH_2)_tCOOH$, wherein t is 1, 2, 3, or 4. In yet another more particular embodiment, wherein $R_1$ is $R_{1a}$, $R_6$ and $R_8$ are both methyl. In yet another more particular embodiment, wherein $R_1$ is $R_{1a}$, $R_7$ is H.

In another more particular embodiment of the second aspect of the invention, $R_1$ is aryl. More particular still, $R_1$ is substituted or unsubstituted phenyl. In another embodiment $R_3$ is iodide.

In another embodiment of the second aspect of the invention, a method of modulating an immune response in a subject is provided, comprising administering a compound selected from the group consisting of:

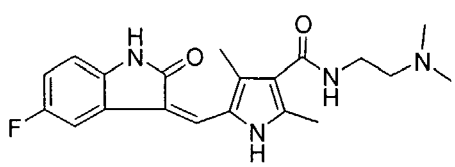

,

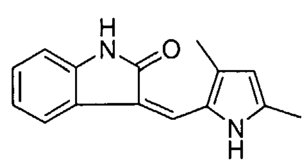

,

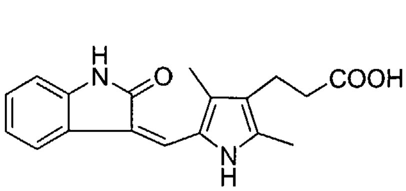

, and

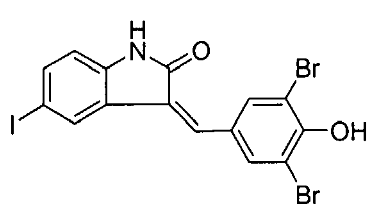

.

In a more particular embodiment of any of the above methods, said modulating is inducing. In another embodiment said inducing stimulates production of cytokines, chemokines, and/or growth factors.

In another embodiment said compound is administered in a subcytotoxic amount to said subject.

In another embodiment said subject is in remission from cancer.

In another embodiment said compound is administered for the treatment of refractory cancer cells.

In another embodiment said compound is administered metronomically.

In another embodiment the subject is not suffering from cancer.

In another embodiment said compound is co-administered with another agent.

In another embodiment said compound is administered in a dose capable of increasing TNF-$\alpha$ levels.

In another embodiment said compound has an average steady state drug concentration in the blood of less than 20 $\mu$M.

In another embodiment subject is suffering from an autoimmune disease. Further still, said autoimmune disease is multiple sclerosis.

In another embodiment said subject is suffering from a viral infection.

In another embodiment said viral infection is HCV, HIV, or HSV.

In another embodiment said subject is suffering from allergies.

In another embodiment said subject is suffering from asthma.

It is contemplated that the invention encompasses all possible combinations of the embodiments described herein.

Preferred SMIPs in accordance with the second aspect of the invention are indolinones, such as N-(2-(dimethylamino)ethyl)-5-((5-fluoro-2-oxoindolin-3-ylidene)methyl)-2,4-dimethyl-1H-pyrrole-3-carboxamide; 3-((3,5-dimethyl-1H-pyrrol-2-yl)methylene)indolin-2-one; 3-(2,4-dimethyl-5-((2-oxoindolin-3-ylidene)methyl)-1H-pyrrol-3-yl)propanoic acid; and 3-(3,5-dibromo-4-hydroxybenzylidene)-5-iodoindolin-2-one, as well as analogs disclosed in the following patents and patent applications: WO 01/60814, US 5,883,113, WO 99/61422, and WO 99/61422. More particularly, preferred SMIPs include those encompassed by Formula I as described in this section, or contained within the aforementioned references.

Reference to "indolinones" indicates compounds having the general structure of Formula I as described in this section. Preferred indolinones are N-(2-(dimethylamino)ethyl)-5-((5-fluoro-2-oxoindolin-3-ylidene)methyl)-2,4-dimethyl-1H-pyrrole-3-carboxamide; 3-((3,5-dimethyl-1H-pyrrol-2-yl)methylene)indolin-2-one; 3-(2,4-dimethyl-5-((2-oxoindolin-3-ylidene)methyl)-1H-pyrrol-3-yl)propanoic acid; and 3-(3,5-dibromo-4-hydroxybenzylidene)-5-iodoindolin-2-one.

The foregoing may be better understood by reference to the Examples, *infra*, that are presented for illustration and not to limit the scope of the inventive concepts. The Example compounds and their analogs are either commercially available, or are easily synthesized by one skilled in the art from procedures described in patents or patent applications listed herein.

**Section III - Third Aspect of the Invention - Chromen-4-ones for Immunopotentiation**

All definitions, descriptors of constituent variables for chemical formulas, and descriptions appearing in the section shall be understood to apply to this section only.

In accordance with the third aspect of the invention, Applicants have discovered methods of stimulating cytokine activity in cells and immunotherapeutics and/or vaccine adjuvants, that will provide effective treatments for disorders described herein and those apparent to one skilled in the art.

In one embodiment of the invention, a method of modulating an immune response in a subject is provided, comprising administering a compound of formula I:

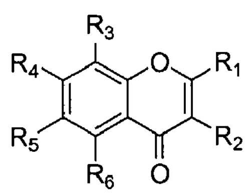

I

wherein

one of $R_1$ and $R_2$ is H, -CN, alkyl, -OH, -$NR_aR_b$, alkoxy, -C(O)$R_c$, -S(O)$_n$$R_d$, heterocyclyl, or aryl, and the other is:

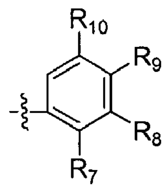

;

$R_3$, $R_4$, $R_5$, and $R_6$ are each independently H, -CN, -OH, halogen, alkyl, aryl, alkoxy, $-NR_aR_b$, $-C(O)R_c$, $-S(O)_nR_d$, or heterocyclyl;

$R_7$ and $R_{10}$ are each independently H, -CN, -OH, halogen, alkyl, aryl, alkoxy, $-NR_aR_b$, $-C(O)R_c$, $-S(O)_nR_d$, or heterocyclyl;

$R_8$ and $R_9$ are each independently H, -CN, -OH, halogen, alkyl, aryl, $-O-(CH_2)_q-R_c$, $-C(O)R_c$, $-O-(CH_2)_q-O-R_c$, $-NR_aR_b$, $-S(O)_nR_d$, or heterocyclyl;

each $R_a$ and $R_b$ is independently H, alkyl, $-C(O)R_c$, aryl, heterocyclyl, or alkoxy; each $R_c$ is independently H, alkyl, alkoxy, $-NH_2$, -NH(alkyl), $-N(alkyl)_2$, aryl, or heterocyclyl;

each $R_d$ is independently H, alkyl, alkenyl, aryl, or $-NR_aR_b$;

each $R_c$ is independently H, halogen, $-C(O)R_c$, aryl, heterocyclyl, or $-NR_aR_b$;

each n is independently 0, 1, or 2; and

each q is independently 0, 1, or 2.

In a more particular embodiment $R_4$ and $R_6$ are both -OH.

In another more particular embodiment $R_1$ is:

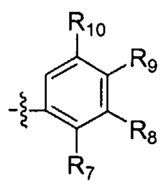

.

In another more particular embodiment $R_3$ is heterocyclyl.

In another more particular embodiment thereof, $R_3$ is:

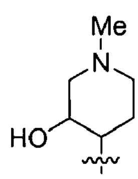

.

In another more particular embodiment $R_7$ is Cl.

In another more particular embodiment $R_4$ and $R_6$ are both -OH.

In another more particular embodiment $R_5$ is alkoxy.

In another more particular embodiment $R_5$ is methoxy.

In another more particular embodiment $R_8$ and $R_9$ are both $-O-(CH_2)_q-R_e$.

In another more particular embodiment $R_8$ and $R_9$ are both $-OCH_3$.

In another more particular embodiment $R_2$ is H.

In another embodiment $R_2$ is:

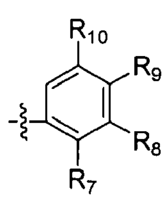

.

In another more particular embodiment $R_1$ is H.

In another more particular embodiment $R_4$ and $R_6$ are both -OH.

In another more particular embodiment $R_9$ is -OH.

Another embodiment of the invention provides a method of modulating an immune response in a subject comprising administering a compound selected from the group consisting of:

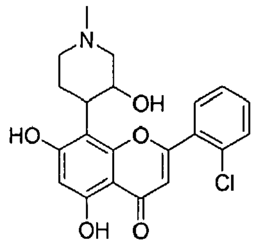 , 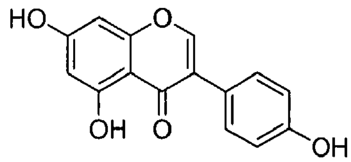 ,

and

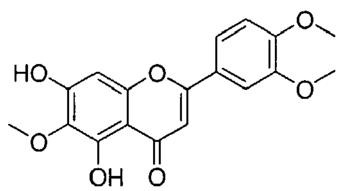 .

In a more particular embodiment of any of the above methods, said modulating is inducing. In another embodiment said inducing stimulates production of cytokines, chemokines, and/or growth factors.

In another embodiment said compound is administered in a subcytotoxic amount to said subject.

In another embodiment said subject is in remission from cancer.

In another embodiment said compound is administered for the treatment of refractory cancer cells.

In another embodiment said compound is administered metronomically.

In another embodiment the subject is not suffering from cancer.

In another embodiment said compound is co-administered with another agent.

In another embodiment said compound is administered in a dose capable of increasing TNF-$\alpha$ levels.

In another embodiment said compound has an average steady state drug concentration in the blood of less than 20 $\mu$M.

In another embodiment subject is suffering from an autoimmune disease. Further still, said autoimmune disease is multiple sclerosis or Crohn's disease.

In another embodiment said subject is suffering from a viral infection.

In another embodiment said viral infection is HCV, HIV, or HSV.

In another embodiment said subject is suffering from allergies.

In another embodiment said subject is suffering from asthma.

In another embodiment the subject is suffering from a disease associated with abnormal cellular proliferation, such as, neuro-fibromatosis, atherosclerosis, pulmonary fibrosis, arthritis, psoriasis, glomerulonephritis, restenosis, proliferative diabetic retinopathy (PDR), hypertrophic scar formation, inflammatory bowel disease, transplantation rejection, angiogenesis, or endotoxic shock.

It is contemplated that the invention encompasses all possible combinations of the embodiments described herein.

Preferred SMIPs in accordance with the third aspect of the invention are chromen-4-ones, such as 2-(2-chlorophenyl)-2,3-dihydro-5,7-dihydroxy-8-(3-hydroxy-1-methylpiperidin-4-yl)chromen-4-one; 5,7-dihydroxy-3-(4-hydroxyphenyl)-4H-chromen-4-one; and 2,3-dihydro-5,7-dihydroxy-6-methoxy-2-(3,4-dimethoxyphenyl)chromen-4-one, as well as analogs disclosed in the following patents and patent applications: WO 97/42949, and WO 98/13344, US 5,554,519, WO 98/04541, and US 6,025,387. More particularly, preferred SMIPs include flavones, isoflavones and those encompassed by Formula I as described in this section, or contained within the aforementioned references.

Reference to "chromen-4-ones" indicates compounds having the general structure of Formula I as described in this section. Preferred chromen-4-ones are flavones and isoflavones selected from the group consisting of 2-(2-chlorophenyl)-2,3-dihydro-5,7-dihydroxy-8-(3-hydroxy-1-methylpiperidin-4-yl)chromen-4-one; 5,7-dihydroxy-3-(4-hydroxyphenyl)-4H-chromen-4-one; and 2,3-dihydro-5,7-dihydroxy-6-methoxy-2-(3,4-dimethoxyphenyl)chromen-4-one.

The foregoing may be better understood by reference to the Examples, *infra*, that are presented for illustration and not to limit the scope of the inventive concepts. The Example compounds and their analogs are either commercially available, or are easily synthesized by one skilled in the art from procedures described in patents or patent applications listed herein.

**Section IV - Fourth Aspect of the Invention - Derivatized Pyridazines for Immunopotentiation**

All definitions, descriptors of constituent variables for chemical formulas, and descriptions appearing in the section shall be understood to apply to this section only.

In accordance with the fourth aspect of the invention, Applicants have discovered methods of stimulating cytokine activity in cells and immunotherapeutics and/or vaccine adjuvants, that will provide effective treatments for disorders described herein and those apparent to one skilled in the art.

One embodiment of the invention provides a method of modulating an immune response comprising administering a derivatized pyridazine or a pharmaceutically acceptable salt thereof to a subject in need thereof.

In one embodiment of the invention, a method of modulating an immune response in a subject is provided, comprising administering a compound of formula I:

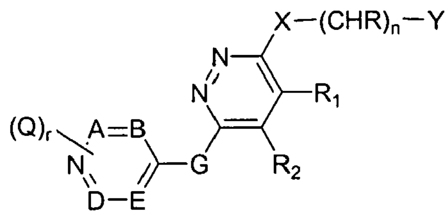

I

wherein

r is 0 to 2,

n is 0 to 2,

m is 0 to 4,

$R_1$ and $R_2$ (i) are lower alkyl, or

(ii) together form a bridge in subformula I*

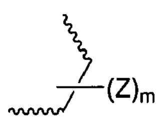

I*

the binding being achieved via the two terminal carbon atoms, or

(iii) together form a bridge in subformula I**

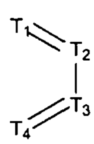

I**

wherein one or two of the ring members $T_1$, $T_2$, $T_3$ and $T_4$ are nitrogen, and the others are in each case CH, and the binding is achieved via $T_1$ and $T_4$;

A, B, D, and E are, independently of one another, N or CH, with the stipulation that not more than 2 of these radicals are N;

G is lower alkylene, lower alkylene substituted by acyloxy or hydroxy, $-CH_2-O-$, $-CH_2-S-$, $-CH_2-NH-$, oxa (-O-), thia (-S-), or imino (-NH-);

Q is lower alkyl, especially methyl;

R is H or lower alkyl;
X is imino, oxa, or thia;
Y is aryl, pyridyl, or unsubstituted or substituted cycloalkyl; and
Z is mono- or disubstituted amino, halogen, alkyl, substituted alkyl, hydroxy, etherified or esterified hydroxy, nitro, cyano, carboxy, esterified carboxy, alkanoyl, carbamoyl, N-mono- or N,N-disubstituted carbamoyl, amidino, guanidino, mercapto, sulfo, phenylthio, phenyl lower alkylthio, alkylphenylthio, phenylsulfinyl, phenyl-lower alkylsulfinyl, alkylphenylsulfinyl, phenylsulfonyl, phenyl-lower alkylsulfonyl, or alkylphenylsulfonyl, wherein--if more than 1 radical Z (m=2) is present--the substituents Z are the same or different from one another;
and wherein the bonds characterized, if present, by a wavy line are either single or double bonds;
or an N-oxide of the defined compound, wherein one or more N atoms carry an oxygen atom;
with the stipulation that, if Y is pyridyl or unsubstituted cycloalkyl, X is imino, and the remaining radicals are as defined, G is selected from the group comprising lower alkylene, $-CH_2-O-$, $-CH_2-S-$, oxa and thia;
or a pharmaceutically acceptable salt thereof.

In a more particular embodiment, $R_1$ and $R_2$ together form a bridge in subformula I*

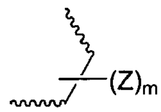

I*

the binding being achieved via the two terminal carbon atoms, wherein both wavy lines represent double bonds.

In another more particular embodiment, G is methylene.

In another more particular embodiment, r is 0.

In another more particular embodiment, X in -NH-.

In another more particular embodiment, n is 0.

In another more particular embodiment, Y is substituted phenyl.

In another more particular embodiment, said phenyl is substituted with chloro.

In another more particular embodiment, said chloro is bound para to the phenyl group's point of attachment.

Another embodiment of the invention provides a method of modulating an immune response in a subject comprising administering a compound having the structure:

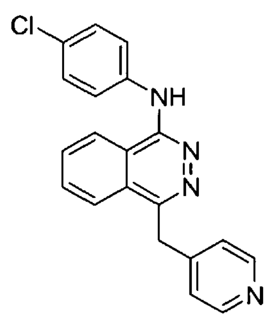

or a pharmaceutically acceptable salt thereof.

In a more particular embodiment of any of the above methods, said modulating is inducing. In another embodiment said inducing stimulates production of cytokines, chemokines, and/or growth factors.

In another embodiment said compound is administered in a subcytotoxic amount to said subject.

In another embodiment said subject is in remission from cancer.

In another embodiment said compound is administered for the treatment of refractory cancer cells.

In another embodiment said compound is administered metronomically.

In another embodiment the subject is not suffering from cancer.

In another embodiment said compound is co-administered with another agent.

In another embodiment said compound is administered in a dose capable of increasing TNF-α levels.

In another embodiment said compound has an average steady state drug concentration in the blood of less than 20 μM.

In another embodiment subject is suffering from an autoimmune disease. Further still, said autoimmune disease is multiple sclerosis or Crohn's disease.

In another embodiment said subject is suffering from a viral infection.

In another embodiment said viral infection is HCV, HIV, or HSV.

In another embodiment said subject is suffering from allergies.

In another embodiment said subject is suffering from asthma.

In another embodiment the subject is suffering from precancerous lesions, such as actinic keratosis, atypical or dysplastic nevi, or premalignant lentigos.

In another embodiment the subject is suffering from a disease associated with abnormal cellular proliferation, such as, neuro-fibromatosis, atherosclerosis, pulmonary fibrosis, arthritis, psoriasis, glomerulonephritis, restenosis, proliferative diabetic retinopathy (PDR), hypertrophic scar formation, inflammatory bowel disease, transplantation rejection, angiogenesis, or endotoxic shock.

The following compositions may be used in any of the methods or indications described herein.

The invention also relates to a compound of formula I, or a pharmaceutically acceptable salt thereof, or an N-oxide thereof, for use in modulating an immune response of a subject, wherein said compound n is 0 and any of r, m, $R_1$, $R_2$, A, B, D, E, G, Q, R, X, Y and Z is as defined above or below.

The invention also relates to a compound of the formula I, a salt thereof or an N-oxide thereof, wherein n is 0 and X is imino or thia, and any of r, m, $R_1$, $R_2$, A, B, D, E, G, Q, R, Y and Z is as defined above or below.

The prefix "lower" denotes a radical having up to and including a maximum of 7, especially up to and including a maximum of 4 carbon atoms, the radicals in question being either linear or branched with single or multiple branching.

Where the plural form is used for compounds, salts, and the like, this is taken to mean also a single compound, salt, or the like.

Any asymmetric carbon atoms (for example in compounds of formula I [or an N-oxide thereof], wherein n=1 and R is lower alkyl) may be present in the (R)-, (S)- or (R,S)-configuration, preferably in the (R)- or (S)- configuration. Substituents at a double bond or a ring may be present in cis (-Z-) or trans (-E-) form. The compounds may thus be present as mixtures of isomers or as pure isomers, preferably as enantiomer-pure diastereomers.

If $R_1$ and $R_2$ together form a bridge in subformula I*, the pertinent compound of formula I has formula IA (compounds of this formula are hereinbefore and hereinafter especially preferred when compounds of formula I are mentioned),

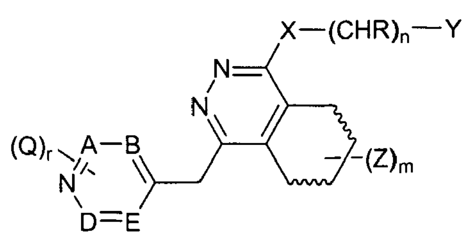

IA

wherein the radicals are as defined above for compounds of formula I. If $R_1$ and $R_2$ together form a bridge in subformula I**, the pertinent compound of formula I has formula IB,

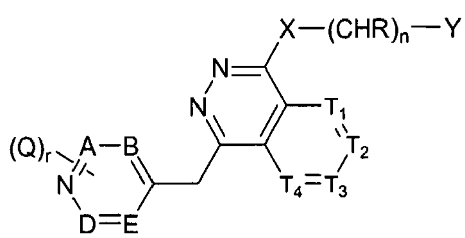

IB

wherein the radicals are as defined above for compounds of formula I.

Of the ring members $T_1$, $T_2$, $T_3$ , and $T_4$ , preferably only one is nitrogen, the remaining three being CH; preferably only $T_3$, especially $T_4$, is nitrogen, whereas the other ring members $T_1$, $T_2$, and $T_4$ or $T_1$, $T_2$, and $T_3$ are CH.

The index r is preferably 0 or 1.

The index n is preferably 0 or 1, especially 0.

The index m is preferably 0, 1, or 2, especially 0 or also 1.

Of ring members A, B, D, and E in formula I, not more than 2 are N, and the remaining ones are CH. Preferably,

each of the ring members A, B, D and E are CH.

If G is a bivalent group $-CH_2-O-$, $-CH_2-S-$, or $-CH_2-NH-$, the methylene group in each case is bound to the ring with ring members A, B, D r and E, whereas the heteroatom (O, S, or NH) is bound to the phthalazine ring in formula I.

Lower alkylene G may be branched or preferably linear and is especially branched or preferably linear $C_1$ -$C_4$ alkylene, especially methylene ($-CH_2-$), ethylene ($-CH_2-CH_2-$), trimethylene ($-CH_2-CH_2-CH_2-$) or tetramethylene ($-CH_2-CH_2-CH_2-CH_2-$). G is preferably methylene.

Acyl in lower alkylene substituted by acyloxy is preferably arylcarbonyloxy, wherein aryl is defined as below, especially benzoyloxy or lower alkanoyloxy, especially benzoyloxy; lower alkylene substituted by acyloxy is especially methylene substituted by benzoyloxy.

Lower alkylene substituted by hydroxy is preferably hydroxymethylene (-CH(OH)-).

G as lower alkylene substituted by acyloxy or hydroxy is preferred, or G as otherwise defined hereinbefore and hereinafter is in each case especially preferred.

Q is preferably bound to A or D (r=1) or to both (r=2), where in the event of binding of Q, A and/or D are/is C(-Q).

Lower alkyl is especially $C_1$ -$C_4$ alkyl, e.g. n-butyl, sec-butyl, tert-butyl, n-propyl, isopropyl, or especially methyl or also ethyl.

In the preferred embodiment, aryl is an aromatic radical having 6 to 14 carbon atoms, especially phenyl, naphthyl, fluorenyl or phenanthrenyl, the radicals defined above being unsubstituted or substituted by one or more, preferably up to three, especially one or two substituents, especially selected from amino, mono- or disubstituted amino, halogen, alkyl, substituted alkyl, hydroxy, etherified or esterified hydroxy, nitro, cyano, carboxy, esterified carboxy, alkanoyl, carbamoyl, N-mono- or N,N-disubstituted carbamoyl, amidino, guanidino, mercapto, sulfo, phenylthio, phenyl-lower alkylthio, alkylphenylthio, phenylsulfinyl, phenyl-lower alkylsulfinyl, alkylphenylsulfinyl, phenylsulfonyl, phenyl- lower alkylsulfonyl, and alkylphenylsulfonyl, or (as an alternative or in addition to the above group of substituents) selected from lower alkenyl, such as ethenyl, phenyl, lower alkylthio, such as methylthio, lower alkanoyl, such as acetyl, lower alkylmercapto, such as methylmercapto ($-S-CH_3$), halogen-lower alkylmercapto, such as trifluoromethylmercapto ($-S-CF_3$), lower alkylsulfonyl, halogen-lower alkylsulfonyl, such as especially trifluoromethane sulfonyl, dihydroxybora ($-B(OH)_2$), heterocyclyl, and lower alkylene dioxy bound at adjacent C-atoms of the ring, such as methylene dioxy; aryl is preferably phenyl which is either unsubstituted or independently substituted by one or two substituents selected from the group comprising amino; lower alkanoylamino, especially acetylamino; halogen, especially fluorine, chlorine, or bromine; lower alkyl, especially methyl or also ethyl or propyl; halogen-lower alkyl, especially trifluoromethyl; hydroxy; lower alkoxy, especially methoxy or also ethoxy; phenyl-lower alkoxy, especially benzyloxy; and cyano, or (as an alternative or in addition to the previous group of substituents) $C_8$ -$C_{12}$ alkoxy, especially n-decyloxy, carbamoyl, lower alkylcarbamoyl, such as n-methyl- or n-tert-butylcarbamoyl, lower alkanoyl, such as acetyl, phenyloxy, halogen-lower alkyloxy, such as trifluoromethoxy or 1,1,2,2-tetrafluoroethyloxy, lower alkoxycarbonyl, such as ethoxycarbonyl, lower alkylmercapto, such as methylmercapto, halogen-lower alkylmercapto, such as trifluoromethylmercapto, hydroxy-lower alkyl, such as hydroxymethyl or 1-hydroxymethyl, lower alkylsulfonyl, such as methane sulfonyl, halogen-lower alkylsulfonyl, such as trifluoromethane sulfonyl, phenylsulfonyl, dihydroxybora ($-B(OH)_2$), 2-methylpyrimidin-4-yl, oxazol-5-yl, 2-methyl-1,3-dioxolan-2-yl, 1H-pyrazol-3-yl, 1-methyl-pyrazol-3-yl and lower alkylene dioxy bound to two adjacent C-atoms, such as methylene dioxy.

Mono- or disubstituted amino is especially amino substituted by one or two radicals selected independently of one another from lower alkyl, such as methyl; hydroxy-lower alkyl, such as 2-hydroxyethyl; phenyl-lower alkyl; lower alkanoyl, such as acetyl; benzoyl; substituted benzoyl, wherein the phenyl radical is unsubstituted or especially substituted by one or more, preferably one or two, substituents selected from nitro or amino, or also from halogen, amino, N-lower alkylamino, N,N-di-lower alkylamino, hydroxy, cyano, carboxy, lower alkoxycarbonyl, lower alkanoyl, and carbamoyl; and phenyl-lower alkoxycarbonyl, wherein the phenyl radical is unsubstituted or especially substituted by one or more, preferably one or two, substituents selected from nitro or amino, or also from halogen, amino, N-lower alkylamino, N, N-di-lower alkylamino, hydroxy, cyano, carboxy, lower alkoxycarbonyl, lower alkanoyl, and carbamoyl; and is preferably N-lower alkylamino, such as N-methylamino, hydroxy-tower alkylamino, such as 2-hydroxyethylamino, phenyl-lower alkylamino, such as benzylamino, N,N-di-lower alkylamino, N-phenyl-lower alkyl-N-lower alkylamino, N,N-di-lower alkylphenylamino, lower alkanoylamino, such as acetylamino, or a substituent selected from the group comprising benzoylamino and phenyl-lower alkoxycarbonylamino, wherein the phenyl radical in each case is unsubstituted or especially substituted by nitro or amino, or also by halogen, amino, N-lower alkylamino, N,N-di-lower alkylamino, hydroxy, cyano, carboxy, lower alkoxycarbonyl, lower alkanoyl or carbamoyl, or as an alternative or in addition to the previous group of radicals by aminocarbonylamino.

Halogen is especially fluorine, chlorine, bromine, or iodine, especially fluorine, chlorine, or bromine and halo is especially fluoro, chloro, bromo, or iodo, especially fluoro, chloro, or bromo.

In the preferred embodiment, alkyl has up to a maximum of 12 carbon atoms and is especially lower alkyl, especially methyl, or also ethyl, n- propyl, isopropyl, or tert-butyl.

Substituted alkyl is alkyl as last defined, especially lower alkyl, preferably methyl, where one or more, especially

up to three, substituents may be present, primarily from the group selected from halogen, especially fluorine, and also from amino, N-lower alkylamino, N,N-di-lower alkylamino, N-lower alkanoylamino, hydroxy, cyano, carboxy, lower alkoxycarbonyl, and phenyl-lower alkoxycarbonyl. Trifluoromethyl is especially preferred.

Etherified hydroxy is especially $C_8$ -$C_{20}$ alkyloxy, such as n-decyloxy, lower alkoxy (preferred), such as methoxy, ethoxy, isopropyloxy, or n-pentyloxy, phenyl-lower alkoxy, such as benzyloxy, or also phenyloxy, or as an alternative or in addition to the previous group $C_8$ -$C_{20}$ alkyloxy, such as n-decyloxy, halogen-lower alkoxy, such as trifluoromethyloxy or 1,1,2,2-tetrafluoroethoxy.

Esterified hydroxy is especially lower alkanoyloxy, benzoyloxy, lower alkoxycarbonyloxy, such as tert-butoxycarbonyloxy, or phenyl-lower alkoxycarbonyloxy, such as benzyloxcarbonyloxy.

Esterified carboxy is especially lower alkoxycarbonyl, such as tert-butoxycarbonyl or ethoxycarbonyl, phenyl-lower alkoxycarbonyl, or phenyloxycarbonyl.

Alkanoyl is primarily alkylcarbonyl, especially lower alkanoyl, e.g. acetyl.

N-mono- or N,N-disubstituted carbamoyl is especially substituted by one or two substituents, lower alkyl, phenyl-lower alkyl, or hydroxy- lower alkyl, at the terminal nitrogen atom.

Alkylphenylthio is especially lower alkylphenylthio.

Alkylphenylsulfinyl is especially lower alkylphenylsulfinyl.

Alkylphenylsulfonyl is especially lower alkylphenylsulfonyl.

Pyridyl Y is preferably 3- or 4-pyridyl.

Z is preferably amino, hydroxy-lower alkylamino, such as 2-hydroxyethylamino, lower alkanoylamino, such as acetylamino, nitrobenzoylamino, such as 3-nitrobenzoylamino, aminobenzoylamino, such as 4-aminobenzoylamino, phenyl-lower alkoxycarbonylamino, such as benzyloxycarbonylamino, or halogen, such as bromine; preferably only one substituent is present (m=1), especially one of the last mentioned, especially halogen. A compound of formula I (or an N-oxide thereof), wherein Z is absent (m=0), is especially preferred.

Unsubstituted or substituted cycloalkyl is preferably $C_3$ -$C_8$ cycloalkyl, which is unsubstituted or substituted in the same way as aryl, especially as defined for phenyl. Cyclohexyl or also cyclopentyl or cyclopropyl are preferred.

Heterocyclyl is especially a five or six-membered heterocyclic system with 1 or 2 heteroatoms selected from the group comprising nitrogen, oxygen, and sulfur, which may be unsaturated or wholly or partly saturated, and is unsubstituted or substituted especially by lower alkyl, such as methyl; a radical selected from 2-methylpyrimidin-4-yl, oxazol-5- yl, 2-methyl-1,3-dioxolan-2-yl, 1H-pyrazol-3-yl, and 1-methyl-pyrazol-3- yl is preferred.

Aryl in the form of phenyl which is substituted by lower alkylene dioxy bound to two adjacent C-atoms, such as methylenedioxy, is preferably 3,4-methylenedioxyphenyl.

The bonds in formula I characterized by wavy lines are present either as single or as double bonds. Preferably both are at the same time either single or double bonds.

An N-oxide of a compound of formula I is preferably an N-oxide in which a phthalazine-ring nitrogen or a nitrogen in the ring with ring members A, B, D, and E carries an oxygen atom, or several of the said nitrogen atoms carry an oxygen atom.

Salts are especially the pharmaceutically acceptable salts of compounds of formula I (or an N-oxide thereof).

Such salts are formed, for example, as acid addition salts, preferably with organic or inorganic acids, from compounds of formula I (or an N- oxide thereof with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, 2-hydroxybutyric acid, gluconic acid, glucosemonocarboxylic acid, fumaric acid, succinic acid, adipic acid, pimelic acid, suberic acid, azelaic acid, malic acid, tartaric acid, citric acid, glucaric acid, galactaric acid, amino acids, such as glutmic acid, asparic acid, N-methylglycine, acetylaminoacetic acid, N-acetylasparagine or N-acetylcysteine, pyruvic acid, acetoacetic acid, phosphoserine, 2- or 3-glycerophosphoric acid, glucose-6-phosphonc acid, glucose- -phosphoric acid, fructose-1,6-bis- phosphoric acid, maleic acid, hydroxymaleic acid, methylmaleic acid, cyclohexanecarboxylic acid, adamantanecarboxylic acid, benzoic acid, salicylic acid, 1- or 3-hydroxynaphthyl-2-carboxylic acid, 3,4,5- trimethoxybenzoic acid, 2-phenoxybenzoic acid, 2-acetoxybenzoic acid, 4- aminosalicylic acid, phthalic acid, phenylacetic acid, mandelic acid, cinnamic acid, glucuronic acid, galacturonic acid, methane- or ethane- sulfonic acid, 2-hydroxyethanesulfonic acid, ethane-1,2-disutfonic acid, benzenesulfonic acid, 2-naphthalenesultonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl-or N-propyl-sulfamic acid, or other organic protonic acids, such as ascorbic acid.

In the presence of negatively charged radicals, such as carboxy or sulfo, salts may also be formed with bases, e.g. metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri(2-hydroxyethyl)amine, or heterocyclic bases, for example N-ethylpipperidine or N, N'-dimethylpiperazine.

When a basic group and an acid group are present in the same molecule, a compound of formula I (or an N-oxide thereof) may also form internal salts.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient.

The compounds of formula I (or an N-oxide thereof have valuable pharmacological properties, as described hereinbefore and hereinafter.

With the groups of preferred compounds of formula I mentioned hereinafter, definitions of substituents from the general definitions mentioned hereinbefore may reasonably be used, for example, to replace more general definitions with more specific definitions or especially with definitions characterized as being preferred;

(A) Preference is given to a compound of formula I comprised in a pharmaceutical preparation or to be used according to the invention wherein
r is 0 to 2, preferably 0,
n is 0 or 1,
m is 0 or also 1,
$R_1$ and $R_2$ (i) are lower alkyl, especially methyl, or

(ii) together form a bridge in subformula I*

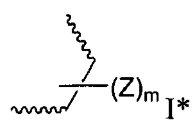

the binding being achieved via the two terminal carbon atoms, or
(iii) together form a bridge in subformula I**

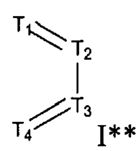

wherein one of the ring members $T_1$, $T_2$, $T_3$ and $T_4$ is nitrogen, and the others are in each case CH, and the binding is achieved via $T_1$ and $T_4$;

A, B, D, and E are in each case CH, or also A, D, and E are each CH and B is N;
G is lower alkylene, especially methylene or ethylene ($-CH_2-CH_2-$), $-CH_2-NH-$, $-CH_2-O-$, hydroxymethylene, or benzoyloxymethylene,
Q is methyl, which is bound to A, D, or to A and D;
R is H or lower alkyl, especially H or methyl,
X is imino, oxa, or thia,
Y is phenyl, which is unsubstituted or is substituted by one or two substituents independently of one another from the group comprising amino; lower alkanoylamino, especially acetylamino; halogen, especially fluorine, chlorine, or bromine; lower alkyl, especially methyl or also ethyl or propyl; halogen-lower alkyl, especially trifluoromethyl; hydroxy; lower alkoxy, especially methoxy or also ethoxy; phenyl-lower alkoxy, especially benzyloxy; and cyano, or (as an alternative or in addition to the previous group of substituents) lower alkenyl, such as ethenyl, $C_8$ -$C_{12}$ alkoxy, especially n-decyloxy, lower alkoxycarbonyl, such as tert-butoxycarbonyl, carbamoyl, lower alkylcarbamoyl, such as N-methyl- or N-tert-butylcarbamoyl, lower alkanoyl, such as acetyl, phenyloxy, halogen- lower alkyloxy, such as trifluoromethoxy or 1,1,2,2-tetrafluoroethyloxy, lower alkoxycarbonyl, such as ethoxycarbonyl, lower alkylmercapto, such as methylmercapto, halogen-lower alkylmercapto, such as trifluoromethylmercapto, hydroxy-lower alkyl, such as hydroxymethyl or 1-hydroxymethyl, lower alkylsulfonyl, such as methanesulfonyl, halogen- lower alkylsulfonyl, such as trifluoromethanesulfonyl, phe-

nylsulfonyl, dihydroxybora (-$B(OH)_2$), 2-methylpyrimidin-4-yl, oxazol-5-yl, 2-methyl- 1,3-dioxolan-2-yl, 1H-pyrazol-3-yl, 1-methylpyrazol-3-yl and lower alkylenedioxy bound to two adjacent C-atoms, such as methylenedioxy, or is also pyridyl, especially 3-pyridyl; especially phenyl, 2-, 3- or 4- aminophenyl, 2-, 3- or 4-acetylaminophenyl, 2-, 3- or 4-fluorophenyl, 2-, 3- or 4-chlorophenyl, 2-, 3- or 4-bromophenyl, 2,3-, 2,4-, 2,5- or 3,4- dichlorophenyl, chlorofluorophenyl, such as 3-chloro-4-fluorophenyl or also 4-chloro-2-fluoroanilino, 2-, 3- or 4-methylphenyl, 2-, 3- or 4-ethylphenyl, 2-, 3-or 4-propylphenyl, methylfluorophenyl, such as 3-fluoro-4-methylphenyl, 2-, 3- or 4-trifluoromethylphenyl, 2-, 3- or 4-hydroxyphenyl, 2-, 3- or 4-methoxyphenyl, 2-, 3- or 4-ethoxyphenyl, methoxychlorophenyl, such as 3-chloro-4-methoxycarbonyl, 2-, 3- or 4- benzyloxyphenyl, 2-, 3- or 4-cyanophenyl, or also 2-, 3- or 4-pyridyl;

Z is amino; N-lower alkylamino, such as N-methylamino; hydroxy-lower alkylamino, such as 2-hydroxyethylamino; phenyl-lower alkylamino, such as benzylamino; N,N-di-lower alkylamino; n-phenyl-lower alkyl-N-lower alkylamino; N,N-di-lower alkylphenylamino; lower alkanoylamino, such as acetylamino; or a substituent from the group comprising benzoylamino or phenyl-lower alkoxycarbonylamino, wherein the phenyl radical in each case is unsubstituted or especially substituted by nitro or amino, or also by halogen, amino, N-lower alkylamino, N,N-di-lower alkylamino, hydroxy, cyano, carboxy, lower alkoxycarbonyl, lower alkanoyl or carbamoyl; or is halogen, especially bromine; especially amino, acetylamino, nitrobenzoylamino, aminobenzoylamino, 2-hydroxyethylamino, benzyloxycarbonylamino or bromine; and,

if present (in formula IA), the bonds characterized by a wavy line are in each case a double bond or in each case a single bond;

or of a pharmaceutically acceptable salt thereof; or to such a compound or pharmaceutically acceptable salt thereof wherein n=0 and the other moieties are as defined under (A) for use in the treatment of a disease mentioned hereinbefore or hereinafter; or to such a compound wherein n=0 and X is thia or is imino, and the other moieties are as defined under (A), or a pharmaceutically acceptable salt thereof.

(B) Special preference is given to a compound of formula I, especially formula IA, comprised in a pharmaceutical preparation or to be used according to the invention wherein

r is 0;

n is 0 or 1,

m is 0;

A, B, 0, and E are in each case CH,

G is lower alkylene, especially methylene,

R is H,

X is imino,

Y is phenyl, which is unsubstituted or is substituted by one or two substituents independently of one another selected from the group comprising amino; lower alkanoylamino, especially acetylamino; halogen, especially fluorine, chlorine, or bromine; lower alkyl, especially methyl; halogen-lower alkyl, especially trifluoromethyl; hydroxy; lower alkoxy, especially methoxy; phenyl-lower alkoxy, especially benzyloxy; and cyano; especially phenyl, 2-, 3- or 4-aminophenyl, 2-, 3- or 4-acetylaminophenyl, 2-, 3- or 4-fluorophenyl, 2-, 3- or 4-chlorophenyl, 2-, 3- or 4-bromophenyl, 2,3-, 2,4-, 2,5- or 3,4-dichlorophenyl, chlorofluorophenyl, such as 3-chloro-4-fluorophenyl, 2,-3- or 4-methylphenyl, 2-, 3- or 4- trifluoromethylphenyl, 2-, 3- or 4-hydroxyphenyl, 2-, 3- or 4- methoxycarbonyl, methoxychlorophenyl, such as 3-chloro-4-methoxycarbonyl, 2-, 3- or 4-benzyloxyphenyl, or 2-, 3- or 4-cyanophenyl; and

the bonds characterized by a wavy line are double bonds;

or a pharmaceutically acceptable salt thereof; or to such a compound of formula I, especially IA, wherein n=0 and the other moieties are as defined under (B), or a salt thereof.

Special preference is given to a compound of formula I, especially formula IA, such as is mentioned in the Examples below, or a pharmaceutically acceptable salt thereof, especially a compound specifically mentioned in the Examples or a salt thereof.

High preference is given to a compound selected from

1-(4-Chloroanilino)-4-(4-pyridylmethyl)phthalazine;

1-(3-Chloroanilino)-4-(4-pyridylmethyl)phthalazine;

1-Anilino-4-(4-pyridylmethyl)phthalazine;

1-Benzylamino-4-(4-pyridylmethyl)phthalazine;

1-(4-Methoxyanilino)-4-(4-pyridylmethyl)phthalazine;

1-(3-Benzyloxyanilino)-4-(4-pyridylmethyl)phthalazine;

1-(3-Methoxyanilino)-4-(4-pyridylmethyl)phthalazine;

1-(2-Methoxyanilino)-4-(4-pyridylmethyl)phthalazine;

1-(4-Trifluoromethylanilino)-4-(4-pyridy=methyl)phthalazine;

1-(4-Fluoroanilino)-4-(4-pyridylmethyl)phthalazine;

1-(3-Hydroxyanilino)-4-(4-pyridyl methyl)phthalazine;

1-(4-Hydroxyanilino)-4-(4-pyridylmethyl)phthalazine;

1-(3-Aminoanilino)-4-(4-pyridylmethyl)phthalazine;

1-(3,4-Dichloroanilino)-4-(4-pyridylmethyl)phthalazine;
1-(4-Bromoanilino)-4-(4-pyridylmethyl)phthalazine;
1-(3-Chloro-4-methoxyanilino)-4-(4-pyridylmethyl)phthalazine;
1-(4-Cyanoanilino)-4-(4-pyridylmethyl)phthalazine;
1-(4-Methylanilino)-4-(4-pyridylmethyl)phthalazine; and also
1-(3-Chloro-4-fluoroanilino)-4-(4-pyridylmethyl)phthalazine;
1-(3-Methylanilino)-4-(4-pyridylmethyl)phthalazine;
or a pharmaceutically acceptable salt thereof.

It is contemplated that the invention encompasses all possible combinations of the embodiments described herein.

Preferred SMIPs in accordance with the fourth aspect of the invention are derivatized pyridazines, such as 1-(4-Chloroanilino)-4-(4-pyridylmethyl)phthalazine, as well as analogs disclosed in US 6,258,812. More particularly, preferred SMIPs include derivatized pyridazines encompassed by Formula I as described in this section, or contained within the aforementioned references.

Reference to "derivatized pyridazines" indicates compounds having the general structure of Formula I as described in this section. A preferred derivatized pyridazine is a phthalazine such as 1-(4-Chloroanilino)-4-(4-pyridylmethyl)phthalazine.

The foregoing may be better understood by reference to the Examples, *infra,* that are presented for illustration and not to limit the scope of the inventive concepts. The Example compounds and their analogs are either commercially available, or are easily synthesized by one skilled in the art from procedures described in patents or patent applications listed herein.

**Section V - Fifth Aspect of the Invention - Staurosporine Analogs for Immunopotentiation**

All definitions, descriptors of constituent variables for chemical formulas, and descriptions appearing in the section shall be understood to apply to this section only.

In accordance with the fifth aspect of the invention, Applicants have discovered methods of stimulating cytokine activity in cells and immunotherapeutics and/or vaccine adjuvants, that will provide effective treatments for disorders described herein and those apparent to one skilled in the art.

In one embodiment of the invention, a method of modulating an immune response in a subject is provided, comprising administering a compound of formula I:

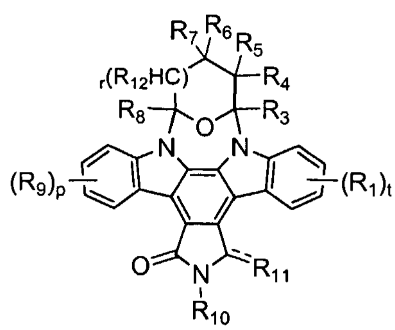

wherein,
$R_1$ and $R_9$ are each independently H, -CN, -OH, halogen, alkyl, aryl, alkoxy, $-NR_aR_b$, $-C(O)R_c$, $-S(O)_nR_d$, or heterocyclyl;
$R_3$ and $R_8$ are each independently H, -CN, -OH, halogen, alkyl, aryl, alkoxy, $-NR_aR_b$, $-C(O)R_c$, $-S(O)_nR_d$, or heterocyclyl;
$R_4$ and $R_7$ are each independently H, -CN, -OH, halogen, alkyl, $-O-(CH_2)_q-R_e$, $- C(O)R_c$, $-O-(CH_2)_q-O-R_e$, $-NR_aR_b$, and $-S(O)_nR_d$;
$R_5$ and $R_6$ are each independently H, -CN, -OH, halogen, alkyl, aryl, alkoxy, $-NR_aR_b$, $-C(O)R_c$, or heterocyclyl;
$R_{10}$ is H, alkyl, aryl, $-C(O)R_c$, or heterocyclyl;
the dotted line is absent and $R_{11}$ is H, -CN, -OH, halogen, alkyl, aryl, alkoxy, $-NR_aR_b$, $-C(O)R_c$, $-S(O)_nR_d$, or heterocyclyl; or the dotted line is present and $R_{11}$ is O or S;
$R_{12}$ is H, alkyl, or alkoxy;
each $R_a$ and $R_b$ is independently H, alkyl, $-C(O)R_c$, aryl, heterocyclyl, or alkoxy; each $R_c$ is independently H, alkyl, alkoxy, $-NH_2$, -NH(alkyl), $-N(alkyl)_2$, aryl, or heterocyclyl;
each $R_d$ is independently H, alkyl, alkenyl, aryl, or $-NR_aR_b$;
each $R_e$ is independently H, halogen, $-C(O)R_c$, aryl, heterocyclyl, or $-NR_aR_b$;
each n is independently 0, 1, or 2;

each p is independently 0, 1, 2, or 3;
each q is independently 0, 1, or 2;
each r is 0, thereby forming a covalent bond, or 1; and
each t is independently 0, 1, 2 or 3.

In another embodiment, $R_1$ and $R_9$ are both H.

In another more particular embodiment, $R_{10}$ is H.

In another more particular embodiment, $R_3$ is methyl.

In another more particular embodiment, r is 1 and $R_{12}$ is H.

In another more particular embodiment, $R_4$ is $-OCH_3$ and $R_5$ is H.

In another more particular embodiment, $R_6$ is H and $R_7$ is $-NR_aR_b$.

In another more particular embodiment, $R_4$ is $-OCH_3$, $R_5$ is H, $R_6$ is H and $R_7$ is $-NR_aR_b$.

In another more particular embodiment, $R_a$ within $R_7$ is methyl and $R_b$ within $R_7$ is H.

In another more particular embodiment, $R_{11}$ is -H.

In another more particular embodiment, $R_{11}$ is -OH.

In another more particular embodiment, $R_{11}$ is =O.

In another more particular embodiment, r is 0.

In another more particular embodiment, $R_5$ is -OH.

In another more particular embodiment, $R_4$ is $-CH_2OH$.

In another more particular embodiment, $R_4$ is $-C(O)R_c$.

Another embodiment of the invention provides a method of modulating an immune response in a subject comprising administering a compound selected from the group consisting of:

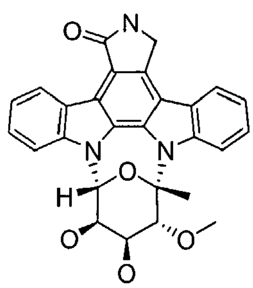
,
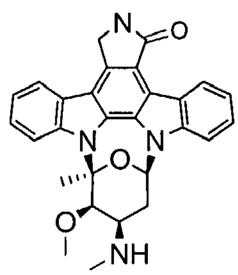
,
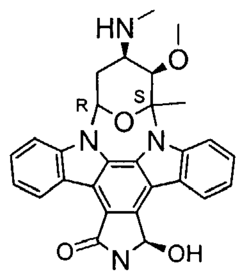
,
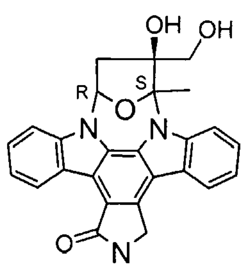
, and
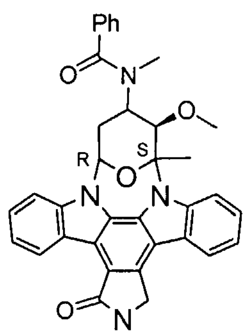
.

In a more particular embodiment of any of the above methods, said modulating is inducing. In another embodiment said inducing stimulates production of cytokines, chemokines, and/or growth factors.

In another embodiment said compound is administered in a subcytotoxic amount to said subject.

In another embodiment said subject is in remission from cancer.

In another embodiment said compound is administered for the treatment of refractory cancer cells.

In another embodiment said compound is administered metronomically.

In another embodiment the subject is not suffering from cancer.

In another embodiment said compound is co-administered with another agent.

In another embodiment said compound is administered in a dose capable of increasing TNF-$\alpha$ levels.

In another embodiment said compound has an average steady state drug concentration in the blood of less

than 20 μM.

In another embodiment subject is suffering from an autoimmune disease. Further still, said autoimmune disease is multiple sclerosis or Crohn's disease.

In another embodiment said subject is suffering from a viral infection.

In another embodiment said viral infection is HCV, HIV, or HSV.

In another embodiment said subject is suffering from allergies.

In another embodiment said subject is suffering from asthma.

In another embodiment the subject is suffering from a disease associated with abnormal cellular proliferation, such as, neuro-fibromatosis, atherosclerosis, pulmonary fibrosis, arthritis, psoriasis, glomerulonephritis, restenosis, proliferative diabetic retinopathy (PDR), hypertrophic scar formation, inflammatory bowel disease, transplantation rejection, angiogenesis, or endotoxic shock.

Any asymmetric carbon atoms (for example in compounds of formula I [or an N-oxide thereof], wherein n=1 and R is lower alkyl) may be present in the (R)-, (S)- or (R,S)-configuration, preferably in the (R)- or (S)- configuration. Substituents at a double bond or a ring may be present in cis (-Z-) or trans (-E-) form. The compounds may thus be present as mixtures of isomers or as pure isomers, preferably as enantiomer-pure diastereomers.

It is contemplated that the invention encompasses all possible combinations of the embodiments described herein.

Preferred SMIPs in accordance with the fifth aspect of the invention are staurosporine analogs, such as 9,13-Epoxy-1H,9H-diindolo[1,2,3-gh:3',2',1'-lm]pyrrolo[3,4j][1,7]benzodiazonin-1-one, 2,3,10,11,12,13-hexahydro-11,12-dihydroxy-10-methoxy-9-methyl; 9,13-Epoxy-1H,9H-diindolo[1,2,3-gh:3',2',1'-lm]pyrrolo[3,4-j][1,7]benzodiazonin-1-one, 2,3,10,11,12,13-hexahydro-10-methoxy-9-methyl-11-(methylamino); 9,13-Epoxy-1H,9H-diindolo[1,2,3-gh:3',2',1'-lm] pyrrolo[3,4-j][1,7]benzodiazonin-1-one, 2,3,10,11,12,13-hexahydro-3-hydroxy-10-methoxy-9-methyl-11-(methylamino); 9,12-Epoxy-1H-diindolo[1,2,3-fg:3',2',1'-kl]pyrrolo[3,4-1][1,6]benzodiazocin-1-one, 2,3,9,10,11,12-hexahydro-10-hydroxy-10-(hydroxymethyl)-9-methyl; and benzamide, N-[(9S,10R,11R,13R)-2,3,10,11,12,13-hexahydro-10-methoxy-9-methyl-1-oxo-9,13-epoxy-1H,9H-diindolo[1,2,3-gh:3',2',1'-lm]pyrrolo[3,4-j][1,7]benzodiazonin-11-yl]-N-methyl, as well as analogs disclosed in the following patents and patent applications: WO 02/30941, WO 97/07081, WO 89/07105, US 5,621,100, WO 93/07153, WO 89/07105, WO 01/04125, WO 02/30941, WO 93/08809, WO 94/06799 WO 00/27422, WO 96/13506, and WO 88/07045. More particularly, preferred SMIPs include flavones, isoflavones and those encompassed by Formula I as described in this section, or contained within the aforementioned references.

Reference to "staurosporine analogs" indicates compounds having the general structure of Formula I as described in this section. Preferred staurosporine analogs are selected from the group consisting of 9,13-Epoxy-1H,9H-diindolo[1,2,3-gh:3',2',1'-lm]pyrrolo[3,4j][1,7]benzodiazonin-1-one, 2,3,10,11,12,13-hexahydro-11,12-dihydroxy-10-methoxy-9-methyl; 9,13-Epoxy-1H,9H-diindolo[1,2,3-gh:3',2',1,-lm]pyrrolo[3,4-j][1,7]benzodiazonin-1-one, 2,3,10,11, 12,13-hexahydro-10-methoxy-9-methyl-11-(methylamino); 9,13-Epoxy-1H,9H-diindolo[1,2,3-gh:3',2',1'-lm]pyrrolo[3,4-j][1,7]benzodiazonin-1-one, 2,3,10,11,12,13-hexahydro-3-hydroxy-10-methoxy-9-methyl-11-(methylamino); 9,12-Epoxy-1H-diindolo[1,2,3-fg:3',2',1'-kl]pyrrolo[3,4-i][1,6]benzodiazocin-1-one, 2,3,9,10,11,12-hexahydro-10-hydroxy-10-(hydroxymethyl)-9-methyl; and benzamide, N-[(9S,10R, 11R,13R)-2,3,10,11,12,13-hexahydro-10-methoxy-9-methyl-1-oxo-9,13-epoxy-1H,9H-diindolo[1,2,3-gh:3',2',1'-lm]pyrrolo[3,4-j][1,7]benzodiazonin-11-yl]-N-methyl, as well as analogs disclosed in the following patents and patent applications: WO 02/30941, WO 97/07081, WO 89/07105, US 5,621,100, WO 93/07153, WO 89/07105, WO 01/04125, WO 02/30941, WO 93/08809, WO 94/06799 WO 00/27422, WO 96/13506, and WO 88/07045. Subsequently, it will become apparent to the skilled artisan that not only 9,13-epoxy analogs are encompassed by the term "staurosporine analogs", but additionally homologs, such as 9,12-epoxy compounds, for example K-252, are encompassed as well.

The foregoing may be better understood by reference to the Examples, *infra*, that are presented for illustration and not to limit the scope of the inventive concepts. The Example compounds and their analogs are either commercially available, or are easily synthesized by one skilled in the art from procedures described in patents or patent applications listed herein.

**Section VI - Sixth Aspect of the Invention - Nucleoside Analogs for Immunopotentiation**

All definitions, descriptors of constituent variables for chemical formulas, and descriptions appearing in the section shall be understood to apply to this section only.

In accordance with the sixth aspect of the invention, Applicants have discovered methods of stimulating cytokine activity in cells and immunotherapeutics and/or vaccine adjuvants, that will provide effective treatments for disorders described herein and those apparent to one skilled in the art.

One embodiment of the invention provides a method of treating a subject suffering from a viral infection comprising administering a small molecule in an amount such that said small molecule concomitantly inhibits viral replication while stimulating an immune response in said subject. In another embodiment thereof said small molecule is a nucleoside analog. In another embodiment thereof said small molecule inhibits viral DNA polymerase. In another embodiment thereof said small molecule is a nucleoside analog. In another embodiment said small molecule is a substituted purine

or pyrimidine.

Another embodiment of the invention provides a method of stimulating an immune response in a subject comprising administering a nucleoside analog of formula I:

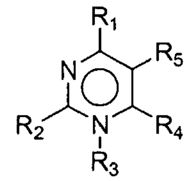

I

wherein,

$R_1$ and $R_2$ are each independently H, halo, $-NR_aR_b$, =O $C_{1-6}$ alkoxy, substituted $C_{1-6}$ alkoxy, heterocyclyl, substituted heterocyclyl, $C_{6-10}$ aryl, substituted $C_{6-10}$ aryl, $C_{1-6}$ alkyl, or substituted $C_{1-6}$ alkyl;

$R_3$ is absent, H, $C_{1-6}$ alkyl, substituted $C_{1-6}$ alkyl, $C_{6-10}$ aryl, substituted $C_{6-10}$ aryl, heterocyclyl, or substituted heterocyclyl;

$R_4$ and $R_5$ are each independently H, halo, heterocyclyl, substituted heterocyclyl,

$-C(O)-R_d$, $C_{1-6}$ alkyl, substituted $C_{1-6}$ alkyl, or bound together to form a 5 membered ring as in $R_{4-5}$:

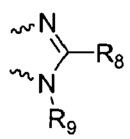

$R_{4-5}$

the binding being achieved at the bonds indicated by a ~ ;

$R_8$ is H, halo, $C_{1-6}$ alkyl, $C_{2-6}$ alkenyl, $C_{2-6}$ alkynyl, -OH, $-NR_aR_b$, $-(CH_2)_n-O-R_c$, $-O-(C_{1-6}$ alkyl), $-S(O)_pR_c$, or $-C(O)-R_d$;

$R_9$ is H, $C_{1-6}$ alkyl, substituted $C_{1-6}$ alkyl, heterocyclyl, or substituted heterocyclyl; each $R_a$ and $R_b$ is independently H, $C_{1-6}$ alkyl, substituted $C_{1-6}$ alkyl, $-C(O)R_d$, or $C_{6-10}$ aryl;

each $R_c$ is independently H, phosphate, diphosphate, triphosphate, $C_{1-6}$ alkyl, or substituted $C_{1-6}$ alkyl;

each $R_d$ is independently H, halo, $C_{1-6}$ alkyl, substituted $C_{1-6}$ alkyl, $C_{1-6}$ alkoxy, substituted $C_{1-6}$ alkoxy, $-NH_2$, $-NH(C_{1-6}$ alkyl), -NH(substituted $C_{1-6}$ alkyl), $-N(C_{1-6}$ alkyl$)_2$, -N(substituted $C_{1-6}$ alkyl$)_2$, $C_{6-10}$ aryl, or heterocyclyl;

each $R_e$ is independently H, $C_{1-6}$ alkyl, substituted $C_{1-6}$ alkyl, $C_{6-10}$ aryl, substituted $C_{6-10}$ aryl, heterocyclyl, or substituted heterocyclyl;

each $R_f$ is independently H, $C_{1-6}$ alkyl, substituted $C_{1-6}$ alkyl, $-C(O)R_d$, phosphate, diphosphate, or triphosphate;

each n is independently 0, 1, 2, or 3;

each p is independently 0, 1, or 2; or

a pharmaceutically acceptable salt thereof, a tautomer thereof, or a pharmaceutically acceptable salt of the tautomer; with the stipulation that if $R_{4-5}$ is present, $R_3$ is H or absent.

In another embodiment of formula I, $R_1$ is $-NH_2$.

In another embodiment of formula I, each $R_f$ is H.

In another embodiment of formula I, $R_2$ is =O.

In another embodiment of formula I, $R_3$ is $-(CH_2)_n-O-R_c$. In still another embodiment thereof, $R_3$ is $-CH_2-O-(C_2$ alkyl or substituted $C_2$ alkyl)-O-(H, phosphate, diphosphate, triphosphate, $C_{1-6}$ alkyl or substituted $C_{1-6}$ alkyl).

In another embodiment of formula I, $R_3$ is $R_{3a}$:

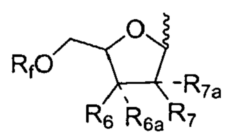

$R_{3a}$

the binding being achieved at the bond indicated by a ;
$R_6$ and $R_7$ are each independently H, halo, $C_{1-6}$ alkoxy, substituted $C_{1-6}$ alkoxy, $C_{2-6}$ alkenyl, substituted $C_{2-6}$ alkenyl, $-NR_aR_b$, $-N_3$, or -OH;
$R_{6a}$ and $R_{7a}$ are each independently H, halo, or $C_{1-6}$ alkyl;
each $R_a$ and $R_b$ is independently H, $C_{1-6}$ alkyl, substituted $C_{1-6}$ alkyl, $-C(O)R_d$, or $C_{6-10}$ aryl;
each $R_d$ is independently H, halo, $C_{1-6}$ alkyl, substituted $C_{1-6}$ alkyl, $C_{1-6}$ alkoxy, substituted $C_{1-6}$ alkoxy, $-NH_2$, $-NH(C_{1-6}$ alkyl), -NH(substituted $C_{1-6}$ alkyl), $-N(C_{1-6}$ alkyl$)_2$, -N(substituted $C_{1-6}$ alkyl$)_2$, $C_{6-10}$ aryl, or heterocyclyl; and $R_f$ is independently H, $C_{1-6}$ alkyl, substituted $C_{1-6}$ alkyl, $-C(O)R_d$, phosphate, diphosphate, or triphosphate.

In another embodiment wherein $R_3$ is $R_{3a}$, $R_6$ is -OH and $R_7$ is -CH=CHF.

In another embodiment wherein $R_3$ is $R_{3a}$, $R_2$ is =O. In still another embodiment wherein $R_3$ is $R_{3a}$ and $R_2$ is =O, $R_4$ and $R_5$ are both H. In still another embodiment wherein $R_3$ is $R_{3a}$ and $R_2$ is =O, $R_1$ is $-NH_2$. In still another embodiment wherein $R_3$ is $R_{3a}$ and $R_2$ is =O, $R_f$ is H.

In another embodiment wherein $R_3$ is $R_{3a}$, $R_2$ is =O, and $R_f$ is H, $R_6$ and $R_7$ are both H.

In another embodiment wherein $R_3$ is $R_{3a}$, $R_2$ is =O, and $R_f$ is H, $R_6$ is - OH. In still another embodiment thereof $R_7$ is H, F, or -OH.

In another embodiment wherein $R_3$ is $R_{3a}$, $R_2$ is =O, and $R_f$ is H, $R_6$ is - $N_3$.

In another embodiment wherein $R_3$ is $R_{3a}$, $R_{6a}$ and $R_{7a}$ are both H.

In another embodiment wherein $R_3$ is $R_{3a}$, both $R_7$ and $R_{7a}$ are methyl.

In another embodiment of formula I, said substituted heterocyclyl within $R_9$ is $R_{9a}$:

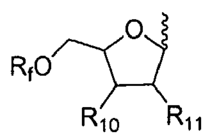

the binding being achieved at the bond indicated by a ;
$R_{10}$ and $R_{11}$ are each independently H, halo, $C_{1-6}$ alkoxy, substituted $C_{1-6}$ alkoxy, - $NR_aR_b$, or -OH;
$R_a$ and $R_b$ are independently H, $C_{1-6}$ alkyl, substituted $C_{1-6}$ alkyl, $-C(O)R_d$, or $C_{6-10}$ aryl;
$R_d$ is H, halo, $C_{1-6}$ alkyl, substituted $C_{1-6}$ alkyl, $C_{1-6}$ alkoxy, substituted $C_{1-6}$ alkoxy, - $NH_2$, $-NH(C_{1-6}$ alkyl), -NH(substituted $C_{1-6}$ alkyl), $-N(C_{1-6}$ alkyl$)_2$, -N(substituted $C_{1-6}$ alkyl$)_2$, $C_{6-10}$ aryl, or heterocyclyl; and
$R_f$ is independently H, $C_{1-6}$ alkyl, substituted $C_{1-6}$ alkyl, $-C(O)R_d$, phosphate, diphosphate, or triphosphate.

Another embodiment of the invention provides a method of stimulating an immune response in a subject comprising administering a nucleoside analog of formula II:

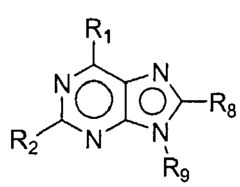

II

wherein,
$R_1$ and $R_2$ are each independently H, halo, $-NR_aR_b$, =O $C_{1-6}$ alkoxy, substituted $C_{1-6}$ alkoxy, heterocyclyl, substituted heterocyclyl, $C_{6-10}$ aryl, substituted $C_{6-10}$ aryl, $C_{1-6}$ alkyl, or substituted $C_{1-6}$ alkyl;
$R_8$ is H, halo, $C_{1-6}$ alkyl, $C_{2-6}$ alkenyl, $C_{2-6}$ alkynyl, heterocyclyl, substituted heterocyclyl, -OH, $-NR_aR_b$, $-(CH_2)_n-O-R_c$, $-O-(C_{1-6}$ alkyl), $-S(O)_pR_e$, or $-C(O)-R_d$; $R_9$ is H, $C_{1-6}$ alkyl, substituted $C_{1-6}$ alkyl, heterocyclyl, or $R_{9a}$:

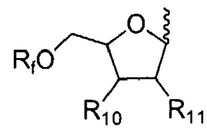

$R_{9a}$

the binding being achieved at the bond indicated by a $\sim$ ;

$R_{10}$ and $R_{11}$ are each independently H, halo, $C_{1-6}$ alkoxy, substituted $C_{1-6}$ alkoxy, - $NR_aR_b$, or -OH;

each $R_a$ and $R_b$ is independently H, $C_{1-6}$ alkyl, substituted $C_{1-6}$ alkyl, -C(O)$R_d$, or $C_{6-10}$ aryl;

each $R_c$ is independently H, phosphate, diphosphate, triphosphate, $C_{1-6}$ alkyl, substituted $C_{1-6}$ alkyl;

each $R_d$ is independently H, halo, $C_{1-6}$ alkyl, substituted $C_{1-6}$ alkyl, $C_{1-6}$ alkoxy, substituted $C_{1-6}$ alkoxy, $-NH_2$, -NH($C_{1-6}$ alkyl), -NH(substituted $C_{1-6}$ alkyl), -N($C_{1-6}$ alkyl)$_2$, -N(substituted $C_{1-6}$ alkyl)$_2$, $C_{6-10}$ aryl, or heterocyclyl;

each $R_e$ is independently H, $C_{1-6}$ alkyl, substituted $C_{1-6}$ alkyl, $C_{6-10}$ aryl, substituted $C_{6-10}$ aryl, heterocyclyl, or substituted heterocyclyl;

each $R_f$ is independently H, $C_{1-6}$ alkyl, substituted $C_{1-6}$ alkyl, -C(O)$R_d$, phosphate, diphosphate, or triphosphate;

each n is independently 0, 1, 2, or 3; and

each p is independently 0, 1, or 2.

In another embodiment of formula II, $R_9$ is $-(CH_2)_n-O-R_c$. In still another embodiment thereof, $R_9$ is $-CH_2-O-(C_2$ alkyl or substituted $C_2$ alkyl)-O-(H, phosphate, diphosphate, triphosphate, $C_{1-6}$ alkyl or substituted $C_{1-6}$ alkyl).

In another embodiment of formula II, $R_2$ is $-NH_2$.

In another embodiment of formula II, $R_1$ is =O.

In another embodiment of formula II, $R_1$ is $-NH_2$.

In another embodiment of formula II, $R_8$ is H.

In another embodiment of formula II, $R_9$ is $R_{9a}$ and $R_f$ is H. In still another embodiment wherein $R_9$ is $R_{9a}$ and $R_f$ is H, $R_{10}$ and $R_{11}$ are both H. In still another embodiment wherein $R_9$ is $R_{9a}$ and $R_f$ is H, $R_{10}$ and $R_{11}$ are both -OH.

Another embodiment of the invention provides a method of stimulating an immune response in a subject comprising administering a compound of formula III:

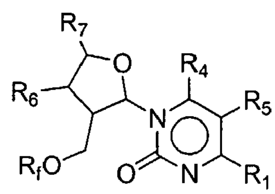

III

wherein,

$R_1$ and $R_2$ are each independently H, halo, $-NR_aR_b$, =O $C_{1-6}$ alkoxy, substituted $C_{1-6}$ alkoxy, heterocyclyl, substituted heterocyclyl, $C_{6-10}$ aryl, substituted $C_{6-10}$ aryl, $C_{1-6}$ alkyl, or substituted $C_{1-6}$ alkyl;

$R_4$ and $R_5$ are each independently H, halo, heterocyclyl, substituted heterocyclyl, -C(O)-$R_d$, $C_{1-6}$ alkyl, or substituted $C_{1-6}$ alkyl;

$R_6$ and $R_7$ are each independently H, halo, $C_{1-6}$ alkoxy, substituted $C_{1-6}$ alkoxy, $C_{2-6}$ alkenyl, substituted $C_{2-6}$ alkenyl, $-NR_aR_b$, $-N_3$, or -OH;

each $R_a$ and $R_b$ is independently H, $C_{1-6}$ alkyl, substituted $C_{1-6}$ alkyl, -C(O)$R_d$, or $C_{6-10}$ aryl;

each $R_d$ is independently H, halo, $C_{1-6}$ alkyl, substituted $C_{1-6}$ alkyl, $C_{1-6}$ alkoxy, substituted $C_{1-6}$ alkoxy, $-NH_2$, -NH($C_{1-6}$ alkyl), -NH(substituted $C_{1-6}$ alkyl), -N($C_{1-6}$ alkyl)$_2$, -N(substituted $C_{1-6}$ alkyl)$_2$, $C_{6-10}$ aryl, or heterocyclyl; and $R_f$ is independently H, $C_{1-6}$ alkyl, substituted $C_{1-6}$ alkyl, -C(O)$R_d$, phosphate, diphosphate, or triphosphate.

In another embodiment of the invention the small molecule or nucleoside analog is:

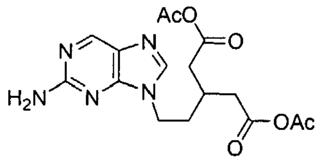, 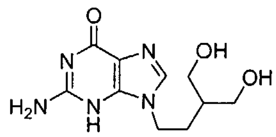, 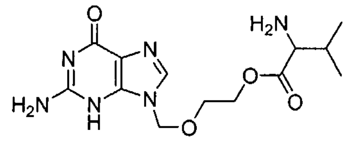,

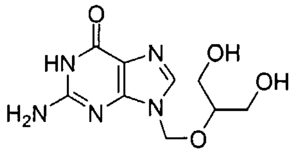, 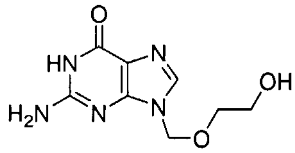, 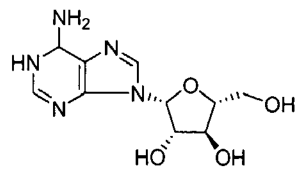,

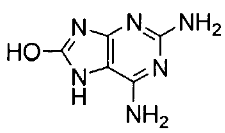, 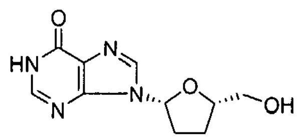, 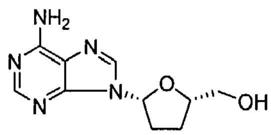, 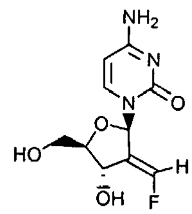

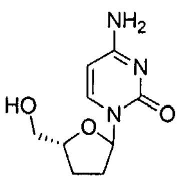, 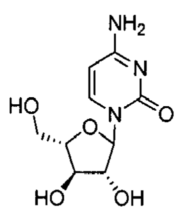, 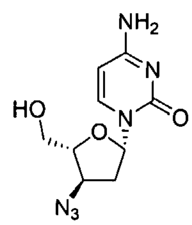, 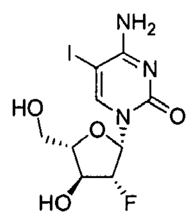, or 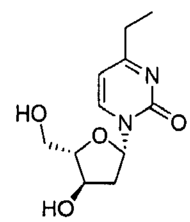.

Further provided is a composition comprising a cytokine or chemokine in combination with any compound(s) disclosed herein, such as a nucleoside, a compound encompassed by formula I, II, or III, or those listed in Table 1. In still a more particular embodiment thereof, the compound is present in the blood at less then 20 uM.

In another embodiment said nucleoside analog induces interferon biosynthesis.

In another embodiment said nucleoside analog is co-administered with another agent to the subject. In still another embodiment the agent is an antigen. In another embodiment the agent is a vaccine.

In another embodiment said nucleoside analog induces the production of TNF-$\alpha$ in the subject. In another embodiment thereof the nucleoside analog has an average steady-state drug concentration in the blood of less than 20 $\mu$M.

In another embodiment the subject is suffering from a microbial infection.

In another embodiment the subject is suffering from a viral infection. In another embodiment the viral infection is a viral infection caused by the hepatitis C virus (HCV). In yet another embodiment the viral infection is caused by the human immunodeficiency virus (HIV). In still another embodiment the viral infection is caused by the herpes simplex virus (HSV). In yet another embodiment the viral infection is caused by the SARS virus.

Further provided are any asymmetric carbon atoms (for example in compounds of formula I [or an N-oxide thereof]) in the (R)-, (S)- or (R,S)-configuration, preferably in the (R)- or (S)- configuration. Substituents at a double bond or a ring may be present in cis (-Z-) or trans (-E-) form. The compounds may thus be present as mixtures of isomers or as pure isomers, preferably as enantiomerically-pure.

Furthermore, it is contemplated that the invention encompasses all possible combinations of the preceding embodiments.

The nucleoside analog compounds can be used with or without an antigen in therapeutic applications, for example to treat cancer or infectious diseases. The nucleoside analog compounds also may be used in combination

with other therapeutic agents, such as anti-virals and monoclonal antibodies in different therapeutic applications.

One preferred embodiment of the method of inducing an immunostimulatory effect in a patient is directed to administering an immunogenic composition comprising a vaccine in an amount effective to stimulate an immune response such as a cell-mediated immune response and, as a vaccine adjuvant, an nucleoside analog compound, in an amount effective to potentiate the immune response such as the cell-mediated immune response to the vaccine.

Agents combined with the nucleoside analog compounds, contemplated to be useful in treating the aforementioned diseases include those well known in the art, such as, anesthetics, hypnotic sedatives, anti-anxieties, antiepileptics, antipyretic antiphlogistics, stimulants, wake amines, anti-parkinson drugs, agents for psychoneuroses, agents for central nervous system, skeletal muscle relaxants, agents for autonomic nervous system, antispastic agents, cytotoxic agents, monoclonal antibodies, drugs for eye, drugs for nose and ear, anti-vertiginous drugs, cardiotonics, antiarrhythmic drugs, diuretics, pressure reduction drugs, vasoconstrictors, coronary vaso-dilators, peripheral vasodilating drugs, hyper-lipemia drugs, breath stimulants, antitussive and expectorant drugs, bronchodilators, drugs for allergy, antidiarrheal drugs, drugs for intestinal disorders, peptic ulcer drugs, stomachic digestants, antacids, cholagogouses, pituitary hormone drugs, salivary gland hormones, thyroid hormone drugs, antithyroid drugs, anabolic steroids, corticosteroids, androgen drugs, estrogen drugs, corpus luteum hormone drugs, mixed hormones, urinary/genital organ drugs, anus drugs, surgical sterilizations/antiseptics, wound protectives, externals for purulent diseases, analgesics, antipruritics, astringents, antiphlogistics, externals for parasite skin diseases, skin-softening drugs, caustics, dental/oral drugs, vitamins, inorganic preparations, supplemental liquids, hemostatics, anticoagulation drugs, drugs for liver diseases, antidotes, habitual intoxication drugs, drugs for treatment of gout, enzyme preparations, diabetic drugs, antioncotics, antihistaminics, antibiotics (such as ketolides, aminoglycosides, sulphonamides, and/or beta lactams), chemotherapeutics, biological preparations, anthelmintics, anti-Protozoas, drugs for preparations, X-ray contrast media, and diagnostic drugs.

In another embodiment methods of treating allergies are provided comprising administering an nucleoside analog compound alone or in combination with another agent known to be effective against allergies, wherein said combination is more effective in treating an allergic condition than the known agent(s) are without the addition of said nucleoside analog compound. In a more preferred embodiment the known agent is an antihistamine and/or a leukotriene inhibitor. In another preferred embodiment, the allergic condition is asthma. In another preferred embodiment, the allergic condition is selected from the group consisting of allergic rhinitis, dermatosis, " and urticaria. In an even more preferred embodiment the combination is administered to a subject enterally, parenterally, intranasally, subcutaneously, or intraarterially.

Preferred SMIPs in accordance with the sixth aspect of the invention include nucleoside analogs and those compounds encompassed by Formula I as described in this section, or contained within any reference cited herein.

A "substituted pyrimidine" refers to an aromatic ring containing two nitrogen atoms as shown:

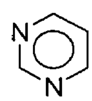,

wherein any of the substituents defined herein or well known in the art may be appended via a single or double bond (excluding double bonds to the nitrogen atoms) to any atom of the ring.

A "substituted purine" refers to two condensed aromatic rings containing two nitrogen atoms each as shown:

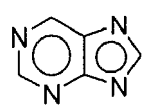,

wherein any of the substituents defined herein or well known in the art may be appended via a single or double bond (excluding double bonds to the nitrogen atoms) to any atom of the ring.

Reference to "halo," "halide," or "halogen" refers to F, Cl, Br, or I atoms, especially F, Cl, and Br.

The foregoing may be better understood by reference to the Examples, *infra*, that are presented for illustration and not to limit the scope of the inventive concepts. The Example compounds and their analogs are easily synthesized by one skilled in the art from procedures described herein, as well as in patents or patent applications listed herein.

**Section VII - Seventh Aspect of the Invention - Small Molecule Compounds for Immunopotentiation**

All definitions, descriptors of constituent variables for chemical formulas, and descriptions appearing in the section shall be understood to apply to this section only.

In accordance with the seventh aspect of the invention, Applicants have discovered methods of stimulating cytokine activity in cells and immunotherapeutics and/or vaccine adjuvants, that will provide effective treatments for disorders described herein and those apparent to one skilled in the art.

One embodiment provides a method of modulating an immune response in a subject comprising administering a compound selected from the group consisting of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate. Another embodiment is provided wherein said compound is coadministered with another agent.

In another more particular embodiment said subject is in remission from cancer. In another more particular embodiment said compound is administered for the treatment of refractory cancer cells. In another more particular embodiment said compound is administered metronomically. In a more particular embodiment the subject is not suffering from cancer. Further still, said subject is suffering from a cancer selected from the group consisting of prostate, breast, ovarian, colon, epidermal, ductal, non-small-cell lung, colorectal, neuroendocrine, spinal, esophageal, pancreas, renal, stomach, lymphoidal, intestinal, bladder, uterine cervix, head and neck, brain, nasopharynx, leukemia, kaposis sarcoma, and mesothelioma. Another embodiment is provided wherein said compound is co-administered with another agent.

One embodiment provides a method of stimulating an immune response in a subject comprising administering a compound selected from the group consisting of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate. Another embodiment is provided wherein said compound is coadministered with another agent. In a more particular embodiment still, said compound is administered in a dose capable of increasing TNF-$\alpha$ levels, more specifically, said compound has an average steady state drug concentration in the blood of less than 20 $\mu$M.

In a more particular embodiment of said method of stimulating an immune response in a subject, said compound is administered in a dose capable of inducing cytokines. In another more particular embodiment said subject is in remission from cancer. In another more particular embodiment said compound is administered for the treatment of refractory cancer cells. In another more particular embodiment said compound is administered metronomically. In a more particular embodiment the subject is not suffering from cancer. Further still, said subject is suffering from a cancer selected from the group consisting of prostate, breast, ovarian, colon, epidermal, ductal, non-small-cell lung, colorectal, neuroendocrine, spinal, esophageal, pancreas, renal, stomach, lymphoidal, intestinal, bladder, uterine cervix, head and neck, brain, nasopharynx, leukemia, kaposis sarcoma, and mesothelioma. Another embodiment is provided wherein said compound is co-administered with another agent. In a more particular embodiment still, said compound is administered in a dose capable of increasing TNF-$\alpha$ levels, more specifically, said compound has an average steady state drug concentration in the blood of less than 20 $\mu$M.

Another embodiment of the invention provides a method of treating a subject in need of immune stimulation, comprising administering to a subject in need thereof, a subcytotoxic amount of a compound selected from the group consisting of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate. Another embodiment is provided wherein said compound is co-administered with another agent. In a more particular embodiment still, said compound is administered in a dose capable of increasing TNF-$\alpha$ levels, more specifically, said compound has an average steady state drug concentration in the blood of less than 20 $\mu$M.

In a more particular embodiment of said method of treating a subject in need of immune stimulation, said amount is sufficient to stimulate cytokine production in the subject. In another more particular embodiment said subject is in remission from cancer. In another more particular embodiment said compound is administered for the treatment of refractory cancer cells. In another more particular embodiment said compound is administered metronomically. In another more particular embodiment the subject is not suffering from cancer. Further still, said subject is suffering from a cancer selected from the group consisting of prostate, breast, ovarian, colon, epidermal, ductal, non-small-cell lung, colorectal, neuroendocrine, spinal, esophageal, pancreas, renal, stomach, lymphoidal, intestinal, bladder, uterine cervix, head and neck, brain, nasopharynx, leukemia, kaposis sarcoma, and mesothelioma. Another embodiment is provided wherein said compound is co-administered with another agent. In a more particular embodiment still, said compound is administered in a dose capable of increasing TNF-$\alpha$ levels, more specifically, said compound has an average steady state drug concentration in the blood of less than 20 $\mu$M.

Another embodiment of the invention provides a method of identifying a subject in need of a compound selected from the group consisting of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate, comprising:

a) taking a blood sample from said subject;
b) monitoring for cytokine levels in said sample; and
c) identifying said subject by decreased cytokine levels in said sample.

In a more particular embodiment of said method of identifying a subject, a further step of administering said compound to the subject, wherein upon administration of said compound, cytokine levels increase is provided. Another embodiment is provided wherein said compound is co-administered with another agent. In a more particular embodiment still, said compound is administered in a dose capable of increasing TNF-$\alpha$ levels, more specifically, said compound has an average steady state drug concentration in the blood of less than 20 $\mu$M.

Another embodiment of the invention provides a method of identifying a subject in need of a compound selected from the group consisting of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate, comprising:

a) taking a blood sample from said subject;
b) monitoring for leukocyte levels in said sample; and
c) identifying said subject by decreased leukocyte levels in said sample.

In a more particular embodiment of said method of identifying a subject, a further step of administering said compound to the subject, wherein upon administration of said compound, leukocyte levels increase is provided. Another embodiment is provided wherein said compound is co-administered with another agent. In a more particular embodiment still, said compound is administered in a dose capable of increasing TNF-$\alpha$ levels, more specifically, said compound has an average steady state drug concentration in the blood of less than 20 $\mu$M.

Another embodiment of the invention provides a method of determining efficacy of a compound selected from the group consisting of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate, comprising:

a) taking a first blood sample from a subject;
b) identifying a first cytokine level in said sample;
c) administering said compound to said subject;
d) taking a second blood sample from said subject;
e) identifying a second cytokine level in said second blood sample, wherein the compound is efficacious if said second cytokine level is greater than said first cytokine level.

Another embodiment of the invention provides a method of treating a subject suffering from an autoimmune disease, comprising administering to a subject in need thereof, a compound selected from the group consisting of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate. In a more particular embodiment said autoimmune disease is Addison's Disease, or Alopecia Areata, or Ankylosing Spondylitis, or Antiphospholipid Syndrome (APS), or Behcet's Disease, or Chronic Fatigue Syndrome, or Crohn's Disease, or Ulcerative Colitis, or Diabetes, or Fibromyalgia, or Goodpasture Syndrome, or Graft Versus Host Disease, or Graves' Disease, or Guillain-Barre Syndrome, or Lupus, or Meniere's, or Multiple Sclerosis, or Myasthenia Gravis, or Myositis, or Pemphigus Vulgaris, or Primary Biliary Cirrhosis, or Psoriasis, or Rheumatic Fever, or Sarcoidosis, or Scleroderma, or Vasculitis, or Vitiligo, or Wegener's Granulomatosis. Another embodiment is provided wherein said compound is co-administered with another agent. In a more particular embodiment still, said compound is administered in a dose capable of increasing TNF-$\alpha$ levels, more specifically, said compound has an average steady state drug concentration in the blood of less than 20 $\mu$M.

Another embodiment of the invention provides a method of treating a subject suffering from a viral infection, comprising administering to a subject in need thereof, a compound selected from the group consisting of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate. In a more particular embodiment said viral infection is HIV, or HCV, or HBV, or HSV. Another embodiment is provided wherein said compound is co-administered with another agent. In a more particular embodiment still, said compound is administered in a dose capable of increasing

TNF-α levels, more specifically, said compound has an average steady state drug concentration in the blood of less than 20 μM.

Another embodiment of the invention provides a method of treating a subject suffering from allergies or asthma, comprising administering to a subject in need thereof, a compound selected from the group consisting of fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate. Another embodiment is provided wherein said compound is co-administered with another agent. In a more particular embodiment still; said compound is administered in a dose capable of increasing TNF-α levels, more specifically, said compound has an average steady state drug concentration in the blood of less than 20 μM.

Preferred SMIPs in accordance with the seventh aspect of the invention include fenretinide, vatalanib, SU-11248, SU 5416, SU 6668, oxaliplatin, bortezomib, R 115777, CEP-701, ZD-6474, MLN-518, lapatinib, gefitinib, erlotinib, perifosine, CYC-202, LY-317615, squalamine, UCN-01, midostaurin, irofulven, alvocidib, genistein, DA-9601, avicine, docetaxel, IM 862, SU 101, and tetrathiomolybdate compositions as well as analogs disclosed in the following patents and patent applications: US 4,323,581, US 6,258,812, WO 98/35958, WO 01/60814, US 5,883,113, WO 99/61422, US 5,883,113, WO 99/61422, WO 03/24978, WO 03/04505, US 5,780,454, US 2003134846, WO 97/21701, US 5,621,100, WO 01/32651, WO 02/16351, US 6,727,256, WO 02/02552, US 5,457,105, US 5,616,582, US 5,770,599, US 5,747,498, WO, 96/30347, US 2003171303, WO 97/20842, WO 99/02162, WO 95/17182, WO 01/79255, WO 89/07105, US 5,439,936, WO 94/18151, WO 97/42949, WO 98/13344, US 5,554,519, WO 98/04541, US 6,025,387, US 2004073044, WO 02/62826, WO 04/06834, US 6,331,555, and WO 01/60814. More particularly, preferred SMIPs include those encompassed by Formula I in the aforementioned patents and patent applications.

The invention also includes compounds encompassed by Formula I, disclosed in the following patents and patent applications: US 4,323,581, US 6,258,812, WO 98/35958, WO 01/60814, US 5,883,113, WO 99/61422, US 5,883,113, WO 99/61422, WO 03/24978, WO 03/04505, US 5,780,454, US 2003134846, WO 97/21701, US 5,621,100, WO 01/32651, WO 02/16351, US 6,727,256, WO 02/02552, US 5,457,105, US 5,616,582, US 5,770,599, US 5,747,498, WO, 96/30347, US 2003171303, WO 97/20842, WO 99/02162, WO 95/17182, WO 01/79255, WO 89/07105, US 5,439,936, WO 94/18151, WO 97/42949, WO 98/13344, US 5,554,519, WO 98/04541, US 6,025,387, US 2004073044, WO 02/62826, WO 04/06834, US 6,331,555, and WO 01/60814.

The foregoing may be better understood by reference to the Examples, *infra*, that are presented for illustration and not to limit the scope of the inventive concepts. The Example compounds and their analogs are either commercially available, or are easily synthesized by one skilled in the art from procedures described in patents or patent applications listed herein.

Each aspect of the present invention also comprises compounds identified by the assay methods described herein.

**Definitions:**

The following Definitions apply throughout this document, in the absence of a countervailing definition in a given section.

| | |
|---|---|
| ATP: | Adenosine triphosphate |
| BCG | Mycobacterium bovis bacillus Calmette-Guerin |
| BSA: | Bovine Serum Albumin |
| FHA | Filamentous haemaglutinin |
| GCMS | Gas Chromatography / Mass Spectroscopy |
| H. Pylori | Helicobacter Pylori |
| HAV | Hepatitis A Virus |
| HBV | Hepatitis B Virus |
| HCV | Hepatitis C Virus |
| HIV | Human Immunodeficiency Virus |
| HPLC | High Performance Liquid Chromatography |
| HSV | Herpes Simplex Virus |
| $IC_{50}$ value: | The concentration of an inhibitor that causes a 50 % reduction in a measured activity. |
| IFN | Interferon |
| IL | Interleukin |
| IMS | Immunomagnetic separation |
| IPV | Inactivated polio virus |
| LCMS | Liquid Chromatography / Mass Spectroscopy |
| LPS | Lip polysaccharide |

| | |
|---|---|
| MAb or mAb | Monoclonal Antibody |
| Men A | Neisseria Meningitidis Type A |
| Men C | Neisseria Meningitidis Type C |
| Men B | Neisseria Meningitidis Type B |
| Men W | Neisseria Meningitidis Type W |
| Men Y | Neisseria Meningitidis Type Y |
| MeOH: | Methanol |
| NANB | Non-A, non-B hepatitis |
| NMR | Nuclear magnetic resonance |
| OMV | Outer membrane vesicle |
| PBMC | Peripheral blood mononuclear cells |
| PT | Petussis holotoxin |
| Rt | Room temperature (25˚C) |
| SMIP | Small Molecule Immune Potentiator |
| TLC | Thin-layer chromatography |
| TNF-$\alpha$ | Tumour necrosis factor-a |

The term "SMIP" refers to small molecule immunopotentiating compounds, including small molecule compounds below about MW 800 g/mol, capable of stimulating or modulating a pro-inflammatory response in a patient. In an embodiment, the SMIP compounds are able to stimulate human peripheral blood mononuclear cells to produce cytokines.

The term "SMIS" refers to small molecule immunosuppressant compounds, including small molecule compounds below about MW 800 g/mol, capable of suppressing or modulating an immune response in a patient. In an embodiment, the SMIS compounds are able to inhibit human peripheral blood mononuclear cell's ability to produce cytokines, chemokines, and/or growth factors. In another embodiment, the SMIS compounds are able to induce TGF-beta production, thereby suppressing an immune response.

The term "refractory cancer cells" refers to cancer cell lines that are resistant to preexisting therapeutics or treatment regimens, including prescribed dosing schedules.

The methods of the invention are useful in treating "allergic diseases," that is accomplished in the same way as other immunotherapeutic methods described herein.

An "allergen" refers to a substance (antigen) that can induce an allergic or asthmatic response in a susceptible subject. The list of allergens is enormous and can include pollens, insect venoms, animal dander, dust, fungal spores, and drugs (e.g. penicillin).

"Asthma" refers to a disorder of the respiratory system characterized by inflammation, narrowing of the airways and increased reactivity of the airways to inhaled agents. Asthma is frequently, although not exclusively associated with atopic or allergic symptoms.

The term "leukotriene inhibitor" includes any agent or compound that inhibits, restrains, retards or otherwise interacts with the action or activity of leukotrienes, such as, but not limited to, 5-lipoxygenase ("5-LO") inhibitors, 5-lipoxygenase activating protein ("FLAP") antagonists, and leukotriene D4 ("LTD4 ") antagonists.

"Modulating" refers to inducing or suppressing.

"Immune-stimulation" or "immune potentiation" refers to activation of the immune system, including humoral or cellular activation, for example, activation of a cell, such as a killer (T or NK) or dendritic cell of the immune system, for example, causing the increase in cytokine production from a dendritic cell leading to an overall enhancement of host defense (immune response).

"Modulating an immune response" refers to either immune potentiation or immune suppression as defined herein.

An "immunogenic composition" refers to a composition capable of modulating the production of cytokines in a subject thereby effecting immune potentiation in the subject.

"Immune suppression" or "immunosuppression" refers to deactivation of the immune system, for example, preventing or lessening cytokine production from a dendritic cell leading to an overall attenuation of host defense (immune response).

An "immune-stimulatory effective amount" is an amount effective for activation of the immune system, for example, causing the increase in cytokine production from a dendritic cell leading to an overall enhancement of host defense (immune response).

"Enhancing the immune response to an antigen" by a compound refers to enhancement of the immune response in comparison to that in the absence of the compound. An enhanced immune-response eliciting composition is a composition generally comprising an antigen and a small molecule immune potentiator compound that elicits an immune response greater that a composition comprising an antigen and not containing one or more small molecule immune potentiator compounds. In this embodiment, the compound acts as an adjuvant, for example for use in vaccine compositions and methods.

A "disease associated with cellular proliferation" includes, but is not limited to neuro-fibromatosis, atherosclerosis, pulmonary fibrosis, arthritis, psoriasis, glomerulonephritis, restenosis, proliferative diabetic retinopathy (PDR), hypertrophic scar formation, inflammatory bowel disease, transplantation rejection, angiogenesis, and endotoxic shock.

The term "effective amount" is an amount necessary or sufficient to realize a desired biological effect. For example, an effective amount of a compound to treat an infectious disorder may be an amount necessary to cause an antigen specific immune response upon exposure to an infectious agent. The effective amount may vary, depending, for example, upon the condition treated, weight of the subject and severity of the disease. One of skill in the art can readily determine the effective amount empirically without undue experimentation.

As used herein "an effective amount for treatment" refers to an amount sufficient to palliate, ameliorate, stabilize, reverse, slow or delay progression of a condition such as a disease state.

Reference to "metronomic administration" or "administered metronomically" refers to increasingly frequent dosing regimens, at lower drug concentrations, as compared with known dosing regimens for an existing therapeutic. Metronomic administration varies from the typical dosing of cytotoxic drugs, which involves episodic (less frequent) administration at maximum tolerated doses (MTDs).

A "subject" or "patient" is meant to describe a human or vertebrate animal including a dog, cat, pocket pet, marmoset, horse, cow, pig, sheep, goat, elephant, giraffe, chicken, lion, monkey, owl, rat, squirrel, slender loris, and mouse.

A "pocket pet" refers to a group of vertebrate animals capable of fitting into a commodious coat pocket such as, for example, hamsters, chinchillas, ferrets, rats, guinea pigs, gerbils, rabbits and sugar gliders.

As used herein, the term "pharmaceutically acceptable ester" refers to esters, which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Representative examples of particular esters include, but are not limited to, formates, acetates, propionates, butyrates, acrylates and ethylsuccinates.

The compounds of the present invention can be used in the form of salts as in "pharmaceutically acceptable salts" derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-napth-alenesulfonate, oxalate, pamoate, pectinate, sulfate, 3-phenylpropionate, picrate, pivalate, propionate, succinate, tartrate, thiocyanate, p-toluenesulfonate and undecanoate. Also, the basic nitrogen-containing groups can be quatemized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

The term "pharmaceutically acceptable prodrugs" as used herein refers to those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed *in vivo* to yield the parent compound of the formula as described herein, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987. Prodrugs as described in U.S. Patent No. 6,284,772 for example may be used.

The symbol ∿ is meant to indicate the point of attachment of an appendage.

Reference to "halo," "halide," or "halogen" refers to F, Cl, Br, or I atoms.

The phrase "alkyl" refers to substituted and unsubstituted alky groups such as methyl, ethyl, propyl, butyl, pentyl, hexyl, heptyl, octyl, nonyl, decyl, undecyl, dodecyl and the like. The phrase also includes branched chain isomers of straight chain alkyl groups, including but not limited to, the following which are provided by way of example: $-CH(CH_3)_2$, $-CH(CH_3)(CH_2CH_3)$, $-CH(CH_2CH_3)_2$, $-C(CH_3)_3$, $-C(CH_2CH_3)_3$, $-CH_2CH(CH_3)_2$, $-CH_2CH(CH_3)(CH_2CH_3)$, $-CH_2CH(CH_2CH_3)_2$, $-CH_2C(CH_3)_3$, $-CH_2C(CH_2CH_3)_3$, $-CH(CH_3)CH(CH_3)(CH_2CH_3)$, $-CH_2CH_2CH(CH_3)_2$, $-CH_2CH_2CH(CH_3)(CH_2CH_3)$, $-CH_2CH_2CH(CH_2CH_3)_2$, $-CH_2CH_2C(CH_3)_3$, $-CH_2CH_2C(CH_2CH_3)_3$, $-CH(CH_3)CH_2CH(CH_3)_2$, $-CH(CH_3)CH(CH_3)CH(CH_3)_2$, $-CH(CH_2CH_3)CH(CH_3)CH(CH_3)(CH_2CH_3)$, and others. The phrase also includes cyclic alkyl groups such as cyclopropyl, cyclobutyl, cyclopentyl, cyclohexyl, cycloheptyl, and cyclooctyl and such rings substituted with straight and branched chain alkyl groups as defined above. The phrase also includes polycyclic alkyl groups such as, but not limited to, adamantyl norbornyl, and bicyclo[2.2.2]octyl and such rings substituted with straight and branched

chain alkyl groups as defined above. The phrase alkyl also includes groups in which one or more bonds to a carbon(s) or hydrogen(s) are replaced by a bond to non-hydrogen and non-carbon atoms such as, but not limited to, a halogen atom in halides such as F, Cl, Br, and I; and oxygen atom in groups such as hydroxyl groups, alkoxy groups, aryloxy groups, and ester groups; a sulfur atom in groups such as thiol groups, alkyl and aryl sulfide groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as in trialkylsilyl groups, dialkylarylsilyl groups, alkyldiarylsilyl groups, and triarylsilyl groups; and other heteroatoms in various other groups. Alkyl groups are those limited to having 1 to 20 carbon atoms and as many as 5 additional heteroatoms as described above. More preferred alkyl groups have from 1 to 5 carbon atoms and as many as 2 heteroatoms. The term "$C_{1-6}$ alkyl" has the same meaning as alkyl, except that it is limited to alkyl groups of six carbon atoms or less. The term "$C_2$ alkyl" indicates an alkyl group having two carbon atoms.

The phrase "aryl" refers to substituted and unsubstituted aryl groups that do not contain heteroatoms. Thus the phrase includes, but is not limited to, groups such as phenyl, biphenyl, anthracenyl, naphthenyl by way of example. Aryl groups also include those in which one of the aromatic carbons is bonded to a non-carbon or non-hydrogen atoms described above (in the alkyl definition) and also includes aryl groups in which one or more aromatic carbons of the aryl group is bonded to a substituted and/or unsubstituted alkyl, alkenyl, or alkynyl group as defined herein. This includes bonding arrangements in which two carbon atoms of an aryl group are bonded to two atoms of an alkyl, alkenyl, or alkynyl group to define a fused ring system (e.g. dihydronaphthyl or tetrahydronaphthyl). Thus, the phrase "aryl" includes, but is not limited to tolyl, and hydroxyphenyl among others. The term "$C_{6-10}$ aryl" has the same meaning as aryl, except that it is limited to aryl groups of from six to ten carbon atoms.

The phrase "alkenyl" refers to straight and branched chain and cyclic groups such as those described with respect to alkyl groups as defined above, except that at least one double bond exists between two carbon atoms. Examples include, but are not limited to vinyl, $-CH=C(H)(CH_3)$, $-CH=C(CH_3)_2$, $-C(CH_3)=C(H)_2$, $-C(CH_3)=C(H)(CH_3)$, $-C(CH_2CH_3)=CH_2$, cyclohexenyl, cyclopentenyl, cyclohexadienyl, butadienyl, pentadienyl, and hexadienyl among others. Included as well are groups in which a non-carbon or non-hydrogen atom is bonded to a carbon double bonded to another carbon and those in which one of the non-carbon or non-hydrogen atoms are bonded to a carbon not involved in a double bond to another carbon. Alkenyl groups are those limited to having 2 to 15 carbon atoms and as many as 4 additional heteroatoms as described above. More preferred alkenyl groups have from 2 to 5 carbon atoms and as many as 2 heteroatoms. The term "$C_{2-6}$ alkenyl" has the same meaning as alkenyl, except that it is limited to alkenyl groups of from two to six carbon atoms.

The phrase "alkoxy" refers to substituted or unsubstituted alkoxy groups of the formula -O-alkyl, wherein the point of attachment is the oxy group and the alkyl group is as defined above. Alkoxy groups are those limited to having 1 to 20 carbon atoms and as many as 5 additional heteroatoms, including the oxygen atom. More preferred alkoxy groups have from 1 to 5 carbon atoms and as many as 2 heteroatoms, including the oxygen atom. The term "$C_{1-6}$ alkoxy" has the same meaning as alkoxy, except that it is limited to alkoxy groups of six carbon atoms or less.

The phrase "alkynyl" refers to straight and branched chain groups such as those described with respect to alkyl groups as defined above, except that at least one triple bond exists between two carbon atoms. Examples include, but are not limited to $-C\equiv C(H)$, $-C\equiv C(CH_3)$, $-C\equiv C(CH_2CH_3)$, $-C(H_2)C\equiv C(H)$, $-C(H)_2C\equiv C(CH_3)$, and $-C(H)_2C\equiv C(CH_2CH_3)$ among others. Included as well are alkynyl groups in which a non-carbon or non-hydrogen atom is bonded to a carbon triple bonded to another carbon and those in which a non-carbon or non-hydrogen atom is bonded to a carbon not involved in a triple bond to another carbon. Alkynyl groups are those limited to having 2 to 15 carbon atoms and as many as 4 additional heteroatoms as described above. More preferred alkynyl groups have from 2 to 5 carbon atoms and as many as 2 heteroatoms. The term "$C_{2-6}$ alkynyl" has the same meaning as alkynyl, except that it is limited to alkynyl groups of from two to six carbon atoms.

The phrase "aryloxy" refers to groups having the formula -O-aryl, wherein the point of attachment is the oxy group and the aryl group is as defined above. The phrase "$C_{6-10}$ aryloxy" has the same meaning as aryloxy, except that it is limited to aryloxy groups of six to ten carbon atoms.

The phrase "trihalomethyl" refers to a methyl group in which the three H atoms of the methyl group are substituted with three halogens which may be same or different. One example of such a group is a $-CF_3$ group in which all three H atoms of the methyl group are substituted with F atoms.

The phrase "$C_{1-6}$ alkoxy-$C_{1-6}$ alkyl" refers to ether groups with as many as 12 carbon atoms. One example of a $C_{1-6}$ alkoxy-$C_{1-6}$ alkyl group is $-CH_2-O-CH_2CH_3$.

The phrase "$C_{6-10}$ aryloxy-$C_{1-6}$ alkyl" refers to aryl ether groups of 16 carbon atoms or less, especially of 10 carbon atoms or less bound at the $C_{1-6}$ alkyl group. One example of $C_{6-10}$ aryloxy-$C_{1-6}$ alkyl group is propoxybenzene.

The phrase "$C_{6-10}$ aryl-$C_{1-6}$ alkyl" refers to arylalkyl groups of 16 carbon atoms or less, especially of 10 carbon atoms or less bound at the $C_{1-6}$ alkyl group. One example of a $C_{6-10}$ aryl-$C_{1-6}$ alkyl group is toluene.

The phrase "heterocyclyl" refers to both aromatic and nonaromatic ring compounds including monocyclic, bicyclic, and polycyclic ring compounds such as, but not limited to, quinuclidyl, containing 3 or more ring members of

which one or more is a heteroatom such as, but not limited to, N, O, and S. Examples of heterocyclyl groups include, but are not limited to: unsaturated 3 to 8 membered rings containing 1 to 4 nitrogen atoms such as, but not limited to pyrrolyl, pyrrolinyl, imidazolyl, pyrazolyl, pyridyl, dihydropyridyl, pyrimidyl, pyrazinyl, pyridazinyl, triazolyl (e.g. 4H-1,2,4-triazolyl, 1H-1,2,3-triazolyl, 2H-1,2,3-triazolyl etc.), tetrazolyl, (e.g. 1H-tetrazolyl, 2H tetrazolyl, etc.); saturated 3 to 8 membered rings containing 1 to 4 nitrogen atoms such as, but not limited to, pyrrolidinyl, imidazolidinyl, piperidinyl, piperazinyl; condensed unsaturated heterocyclic groups containing 1 to 4 nitrogen atoms such as, but not limited to, indolyl, isoindolyl, indolinyl, indolizinyl, benzimidazolyl, quinolyl, isoquinolyl, indazolyl, benzotriazolyl; unsaturated 3 to 8 membered rings containing 1 to 2 oxygen atoms such as, but not limited to furanyl; unsaturated 3 to 8 membered rings containing 1 to 2 oxygen atoms and 1 to 3 nitrogen atoms such as, but not limited to, oxazolyl, isoxazolyl, oxadiazolyl (e.g. 1,2,4-oxadiazolyl, 1,3,4-oxadiazolyl, 1,2,5-oxadiazolyl, etc.); saturated 3 to 8 membered rings containing 1 to 2 oxygen atoms and 1 to 3 nitrogen atoms such as, but not limited to, morpholinyl; unsaturated condensed heterocyclic groups containing 1 to 2 oxygen atoms and 1 to 3 nitrogen atoms, for example, benzoxazolyl, benzoxadiazolyl, benzoxazinyl (e.g. 2H-1,4-benzoxazinyl etc.); unsaturated 3 to 8 membered rings containing 1 to 3 sulfur atoms and 1 to 3 nitrogen atoms such as, but not limited to, thiazolyl, isothiazolyl, thiadiazolyl (e.g. 1,2,3-thiadiazolyl, 1,2,4-thiadiazolyl, 1,3,4-thiadiazolyl, 1,2,5-thiadiazolyl, etc.); saturated 3 to 8 membered rings containing 1 to 2 sulfur atoms and 1 to 3 nitrogen atoms such as, but not limited to, thiazolodinyl; saturated and unsaturated 3 to 8 membered rings containing 1 to 2 sulfur atoms such as, but not limited to, thienyl, dihydrodithiinyl, dihydrodithionyl, tetrahydrothiophene, tetrahydrothiopyran; unsaturated condensed heterocyclic rings containing 1 to 2 sulfur atoms and 1 to 3 nitrogen atoms such as, but not limited to, benzothiazolyl, benzothiadiazolyl, benzothiazinyl (e.g. 2H-1,4-benzothiazinyl, etc.), dihydrobenzothiazinyl (e.g. 2H-3,4-dihydrobenzothiazinyl, etc.), unsaturated condensed heterocyclic rings containing 1 to 2 oxygen atoms such as benzodioxolyl (e.g. 1,3-benzodioxoyl, etc.); unsaturated 3 to 8 membered rings containing an oxygen atom and 1 to 2 sulfur atoms such as, but not limited to, dihydrooxathiinyl; saturated 3 to 8 membered rings containing 1 to 2 oxygen atoms and 1 to 2 sulfur atoms such as 1,4-oxathiane; unsaturated condensed rings containing 1 to 2 sulfur atoms such as benzothienyl, benzodithiinyl; and unsaturated condensed heterocyclic rings containing an oxygen atom and 1 to 2 oxygen atoms such as benzoxathiinyl. Heterocyclyl group also include those described above in which one or more S atoms in the ring is double-bonded to one or two oxygen atoms (sulfoxides and sulfones). For example, heterocyclyl groups include tetrahydrothiophene, tetrahydrothiophene oxide, and tetrahydrothiophene 1,1-dioxide. Preferred heterocyclyl groups contain 5 or 6 ring members. More preferred heterocyclyl groups include morpholine, piperazine, piperidine, pyrrolidine, imidazole, pyrazole, 1,2,3-triazole, 1,2,4-triazole, tetrazole, thiomorpholine, thiomorpholine in which the S atom of the thiomorpholine is bonded to one or more O atoms, pyrrole, homopiperazine, oxazolidin-2-one, pyrrolidin-2-one, oxazole, quinuclidine, thiazole, isoxazole, furan, and tetrahydrofuran. "Heterocyclyl" also refers to those groups as defined above in which one of the ring members is bonded to a non-hydrogen atom such as described above with respect to substituted alkyl groups and substituted aryl groups (also referred to herein as "substituted heterocyclyl"). Examples, include, but are not limited to, 2-methylbenzimidazolyl, 5-methylbenzimidazolyl, 5-chlorobenzthiazolyl, 1-methyl piperazinyl, and 2-chloropyridyl among others. Heterocyclyl groups are those limited to having 2 to 15 carbon atoms and as many as 6 additional heteroatoms as described above. More preferred heterocyclyl groups have from 3 to 5 carbon atoms and as many as 2 heteroatoms.

The term "substituted" as applied to an undefined, yet well known in the art group, such as phenyl, will have the same meaning with respect to the optional appendages as described in the definition of alkyl. Some preferred substitution groups include, for example, hydroxyl, nitro, amino, imino, cyano, halo, thio, thioamido, amidino, imidino, oxo, oxamidino, methoxamidino, imidino, guanidino, sulfonamido, carboxyl, formyl, alkyl, heterocyclyl, aryl, haloalkyl, alkoxy, alkoxyalkyl, alkylcarbonyl, arylcarbonyl, aralkylcarbonyl, alkylthio, aminoalkyl, alkylamino, cyanoalkyl, phosphate, diphosphate, triphosphate and the like. For example, one preferred "substituted $C_{1-6}$ alkyl" is tert-butanol.

The substitution group can itself be substituted one time. For example, an alkoxy substituent of an alkyl group may be substituted with a halogen, and oxo group, an aryl group, or the like. The group substituted onto the substitution group can be carboxyl, halo, nitro, oxo, amino, cyano, hydroxyl, $C_{1-6}$ alkyl, $C_{1-6}$ alkoxy, $C_{6-10}$ aryl, aminocarbonyl, -SR, thioamido, $-SO_3H$, $-SO_2R$ or cycloalkyl, where R is typically hydrogen, hydroxyl or $C_{1-6}$ alkyl.

When the substituted substituent includes a straight chain group, the substitution can occur either within the chain (e.g., 2-hydroxypropyl, 2-aminobutyl, and the like) or at the chain terminus (e.g., 2-hydroxyethyl, 3-cyanopropyl, and the like). Substituted substituents can be straight chain, branched or cyclic arrangements of covalently bonded carbon atoms or heteroatoms.

Reference to "phosphate" indicates $-O-PO_3$, where the point of attachment is oxo. Where a "phosphate" group is attached at an oxo moiety (e.g. $CH_3$-O-phosphate) the phosphate substituent is $-PO_3$ (e.g. $CH_3-O-PO_3$). "Diphosphate" and "triphosphate" groups are respectively 2 and 3 phosphate moieties bound together as in $-O-P(O)_2-O-P(O)_2-O-PO_3$ for triphophate.

The term "protected" or a "protecting group" with respect to hydroxyl groups, amine groups, and sulfhydryl groups refers to forms of these functionalities which are protected from undesirable reaction with a protecting group known to those skilled in the art such as those set forth in Protective Groups in Organic Synthesis, Greene, T.W., John

Wiley & Sons, New York, NY, (1st Edition, 1981) which can be added or removed using the procedures set forth therein. Examples of protected hydroxyl groups include, but are not limited to, silyl ethers such as those obtained by reaction of a hydroxyl group with a reagent such as, but not limited to, t-butyldimethyl-chlorosilane, trimethylchlorosilane, triisopropylchlorosilane, triethylchlorosilane; substituted methyl and ethyl ethers such as, but not limited to methoxymethyl ether, methythiomethyl ether, benzyloxymethyl ether, t-butoxymethyl ether, 2-methoxyethoxymethyl ether, tetrahydropyranyl ethers, 1-ethoxyethyl ether, allyl ether, benzyl ether; esters such as, but not limited to, benzoylformate, formate, acetate, trichloroacetate, and trifluoracetate. Examples of protected amine groups include, but are not limited to, benzyl or dibenzyl, amides such as, formamide, acetamide, trifluoroacetamide, and benzamide; imides, such as phthalimide, and dithiosuccinimide; and others. In some embodiments, a protecting group for amines is a benzyl group. Examples of protected sulfhydryl groups include, but are not limited to, thioethers such as S-benzyl thioether, and S-4-picolyl thioether; substituted S-methyl derivatives such as hemithio, dithio and aminothio acetals; and others.

Within the present invention it is to be understood that a 3,4-di(1H-indol-3-yl)-1H-pyrrole-2,5-dione, staurosporine analog, derivatized pyridazine, chromen-4-one, indolinone, quinazoline, nucleoside analog, or other small molecule as described herein may exhibit the phenomenon of tautomerism and that the formulae drawings within this specification can represent only one of the possible tautomeric forms. It is to be understood that the invention encompasses any tautomeric form which possesses immunomodulatory activity and is not to be limited merely to any one tautomeric form utilized within the formulae drawings.

It is also to be understood that certain 3,4-di(1H-indol-3-yl)-1H-pyrrole-2,5-diones, staurosporine analogs, derivatized pyridazines, chromen-4-ones, indolinones, quinazolines, nucleoside analogs, or other small molecules as described herein can exist in solvated as well as unsolvated forms such as, for example, hydrated forms. It is to be understood that the invention encompasses all such solvated forms which possess immunomodulatory activity.

The invention also includes isotopically-labeled compounds, that are structurally identical to those disclosed above, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chlorine, such as $^{2}H$, $^{3}H$, $^{13}C$, $^{14}C$, $^{15}N$, $^{18}O$, $^{17}O$, $^{31}P$, $^{32}P$, $^{3S}S$, $^{18}F$ and $^{36}Cl$, respectively. Compounds of the present invention, prodrugs thereof, and pharmaceutically acceptable salts of said compounds and of said prodrugs that contain the aforementioned isotopes and/or other isotopes of other atoms are within the scope of this invention. Certain isotopically labeled compounds of the present invention, for example those into which radioactive isotopes such as $^{3}H$ and $^{14}C$ are incorporated, are useful in drug and/or substrate tissue distribution assays. Tritiated, i.e., $^{3}H$, and carbon-14, i.e., $^{14}C$, isotopes are particularly preferred for their ease of preparation and detectability. Further, substitution with heavier isotopes such as deuterium, i.e., $^{2}H$, may afford certain therapeutic advantages resulting from greater metabolic stability, for example increased in vivo half-life or reduced dosage requirements and, hence, may be preferred in some circumstances. Isotopically labeled compounds of this invention and prodrugs thereof can generally be prepared by carrying out known or referenced procedures and by substituting a readily available isotopically labeled reagent for a non-isotopically labeled reagent.

Preferred "cytokines" include IL 1-30 as well as TNF-alpha, TNF-beta, IFN-alpha (family), IFN-beta and IFN-gamma.

Reference to "IL 1-30" indicates interleukin cytokines selected from the group consisting of IL1A, IL1B, IL1F5, IL1F6, IL1F7, IL1F8, IL1F9, IL1F10, IL1R1, IL1R2, IL1RAP, IL1RAPL1, IL1RAPL2, IL1RL1, IL1RL2, IL1RN, IL2, IL2RA, IL2RB, IL2RG, IL3, IL3RA, IL4, IL4R, IL5, IL5RA, IL6, IL6R, IL6RL1, IL6ST, IL6ST2, IL6STP, IL7, IL7R, IL8, IL8RA, IL8RB, IL8RBP, IL9, IL9R, IL9RP1, IL9RP2, IL9RP3, IL9RP4, IL10, IL10RA, IL10RB, IL11, IL11RA, IL11RB, IL12A, IL12B, IL12RB1, IL12RB2, IL13, IL13RA1, IL13RA2, IL14, IL15, IL15RA, IL15RB, IL16, IL17, IL17B, IL17C, IL17D, IL17E, IL17F, IL17R, IL17RB, IL17RC, IL17RD, IL17RE, IL18, IL18BP, IL18R1, IL18RAP, IL19, IL20, IL20RA, IL20RB, IL21, IL21R, IL22, IL22RA1, IL22RA2, IL23A, IL24, IL26, IL28A, IL28B, IL28RA, IL29, and IL30. More preferred interleukins include IL-1b IL-2, IL-4, IL-5, IL-6, IL-10, IL-12, and IL-13.

Reference to "chemokines" indicates: CXC chemokines including CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, and CXCL16; C chemokines including XCL1, and XCL2; $CX_3C$ chemokines including $CX_3CL1$; and CC chemokines including CCL1, CCL2, CCL3, CCL3L1, CCL4, CCL5, CCL6, CCL7, CCL8, CCL9/CCL10, CCL11, CCL12, CCL13, CCL14, CCL15, CCL16, CCL17, CCL18, CCL19, CCL20, CCL21, CCL22, CCL23, CCL24, CCL25, CCL26, CCL27, and CCL28.

"Concomitantly" refers to occurring or existing concurrently or in parallel with another.

Vaccine compositions contemplated to be within the scope of the several aspects of the present invention may include (an) additional adjuvant(s). Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without specific immunostimulating agents such as muramyl peptides or bacterial cell wall components), such as, for example (a) MF59™ (WO90/14837), containing 5% squalene, 0.5% Tween 80, and 0.5% Span 85 (optionally containing MTP-PE) formulated into submicron particles using a microfluidizer, (b)

SAF, containing 5% squalene, 0.5% Tween 80, 5% pluronic-blocked polymer L121, and thr-MDP either microfluidized into a submicron emulsion or vortexed to generate a larger particle size emulsion, and (c) Ribi ™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox ™); (3) saponin adjuvants, such as QS21 or Stimulon ™ (Cambridge Bioscience, Worcester, MA) may be used or particles generated therefrom such as ISCOMs (immunostimulating complexes), which ISCOMs may be devoid of additional detergent e.g. WO00/07621; (4) Complete Freund's Adjuvant (CFA) and Incomplete Freund's Adjuvant (IFA); (5) cytokines, such as interleukins (e.g. IL-1, IL-2, IL-4, IL-5, IL-6, IL-7, IL-12 (WO99/44636), etc.), interferons (e.g. gamma interferon), macrophage colony stimulating factor (M-CSF), tumor necrosis factor (TNF), etc.; (6) momophosphoryl lipid A (MPL) or 3-O-deacylated MPL (3dMPL), optionally in the substantial absence of alum when used with pneumococcal saccharides e.g. WO00/56358; and RC529 (7) combinations of 3dMPL with, for example, QS21 and /or oil-in-water emulsions e.g. EP-A-0835318; (8) oligonucleotides comprising CpG motifs, i.e. containing at least one CG dinucleotide, with 5-methylcytosine optionally being used in place of cytosine; (9) a polyoxyethylene ether or a polyoxyethylene ester e.g. WO99/52549; (10) a polyoxyethylene sorbitan ester surfactant in combination with an octoxynol (WO0121207) or a polyoxyethylene alkyl ether or ester surfactant in combination with at least one additional non-ionic surfactant such as an octoxynol (WO01/21152); (11) a saponin and an immunostimulatory oligonucleotide (e.g. a CpG oligonucleotide) (WO00/62800); (12) an immunostimulant and a particle of metal salt e.g WO00/23105; (13) a saponin and an oil-in-water emulsion e.g. WO99/11241; (14) a saponin (e.g. QS21) + 3dMPL +IL-12 (optionally + a sterol) e.g. WO98/57659; (14) other substances that act as immunostimulating agents to enhance the effectiveness of the composition. In one particular embodiment, Alum (especially aluminium phosphate and/or hydroxide) and MF59 are preferred for use with saccharide antigens.

In some aspects, the invention is also directed to methods of administering the vaccine composition. The vaccine is administered in an amount effective to stimulate an immune response. The amount that constitutes an effective amount depends, *inter alia*, on the particular vaccine used, the particular adjuvant compound being administered and the amount thereof, the immune response that is to be enhanced (humoral or cell mediated), the state of the immune system (e.g., suppressed, compromised, stimulated), and the desired therapeutic result. Accordingly it is not practical to set forth generally the amount that constitutes an effective amount of the vaccine. Those of ordinary skill in the art, however, can readily determine the appropriate amount with due consideration of such factors.

The vaccine compositions of the invention can be administered to animals, e.g., mammals human and non-human, including, for example, pocket pets, fowl, and the like according to conventional methods well known to those skilled in the art (e.g., orally, subcutaneously, nasally, topically).

Suitable vaccines include, but are not limited to, any material that raises either humoral or cell mediated immune response, or both. Suitable vaccines include live viral and bacterial antigens and inactivated viral, tumor-derived, protozoal, organism-derived, fungal, and bacterial antigens, toxoids, toxins, polysaccharides, proteins, glycoproteins, peptides, and the like. Conventional vaccines, such as those used in connection with BCG (live bacteria), cholera, plague, and typhoid (killed bacteria), hepatitis B, influenza, inactivated polio, and rabies (inactivated virus), measles, mumps, rubella, oral polio, SARS vaccines, and yellow fever (live virus), tetanus and diphtheria (toxoids), hemophilus influenzae b, meningococcal, and pneumococcal (bacterial polysaccharides) also can be used. Any antigen known in the art or disclosed herein may be used.

Furthermore, it is contemplated that certain currently experimental vaccines, especially materials such as recombinant proteins, glycoproteins, and peptides that do not raise a strong immune response, will also find use in connection with the 3,4-di(1H-indol-3-yl)-1H-pyrrole-2,5-dione compound. Exemplary experimental subunit antigens include those related to viral disease such as adenovirus, AIDS, chicken pox, cytomegalovirus, dengue, feline leukemia, fowl plague, hepatitis A, hepatitis B, hepatitis C, HSV-1, HSV-2, hog cholera, influenza A, influenza B, Japanese encephalitis, measles, parainfluenza, rabies, respiratory syncytial virus, SARS virus, rotavirus, wart, and yellow fever.

Specific antigens include: a protein antigen from *N. meningitides* serogroup B (1-7); an outer-membrane vesicle (OMV) preparation from *N. meningitides* serogroup B. (8, 9, 10, 11); a saccharide antigen from *N. meningitides* serogroup A, C W135 and/or Y, such as the oligosaccharide (12) from serogroup C (13); a saccharide antigen from *Streptococcus pneumoniae* (14, 15, 16); an antigen from *N. gonorrhoeae* (1, 2, 3); an antigen from *Chlamydia pneumoniae* (17, 18, 19, 20, 21, 22, 23); an antigen from *Chlamydia trachomatis* (24); an antigen from hepatitis A virus, such as inactivated virus (25, 26); an antigen from hepatitis B virus, such as the surface and/or core antigens (e.g. 26, 27); an antigen from hepatitis C virus (28); an antigen from *Bordetella pertussis,* such as petussis holotoxin (PT) and filamentous haemagglutinin (FHA) from *B. pertussis,* optionally also combination with pertactin and/or agglutinogens 2 and 3 (29, 30); a diphtheria antigen, such as a diphtheria toxoid (31:chapter 3) e.g. the $CRM_{197}$ mutant (32); a tetanus antigen, such as a tetanus toxoid (31 :chapter 4); a protein antigen from Helicobacter pylori such as CagA (33), VacA (33), NAP (34), HopX (5), HopY (35) and/or urease; a saccharide antigen from Haemophilus influenzae B (13); an antigen from Porphyromonas gingivalis (36); polio antigen(s) (37, 38) such as IPV or OPV; rabies antigen(s) (39) such lyophilized inactivated virus (40, RabAvert™); measles, mumps and/or rubella antigens (31: chapters 9, 10, & 11); influenza antigen(s)

(31:chapter 19), such as the haemagglutinin and/or neuraminidase surface proteins; an antigen from Moraxella catarrhalis (41); an antigen from Streptococcus agalactiae (group B streptococcus) (42, 43); an antigen from Streptococcus pyogenes (group A streptococcus) (43, 44, 45); and an antigen from Staphylococcus aureus (46).

The composition may comprise one or more of the above antigens.

Where a saccharide or carbohydrate antigen is used, it is preferably conjugated to a carrier protein in order to enhance antigenicity (47-56). Preferred carrier proteins are bacterial toxins or toxoids, such as diphtheria or tetanus toxoids. The $CRM_{197}$ diphtheria toxoid is particularly preferred. Other suitable carrier proteins include the *N. meningitides* outer membrane protein (57), synthetic peptides (58, 59), heat shock proteins (60), pertussis proteins (61, 62), protein D from *H. influenzae* (63), toxin A or B from *C. difficile* (64) etc. Where a mixture comprises capsular saccharides from both serogroups A and C, it is preferred that the ratio (w/w) of MenA saccharide:MenC saccharide is greater than 1 (e.g. 2:1, 3:1, 4:4, 5:1, 10:1 or higher). Saccharides from different serogroups of N. meningitides may be conjugated to the same or different carrier proteins.

Any suitable conjugation reaction can be used, with any suitable linker where necessary. Toxic protein antigens may be detoxified where necessary (e.g. detoxification of pertussis toxin by chemical and/or genetic means (30)). Where a diphtheria antigen is included in the composition it is preferred also to include tetanus antigens and pertussis antigens. Similar, where a tetanus antigen is include it is preferred also to include diphtheria and pertussis antigens. Similar, where pertussis antigen is included it is preferred also to include diphtheria and tetanus antigens.

The pharmaceutical compositions containing the compounds described herein can include additives such as excipients. Suitable pharmaceutically acceptable excipients include processing agents and drug delivery modifiers and enhancers, such as, for example, calcium phosphate, magnesium stearate, talc, monosaccharides, disaccharides, starch, gelatin, cellulose, methyl cellulose, sodium carboxymethyl cellulose, dextrose, hydroxypropyl-β-cyclodextrin, polyvinylpyrrolidinone, low melting waxes, ion exchange resins, and the like, as well as combinations of any two or more thereof. Other suitable pharmaceutically acceptable excipients are described in "Remington's Pharmaceutical Sciences," Mack Pub. Co., New Jersey (1991), incorporated herein by reference.

Pharmaceutical compositions containing the compounds of the invention may be in any form suitable for the intended method of administration, including, for example, a solution, a suspension, or an emulsion. Liquid carriers are typically used in preparing solutions, suspensions, and emulsions. Liquid carriers contemplated for use in the practice of the present invention include, for example, water, saline, pharmaceutically acceptable organic solvent(s), pharmaceutically acceptable oils or fats, and the like, as well as mixtures of two or more thereof. The liquid carrier may contain other suitable pharmaceutically acceptable additives such as solubilizers, emulsifiers, nutrients, buffers, preservatives, suspending agents, thickening agents, viscosity regulators, stabilizers, and the like. Suitable organic solvents include, for example, monohydric alcohols, such as ethanol, and polyhydric alcohols, such as glycols. Suitable oils include, for example, soybean oil, coconut oil, olive oil, safflower oil, cottonseed oil, and the like. For parenteral administration, the carrier can also be an oily ester such as ethyl oleate, isopropyl myristate, and the like. Compositions of the present invention may also be in the form of microparticles, microcapsules, and the like, as well as combinations of any two or more thereof.

The compounds and combinations of the present invention can also be administered in the form of liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic. Methods to form liposomes are known in the art. See, for example, Prescott, Ed., Methods in Cell Biology, Volume XIV, Academic Press, New York, N.W., p. 33 et seq (1976).

Other additives include immunostimulatory agents known in the art or listed herein. Immunostimulatory oligonucleotides and polynucleotides are described in PCT WO 98/55495 and PCT WO 98/16247. U.S. Patent Application No. 2002/0164341 describes adjuvants including an unmethylated CpG dinucleotide (CpG ODN) and a non-nucleic acid adjuvant. U.S. Patent Application No. 2002/0197269 describes compositions comprising an antigen, an antigenic CpG-ODN and a polycationic polymer. Other immunostimulatory additives described in the art may be used, for example, as described in U.S. Patent No. 5,026,546; U.S. Patent No. 4,806,352; and U.S. Patent No. 5,026,543. Additionally, SMIP compounds as described in USSN 10/814480 and 60/582654 are contemplated as effective co-administration agents or combination with the compositions of the instant invention.

A controlled release delivery system may be used, such as a diffusion controlled matrix system or an erodible system, as described for example in: Lee, "Diffusion-Controlled Matrix Systems", pp. 155-198 and Ron and Langer, "Erodible Systems", pp. 199-224, in "Treatise on Controlled Drug Delivery", A. Kydonieus Ed., Marcel Dekker, Inc., New York 1992. The matrix may be, for example, a biodegradable material that can degrade spontaneously *in situ* and *in vivo* for, example, by hydrolysis or enzymatic cleavage, e.g., by proteases. The delivery system may be, for example, a naturally occurring or synthetic polymer or copolymer, for example in the form of a hydrogel. Exemplary polymers with

cleavable linkages include polyesters, polyorthoesters, polyanhydrides, polysaccharides, poly(phosphoesters), polyamides, polyurethanes, poly(imidocarbonates) and poly(phosphazenes).

The compounds of the invention may be administered enterally, orally, parenterally, sublingually, by inhalation spray, rectally, or topically in dosage unit formulations containing conventional nontoxic pharmaceutically acceptable carriers, adjuvants, and vehicles as desired. For example, suitable modes of administration include oral, subcutaneous, transdermal, transmucosal, iontophoretic, intravenous, intramuscular, intraperitoneal, intranasal, subdermal, rectal, and the like. Topical administration may also involve the use of transdermal administration such as transdermal patches or ionophoresis devices. The term parenteral as used herein includes subcutaneous injections, intravenous, intramuscular, intrastemal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols that are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

Effective amounts of the compounds of the invention generally include any amount sufficient to detectably treat the disorders described herein.

Successful treatment of a subject in accordance with the invention may result in the inducement of a reduction or alleviation of symptoms in a subject afflicted with a medical or biological disorder to, for example, halt the further progression of the disorder, or the prevention of the disorder.

The amount of active ingredient that may be combined with the carrier materials to produce a single dosage form will vary depending upon the host treated and the particular mode of administration. It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy. The therapeutically effective amount for a given situation can be readily determined by routine experimentation and is within the skill and judgment of the ordinary clinician.

Compositions of the invention may be administered in conjunction with one or more antigens for use in therapeutic, prophylactic, or diagnostic methods of the present invention. Preferred antigens include those listed below. Additionally, the compositions of the present invention may be used to treat or prevent infections caused by any of the below-listed microbes.

In addition to combination with the antigens described below, the compositions of the invention may also be combined with an adjuvant as described herein. Antigens for use with the invention include, but are not limited to, one or more of the following antigens set forth below, or antigens derived from one or more of the pathogens set forth below:

### A. Bacterial Antigens

Bacterial antigens suitable for use in the invention include proteins, polysaccharides, lipopolysaccharides, and outer membrane vesicles which may be isolated, purified or derived from a bacteria. In addition, bacterial antigens may include bacterial lysates and inactivated bacteria formulations. Bacteria antigens may be produced by recombinant expression. Bacterial antigens preferably include epitopes which are exposed on the surface of the bacteria during at least one stage of its life cycle. Bacterial antigens are preferably conserved across multiple serotypes. Bacterial antigens include antigens derived from one or more of the bacteria set forth below as well as the specific antigens examples identified below.

*Neisseria meningitides: Meningitides* antigens may include proteins (such as those identified in References 1

- 7), saccharides (including a polysaccharide, oligosaccharide or lipopolysaccharide), or outer-membrane vesicles (References 8, 9, 10, 11) purified or derived from *N. meningitides* serogroup A, C, W135, Y, and/or B. Meningitides protein antigens may be selected from adhesions, autotransporters, toxins, Fe acquisition proteins, and membrane associated proteins (preferably integral outer membrane protein).

*Streptococcus pneumoniae*: *Streptococcus pneumoniae* antigens may include a saccharide (including a polysaccharide or an oligosaccharide) or protein from *Streptococcus pneumoniae.* Saccharide antigens may be selected from serotypes 1, 2, 3, 4, 5, 6B, 7F, 8, 9N, 9V, 10A, 11A, 12F, 14, 15B, 17F, 18C, 19A, 19F, 20, 22F, 23F, and 33F. Protein antigens may be selected from a protein identified in WO 98/18931, WO 98/18930, US Patent No. 6,699,703, US Patent No. 6,800,744, WO 97/43303, and WO 97/37026. Streptococcus pneumoniae proteins may be selected from the Poly Histidine Triad family (PhtX), the Choline Binding Protein family (CbpX), CbpX truncates, LytX family, LytX truncates, CbpX truncate-LytX truncate chimeric proteins, pneumolysin (Ply), PspA, PsaA, Sp128, Sp101, Sp130, Sp125 or Sp133.

*Streptococcus pyogenes (Group A Streptococcus):* Group A Streptococcus antigens may include a protein identified in WO 02/34771 or WO 2005/032582 (including GAS 40), fusions of fragments of GAS M proteins (including those described in WO 02/094851, and Dale, Vaccine (1999) 17:193-200, and Dale, Vaccine 14(10): 944-948), fibronectin binding protein (Sfb1), Streptococcal hemeassociated protein (Shp), and Streptolysin S (SagA).

*Moraxella catarrhalis:* Moraxella antigens include antigens identified in WO 02/18595 and WO 99/58562, outer membrane protein antigens (HMW-OMP), C-antigen, and/or LPS.

*Bordetella pertussis:* Pertussis antigens include petussis holotoxin (PT) and filamentous haemagglutinin (FHA) from *B. pertussis,* optionally also combination with pertactin and/or agglutinogens 2 and 3 antigen.

*Staphylococcus aureus:* Staph aureus antigens include *S. aureus* type 5 and 8 capsular polysaccharides optionally conjugated to nontoxic recombinant *Pseudomonas aeruginosa* exotoxin A, such as StaphVAX™, or antigens derived from surface proteins, invasins (leukocidin, kinases, hyaluronidase), surface factors that inhibit phagocytic engulfment (capsule, Protein A), carotenoids, catalase production, Protein A, coagulase, clotting factor, and/or membrane-damaging toxins (optionally detoxified) that lyse eukaryotic cell membranes (hemolysins, leukotoxin, leukocidin).

*Staphylococcus epidermis: S. epidermidis* antigens include slime-associated antigen (SAA).

*Tetanus:* Tetanus antigens include tetanus toxoid (TT), preferably used as a carrier protein in conjunction/conjugated with the compositions of the present invention.

*Diphtheria:* Diphtheria antigens include diphtheria toxin, preferably detoxified, such as $CRM_{197}$, additionally antigens capable of modulating, inhibiting or associated with ADP ribosylation are contemplated for combination/co-administration/conjugation with the compositions of the present invention, the diphtheria toxoids are preferably used as carrier proteins.

*Haemophilus influenzae B (Hib):* Hib antigens include a Hib saccharide antigen.

*Pseudomonas aeruginosa:* Pseudomonas antigens include endotoxin A, Wzz protein, *P. aeruginosa* LPS, more particularly LPS isolated from PAO1 (O5 serotype), and/or Outer Membrane Proteins, including Outer Membrane Proteins F (OprF) (Infect Immun. 2001 May; 69(5): 3510-3515).

*Legionella pneumophila* (Legionnairs' Disease): L. pneumophila antigens may optionally derived from cell lines with disrupted *asd* genes (Infect Immun. 1998 May; 66(5): 1898).

*Streptococcus agalactiae (Group B Streptococcus)*: Group B Streptococcus antigens include a protein or saccharide antigen identified in WO 02/34771, WO 03/093306, WO 04/041157, or WO 2005/002619 (including proteins GBS 80, GBS 104, GBS 276 and GBS 322, and including saccharide antigens derived from serotypes Ia, Ib, Ia/c, II, III, IV, V, VI, VII and VIII).

*Neiserria gonorrhoeae:* Gonorrhoeae antigens include Por (or porin) protein, such as PorB (*see* Zhu et al., Vaccine (2004) 22:660 - 669), a transferring binding protein, such as TbpA and TbpB (See Price et al., Infection and Immunity (2004) 71(1):277 - 283), a opacity protein (such as Opa), a reduction-modifiable protein (Rmp), and outer membrane vesicle (OMV) preparations (*see* Plante et al., J Infectious Disease (2000) 182:848 - 855), also see *e.g.* WO99/24578, WO99/36544, WO99/57280, WO02/079243).

*Chlamydia trachomatis:* Chlamydia trachomatis antigens include antigens derived from serotypes A, B, Ba and C are (agents of trachoma, a cause of blindness), serotypes $L_1$, $L_2$ & $L_3$ (associated with Lymphogranuloma venereum), and serotypes, D-K. Chlamydia trachomas antigens may also include an antigen identified in WO 00/37494, WO 03/049762, WO 03/068811, or WO 05/002619.

*Treponema pallidum* (Syphilis): Syphilis antigens include TmpA antigen.

*Haemophilus ducreyi* (causing chancroid)*:* Ducreyi antigens include outer membrane protein (DsrA).

*Enterococcus faecalis or Enterococcus faecium:* Antigens include a trisaccharide repeat or other *Enterococcus* derived antigens provided in US Patent No. 6,756,361.

*Helicobacter pylori:* H pylori antigens include Cag, Vac, Nap, HopX, HopY and/or urease antigen.

*Staphylococcus saprophyticus:* Antigens include the 160 kDa hemagglutinin of *S. saprophyticus* antigen.

*Yersinia enterocolitica* Antigens include LPS (Infect Immun. 2002 August; 70(8): 4414).

*E. coli:* E. coli antigens may be derived from enterotoxigenic *E. coli* (ETEC), enteroaggregative *E. coli* (EAggEC), diffusely adhering *E. coli* (DAEC), enteropathogenic *E. coli* (EPEC), and/or enterohemorrhagic *E. coli* (EHEC).

*Bacillus anthracis* (anthrax): *B. anthracis* antigens are optionally detoxified and may be selected from A-components (lethal factor (LF) and edema factor (EF)), both of which can share a common B-component known as protective antigen (PA).

*Yersinia pestis* (plague): Plague antigens include F1 capsular antigen (Infect Immun. 2003 Jan; 71(1)): 374-383, LPS (Infect Immun. 1999 Oct; 67(10): 5395), Yersinia pestis V antigen (Infect Immun. 1997 Nov; 65(11): 4476-4482).

*Mycobacterium tuberculosis:* Tuberculosis antigens include lipoproteins, LPS, BCG antigens, a fusion protein of antigen 85B (Ag85B) and/or ESAT-6 optionally formulated in cationic lipid vesicles (Infect Immun. 2004 October; 72 (10): 6148), Mycobacterium tuberculosis (Mtb) isocitrate dehydrogenase associated antigens (Proc Natl Acad Sci U S A. 2004 Aug 24; 101(34): 12652), and/or MPT51 antigens (Infect Immun. 2004 July; 72(7): 3829).

*Rickettsia:* Antigens include outer membrane proteins, including the outer membrane protein A and/or B (OmpB) (Biochim Biophys Acta. 2004 Nov 1; 1702(2):145), LPS, and surface protein antigen (SPA) (*J Autoimmun.* 1989 Jun;2 Suppl:81).

*Listeria monocytogenes* : Antigens derived from *L. monocytogenes* are preferably used as carriers/vectors for intracytoplasmic delivery of conjugates/associated compositions of the present invention.

*Chlamydia pneumoniae:* Antigens include those identified in WO 02/02606.

*Vibrio cholerae:* Antigens include proteinase antigens, LPS, particularly lipopolysaccharides of Vibrio cholerae II, O1 Inaba O-specific polysaccharides, V. cholera O139, antigens of IEM108 vaccine (Infect Immun. 2003 Oct;71(10): 5498-504), and/or Zonula occludens toxin (Zot).

*Salmonella typhi* (typhoid fever): Antigens include capsular polysaccharides preferably conjugates (Vi, i.e. vax-TyVi).

*Borrelia burgdorferi* (Lyme disease): Antigens include lipoproteins (such as OspA, OspB, Osp C and Osp D), other surface proteins such as OspE-related proteins (Erps), decorin-binding proteins (such as DbpA), and antigenically variable VI proteins. , such as antigens associated with P39 and P13 (an integral membrane protein, Infect Immun. 2001 May; 69(5): 3323-3334), VlsE Antigenic Variation Protein (J Clin Microbiol. 1999 Dec; 37(12): 3997).

*Porphyromonas gingivalis:* Antigens include *P. gingivalis* outer membrane protein (OMP).

*Klebsiella:* Antigens include an OMP, including OMP A, or a polysaccharide optionally conjugated to tetanus toxoid.

Where not specifically referenced, further bacterial antigens of the invention may be capsular antigens, polysaccharide antigens or protein antigens of any of the above. Further bacterial antigens may also include an outer membrane vesicle (OMV) preparation. Additionally, antigens include live, attenuated, split, and/or purified versions of any of the aforementioned bacteria. The bacterial or microbial derived antigens of the present invention may be gram-negative or gram-positive and aerobic or anaerobic.

Additionally, any of the above bacterial-derived saccharides (polysaccharides, LPS, LOS or oligosaccharides) can be conjugated to another agent or antigen, such as a carrier protein (for example $CRM_{197}$). Such conjugation may be direct conjugation effected by reductive amination of carbonyl moieties on the saccharide to amino groups on the protein, as provided in US Patent No. 5,360,897 and Can J Biochem Cell Biol. 1984 May;62(5):270-5. Alternatively, the saccharides can be conjugated through a linker, such as, with succinamide or other linkages provided in Bioconjugate Techniques, 1996 and CRC, Chemistry of Protein Conjugation and Cross-Linking, 1993.

**B. Viral Antigens**

Viral antigens suitable for use in the invention include inactivated (or killed) virus, attenuated virus, split virus formulations, purified subunit formulations, viral proteins which may be isolated, purified or derived from a virus, and Virus Like Particles (VLPs). Viral antigens may be derived from viruses propagated on cell culture or expressed recombinantly. Viral antigens preferably include epitopes which are exposed on the surface of the virus during at least one stage of its life cycle. Viral antigens are preferably conserved across multiple serotypes. Viral antigens include antigens derived from one or more of the viruses set forth below as well as the specific antigens examples identified below.

*Orthomyxovirus:* Viral antigens may be derived from an Orthomyxovirus, such as Influenza A, B and C. Orthomyxovirus antigens may be selected from one or more of the viral proteins, including hemagglutinin (HA), neuraminidase (NA), nucleoprotein (NP), matrix protein (M1), membrane protein (M2), one or more of the transcriptase components (PB1, PB2 and PA). Preferred antigens include HA and NA.

Influenza antigens may be derived from interpandemic (annual) flu strains. Alternatively influenza antigens may be derived from strains with the potential to cause pandemic a pandemic outbreak (i.e., influenza strains with new haemagglutinin compared to the haemagglutinin in currently circulating strains, or influenza strains which are pathogenic in avian subjects and have the potential to be transmitted horizontally in the human population, or influenza strains which are pathogenic to humans).

*Paramyxoviridae* viruses: Viral antigens may be derived from Paramyxoviridae viruses, such as Pneumoviruses (RSV), Paramyxoviruses (PIV) and Morbilliviruses (Measles).

*Pneumovirus:* Viral antigens may be derived from a Pneumovirus, such as Respiratory syncytial virus (RSV), Bovine respiratory syncytial virus, Pneumonia virus of mice, and Turkey rhinotracheitis virus. Preferably, the Pneumovirus is RSV. Pneumovirus antigens may be selected from one or more of the following proteins, including surface proteins Fusion (F), Glycoprotein (G) and Small Hydrophobic protein (SH), matrix proteins M and M2, nucleocapsid proteins N, P and L and nonstructural proteins NS1 and NS2. Preferred Pneumovirus antigens include F, G and M. See e.g., J Gen Virol. 2004 Nov; 85(Pt 11):3229). Pneumovirus antigens may also be formulated in or derived from chimeric viruses. For example, chimeric RSV/PIV viruses may comprise components of both RSV and PIV.

*Paramyxovirus*: Viral antigens may be derived from a Paramyxovirus, such as Parainfluenza virus types 1 - 4 (PIV), Mumps, Sendai viruses, Simian virus 5, Bovine parainfluenza virus and Newcastle disease virus. Preferably, the Paramyxovirus is PIV or Mumps. Paramyxovirus antigens may be selected from one or more of the following proteins: Hemagglutinin -Neuraminidase (HN), Fusion proteins F1 and F2, Nucleoprotein (NP), Phosphoprotein (P), Large protein (L), and Matrix protein (M). Preferred Paramyxovirus proteins include HN, F1 and F2. Paramyxovirus antigens may also be formulated in or derived from chimeric viruses. For example, chimeric RSV/PIV viruses may comprise components of both RSV and PIV. Commercially available mumps vaccines include live attenuated mumps virus, in either a monovalent form or in combination with measles and rubella vaccines (MMR).

*Morbillivirus*: Viral antigens may be derived from a Morbillivirus, such as Measles. Morbillivirus antigens may be selected from one or more of the following proteins: hemagglutinin (H), Glycoprotein (G), Fusion factor (F), Large protein (L), Nucleoprotein (NP), Polymerase phosphoprotein (P), and Matrix (M). Commercially available measles vaccines include live attenuated measles virus, typically in combination with mumps and rubella (MMR).

*Picornavirus*: Viral antigens may be derived from Picornaviruses, such as Enteroviruses, Rhinoviruses, Heparnavirus, Cardioviruses and Aphthoviruses. Antigens derived from Enteroviruses, such as Poliovirus are preferred.

*Enterovirus*: Viral antigens may be derived from an Enterovirus, such as Poliovirus types 1, 2 or 3, Coxsackie A virus types 1 to 22 and 24, Coxsackie B virus types 1 to 6, Echovirus (ECHO) virus) types 1 to 9, 11 to 27 and 29 to 34 and Enterovirus 68 to 71. Preferably, the Enterovirus is poliovirus. Enterovirus antigens are preferably selected from one or more of the following Capsid proteins VP1, VP2, VP3 and VP4. Commercially available polio vaccines include Inactivated Polio Vaccine (IPV) and Oral poliovirus vaccine (OPV).

*Heparnavirus*: Viral antigens may be derived from an Heparnavirus, such as Hepatitis A virus (HAV). Commercially available HAV vaccines include inactivated HAV vaccine.

*Togavirus*: Viral antigens may be derived from a Togavirus, such as a Rubivirus, an Alphavirus, or an Arterivirus. Antigens derived from Rubivirus, such as Rubella virus, are preferred. Togavirus antigens may be selected from E1, E2, E3, C, NSP-1, NSPO-2, NSP-3 or NSP-4. Togavirus antigens are preferably selected from E1, E2 or E3. Commercially available Rubella vaccines include a live cold-adapted virus, typically in combination with mumps and measles vaccines (MMR).

*Flavivirus*: Viral antigens may be derived from a Flavivirus, such as Tickborne encephalitis (TBE), Dengue (types 1, 2, 3 or 4), Yellow Fever, Japanese encephalitis, West Nile encephalitis, St. Louis encephalitis, Russian spring-summer encephalitis, Powassan encephalitis. Flavivirus antigens may be selected from PrM, M, C, E, NS-1, NS-2a, NS2b, NS3, NS4a, NS4b, and NS5. Flavivirus antigens are preferably selected from PrM, M and E. Commercially available TBE vaccine include inactivated virus vaccines.

*Pestivirus*: Viral antigens may be derived from a Pestivirus, such as Bovine viral diarrhea (BVDV), Classical swine fever (CSFV) or Border disease (BDV).

*Hepadnavirus*: Viral antigens may be derived from a Hepadnavirus, such as Hepatitis B virus. Hepadnavirus antigens may be selected from surface antigens (L, M and S), core antigens (HBc, HBe). Commercially available HBV vaccines include subunit vaccines comprising the surface antigen S protein.

*Hepatitis C virus*: Viral antigens may be derived from a Hepatitis C virus (HCV). HCV antigens may be selected from one or more of E1, E2, E1/E2, NS345 polyprotein, NS 345-core polyprotein, core, and/or peptides from the nonstructural regions (Houghton et al., *Hepatology* (1991) 14:381).

*Rhabdovirus*: Viral antigens may be derived from a Rhabdovirus, such as a Lyssavirus (Rabies virus) and Vesiculovirus (VSV). Rhabdovirus antigens may be selected from glycoprotein (G), nucleoprotein (N), large protein (L), nonstructural proteins (NS). Commercially available Rabies virus vaccine comprise killed virus grown on human diploid cells or fetal rhesus lung cells.

*Caliciviridae;* Viral antigens may be derived from Calciviridae, such as Norwalk virus.

*Coronavirus:* Viral antigens may be derived from a Coronavirus, SARS, Human respiratory coronavirus, Avian infectious bronchitis (IBV), Mouse hepatitis virus (MHV), and Porcine transmissible gastroenteritis virus (TGEV). Coronavirus antigens may be selected from spike (S), envelope (E), matrix (M), nucleocapsid (N), and Hemagglutinin-esterase glycoprotein (HE). Preferably, the Coronavirus antigen is derived from a SARS virus. SARS viral antigens are described in WO 04/92360;

Retrovirus: Viral antigens may be derived from a Retrovirus, such as an Oncovirus, a Lentivirus or a Spumavirus. Oncovirus antigens may be derived from HTLV-1, HTLV-2 or HTLV-5. Lentivirus antigens may be derived from HIV-1 or HIV-2. Retrovirus antigens may be selected from gag, pol, env, tax, tat, rex, rev, nef, vif, vpu, and vpr. HIV antigens may be selected from gag (p24gag and p55gag), env (gp160 and gp41), pol, tat, nef, rev vpu, miniproteins, (preferably p55 gag and gp140v delete). HIV antigens may be derived from one or more of the following strains: $HIV_{IIIb}$, $HIV_{SF2}$, $HIV_{LAV}$, $HIV_{LAI}$, $HIV_{MN}$, HIV-$1_{CM235}$, HIV-$1_{US4}$.

*Reovirus*: Viral antigens may be derived from a Reovirus, such as an Orthoreovirus, a Rotavirus, an Orbivirus, or a Coltivirus. Reovirus antigens may be selected from structural proteins λ1, λ2, λ3, μl, μ2, σ1, σ2, or σ3, or nonstructural proteins σNS, μNS, or σ1s. Preferred Reovirus antigens may be derived from a Rotavirus. Rotavirus antigens may be selected from VP1, VP2, VP3, VP4 (or the cleaved product VP5 and VP8), NSP 1, VP6, NSP3, NSP2, VP7, NSP4, or NSP5. Preferred Rotavirus antigens include VP4 (or the cleaved product VP5 and VP8), and VP7.

*Parvovirus:* Viral antigens may be derived from a Parvovirus, such as Parvovirus B 19. Parvovirus antigens may be selected from VP-1, VP-2, VP-3, NS-1 and NS-2. Preferably, the Parvovirus antigen is capsid protein VP-2.

*Delta hepatitis virus (HDV):* Viral antigens may be derived HDV, particularly δ-antigen from HDV (see, e.g., U.S. Patent No. 5,378,814).

*Hepatitis E virus (HEV)*: Viral antigens may be derived from HEV.

*Hepatitis G virus (HGV)*: Viral antigens may be derived from HGV.

*Human Herpesvirus:* Viral antigens may be derived from a Human Herpesvirus, such as Herpes Simplex Viruses (HSV), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8). Human Herpesvirus antigens may be selected from immediate early proteins (α), early proteins (β), and late proteins (γ). HSV antigens may be derived from HSV-1 or HSV-2 strains. HSV antigens may be selected from glycoproteins gB, gC, gD and gH, fusion protein (gB), or immune escape proteins (gC, gE, or gI). VZV antigens may be selected from core, nucleocapsid, tegument, or envelope proteins. A live attenuated VZV vaccine is commercially available. EBV antigens may be selected from early antigen (EA) proteins, viral capsid antigen (VCA), and glycoproteins of the membrane antigen (MA). CMV antigens may be selected from capsid proteins, envelope glycoproteins (such as gB and gH), and tegument proteins

*Papovaviruses*: Antigens may be derived from Papovaviruses, such as Papillomaviruses and Polyomaviruses. Papillomaviruses include HPV serotypes 1, 2, 4, 5, 6, 8, 11, 13, 16, 18, 31, 33, 35, 39, 41, 42, 47, 51, 57, 58, 63 and 65. Preferably, HPV antigens are derived from serotypes 6, 11, 16 or 18. HPV antigens may be selected from capsid proteins (L1) and (L2), or E1 - E7, or fusions thereof. HPV antigens are preferably formulated into virus-like particles (VLPs). Polyomyavirus viruses include BK virus and JK virus. Polyomavirus antigens may be selected from VP1, VP2 or VP3.

Further provided are antigens, compositions, methods, and microbes included in Vaccines, 4th Edition (Plotkin and Orenstein ed. 2004); Medical Microbiology 4th Edition (Murray et al. ed. 2002); Virology, 3rd Edition (W.K. Joklik ed. 1988); Fundamental Virology, 2nd Edition (B.N. Fields and D.M. Knipe, eds. 1991), which are contemplated in conjunction with the compositions of the present invention.

**Fungal Antigens**

In some embodiments compositions of the present invention are combined with fungal antigens for use in methods of the present invention, including treatment or prevention of mycoses. Fungal antigens for use herein, associated with vaccines include those described in: U.S. Pat. Nos. 4,229,434 and 4,368,191 for prophylaxis and treatment of trichopytosis caused by Trichophyton mentagrophytes; U.S. Pat. Nos. 5,277,904 and 5,284,652 for a broad spectrum dermatophyte vaccine for the prophylaxis of dermatophyte infection in animals, such as guinea pigs, cats, rabbits, horses and lambs, these antigens comprises a suspension of killed *T. equinum,* T. mentagrophytes (var. granulare), *M. canis* and/or *M. gypseum* in an effective amount optionally combined with an adjuvant; U.S. Pat. Nos. 5,453,273 and 6,132,733 for a ringworm vaccine comprising an effective amount of a homogenized, formaldehyde-killed fungi, i.e., *Microsporum canis* culture in a carrier; U.S. Pat. No. 5,948,413 involving extracellular and intracellular proteins for pythiosis. Additional antigens identified within antifungal vaccines include Ringvac bovis LTF-130 and Bioveta.

Further, fungal antigens for use herein may be derived from Dermatophytres, including: *Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum* var. album, var. discoides, var. ochraceum, *Trichophyton violaceum,* and/or *Trichophyton faviforme.*

Fungal pathogens for use as antigens or in derivation of antigens in conjunction with the compositions of the present invention comprise *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans,*

*Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei,* Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, and Saksenaea spp.

Other fungi from which antigens are derived include Alternaria spp, Curvularia spp, Helminthosporium spp, Fusarium spp, Aspergillus spp, Penicillium spp, Monolinia spp, Rhizoctonia spp, Paecilomyces spp, Pithomyces spp, and Cladosporium spp.

Processes for producing a fungal antigens are well known in the art (see US Patent No. 6,333,164). In a preferred method a solubilized fraction extracted and separated from an insoluble fraction obtainable from fungal cells of which cell wall has been substantially removed or at least partially removed, **characterized in that** the process comprises the steps of: obtaining living fungal cells; obtaining fungal cells of which cell wall has been substantially removed or at least partially removed; bursting the fungal cells of which cell wall has been substantially removed or at least partially removed; obtaining an insoluble fraction; and extracting and separating a solubilized fraction from the insoluble fraction.

## STD Antigens

Embodiments of the invention include compositions and methods related to a prophylactic and therapeutic treatments for microbes that can be neutralized prior to infection of a cell. In particular embodiments, microbes (bacteria, viruses and/or fungi) against which the present compositions and methods can be implement include those that cause sexually transmitted diseases (STDs) and/or those that display on their surface an antigen that can be the target or antigen composition of the invention. In a preferred embodiment of the invention, compositions are combined with antigens derived from a viral or bacterial STD. Antigens derived from bacteria or viruses can be administered in conjunction with the compositions of the present invention to provide protection against at least one of the following STDs, among others: chlamydia, genital herpes, hepatitis (particularly HCV), genital warts, gonorrhea, syphilis and/or chancroid (See, WO00/15255).

In another embodiment the compositions of the present invention are coadministered with an antigen for the prevention or treatment of an STD.

Antigens derived from the following viruses associated with STDs, which are described in greater detail above, are preferred for co-administration with the compositions of the present invention: hepatitis (particularly HCV), HPV, HIV, or HSV.

Additionally, antigens derived from the following bacteria associated with STDs, which are described in greater detail above, are preferred for co-administration with the compositions of the present invention: *Neiserria gonorrhoeae, Chlamydia pneumoniae, Chlamydia trachomatis, Treponema pallidum,* or *Haemophilus ducreyi.*

## Respiratory Antigens

The invention provides methods of preventing and/or treating infection by a respiratory pathogen, including a virus, bacteria, or fungi such as respiratory syncytial virus (RSV), PIV, SARS virus, influenza, *Bacillus anthracis,* particularly by reducing or preventing infection and/or one or more symptoms of respiratory virus infection. A composition comprising an antigen described herein, such as one derived from a respiratory virus, bacteria or fungus is administered in conjunction with the compositions of the present invention to an individual which is at risk of being exposed to that particular respiratory microbe, has been exposed to a respiratory microbe or is infected with a respiratory virus, bacteria or fungus. The composition(s) of the present invention is/are preferably co-administered at the same time or in the same formulation with an antigen of the respiratory pathogen. Administration of the composition results in reduced incidence and/or severity of one or more symptoms of respiratory infection.

## Tumor Antigens

One embodiment of the present involves a tumor antigen or cancer antigen in conjunction with the compositions of the present invention. Tumor antigens can be, for example, peptide-containing tumor antigens, such as a polypeptide tumor antigen or glycoprotein tumor antigens. A tumor antigen can also be, for example, a saccharide-containing tumor antigen, such as a glycolipid tumor antigen or a ganglioside tumor antigen. The tumor antigen can further be, for example, a polynucleotide-containing tumor antigen that expresses a polypeptide-containing tumor antigen, for instance, an RNA

vector construct or a DNA vector construct, such as plasmid DNA.

Tumor antigens appropriate for the practice of the present invention encompass a wide variety of molecules, such as (a) polypeptide-containing tumor antigens, including polypeptides (which can range, for example, from 8-20 amino acids in length, although lengths outside this range are also common), lipopolypeptides and glycoproteins, (b) saccharide-containing tumor antigens, including poly-saccharides, mucins, gangliosides, glycolipids and glycoproteins, and (c) polynucleotides that express antigenic polypeptides.

The tumor antigens can be, for example, (a) full length molecules associated with cancer cells, (b) homologs and modified forms of the same, including molecules with deleted, added and/or substituted portions, and (c) fragments of the same. Tumor antigens can be provided in recombinant form. Tumor antigens include, for example, class I-restricted antigens recognized by CD8+ lymphocytes or class II-restricted antigens recognized by CD4+ lymphocytes.

Numerous tumor antigens are known in the art, including: (a) cancer-testis antigens such as NY-ESO-1, SSX2, SCP1 as well as RAGE, BAGE, GAGE and MAGE family polypeptides, for example, GAGE-1, GAGE-2, MAGE-1, MAGE-2, MAGE-3, MAGE-4, MAGE-5, MAGE-6, and MAGE-12 (which can be used, for example, to address melanoma, lung, head and neck, NSCLC, breast, gastrointestinal, and bladder tumors), (b) mutated antigens, for example, p53 (associated with various solid tumors, e.g., colorectal, lung, head and neck cancer), p21/Ras (associated with, e.g., melanoma, pancreatic cancer and colorectal cancer), CDK4 (associated with, e.g., melanoma), MUM1 (associated with, e.g., melanoma), caspase-8 (associated with, e.g., head and neck cancer), CIA 0205 (associated with, e.g., bladder cancer), HLA-A2-R1701, beta catenin (associated with, e.g., melanoma), TCR (associated with, e.g., T-cell non-Hodgkins lymphoma), BCR-abl (associated with, e.g., chronic myelogenous leukemia), triosephosphate isomerase, KIA 0205, CDC-27, and LDLR-FUT, (c) over-expressed antigens, for example, Galectin 4 (associated with, e.g., colorectal cancer), Galectin 9 (associated with, e.g., Hodgkin's disease), proteinase 3 (associated with, e.g., chronic myelogenous leukemia), WT 1 (associated with, e.g., various leukemias), carbonic anhydrase (associated with, e.g., renal cancer), aldolase A (associated with, e.g., lung cancer), PRAME (associated with, e.g., melanoma), HER-2/neu (associated with, e.g., breast, colon, lung and ovarian cancer), alphafetoprotein (associated with, e.g., hepatoma), KSA (associated with, e.g., colorectal cancer), gastrin (associated with, e.g., pancreatic and gastric cancer), telomerase catalytic protein, MUC-1 (associated with, e.g., breast and ovarian cancer), G-250 (associated with, e.g., renal cell carcinoma), p53 (associated with, e.g., breast, colon cancer), and carcinoembryonic antigen (associated with, e.g., breast cancer, lung cancer, and cancers of the gastrointestinal tract such as colorectal cancer), (d) shared antigens, for example, melanoma-melanocyte differentiation antigens such as MART-1/Melan A, gp100, MC1R, melanocyte-stimulating hormone receptor, tyrosinase, tyrosinase related protein-1/TRP1 and tyrosinase related protein-2/TRP2 (associated with, e.g., melanoma), (e) prostate associated antigens such as PAP, PSA, PSMA, PSH-P1, PSM-P1, PSM-P2, associated with e.g., prostate cancer, (f) immunoglobulin idiotypes (associated with myeloma and B cell lymphomas, for example), and (g) other tumor antigens, such as polypeptide- and saccharide-containing antigens including (i) glycoproteins such as sialyl Tn and sialyl $Le^x$ (associated with, e.g., breast and colorectal cancer) as well as various mucins; glycoproteins may be coupled to a carrier protein (e.g., MUC-1 may be coupled to KLH); (ii) lipopolypeptides (e.g., MUC-1 linked to a lipid moiety); (iii) polysaccharides (e.g., Globo H synthetic hexasaccharide), which may be coupled to a carrier proteins (e.g., to KLH), (iv) gangliosides such as GM2, GM12, GD2, GD3 (associated with, e.g., brain, lung cancer, melanoma), which also may be coupled to carrier proteins (e.g., KLH). Additional tumor antigens which are known in the art include p15, Hom/Mel-40, H-Ras, E2A-PRL, H4-RET, IGH-IGK, MYL-RAR, Epstein Barr virus antigens, EBNA, human papillomavirus (HPV) antigens, including E6 and E7, hepatitis B and C virus antigens, human T-cell lymphotropic virus antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791 Tgp72, beta-HCG, BCA225, BTAA, CA 125, CA 15-3 (CA 27.29\BCAA), CA 195, CA 242, CA-50, CAM43, CD68\KP1, CO-029, FGF-5, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, TPS, and the like. These as well as other cellular components are described for example in United States Patent Application 20020007173 and references cited therein.

Polynucleotide-containing antigens in accordance with the present invention typically comprise polynucleotides that encode polypeptide cancer antigens such as those listed above. Preferred polynucleotide-containing antigens include DNA or RNA vector constructs, such as plasmid vectors (e.g., pCMV), which are capable of expressing polypeptide cancer antigens *in vivo*.

Tumor antigens may be derived, for example, from mutated or altered cellular components. After alteration, the cellular components no longer perform their regulatory functions, and hence the cell may experience uncontrolled growth. Representative examples of altered cellular components include ras, p53, Rb, altered protein encoded by the Wilms' tumor gene, ubiquitin, mucin, protein encoded by the DCC, APC, and MCC genes, as well as receptors or receptor-like structures such as neu, thyroid hormone receptor, platelet derived growth factor (PDGF) receptor, insulin receptor, epidermal growth factor (EGF) receptor, and the colony stimulating factor (CSF) receptor. These as well as other cellular components are described for example in U.S. Patent No. 5,693,522 and references cited therein.

Additionally, bacterial and viral antigens, may be used in conjunction with the compositions of the present

invention for the treatment of cancer. In particular, carrier proteins, such as $CRM_{197}$, tetanus toxoid, or *Salmonella typhimurium* antigen can be used in conjunction/conjugation with compounds of the present invention for treatment of cancer. The cancer antigen combination therapies will show increased efficacy and bioavailability as compared with existing therapies.

Additional information on cancer or tumor antigens can be found, for example, in Moingeon P, "Cancer vaccines," Vaccine, 2001, 19:1305-1326; Rosenberg SA, "Progress in human tumor immunology and immunotherapy," Nature, 2001, 411:380-384; Dermine, S. et al, "Cancer Vaccines and Immunotherapy," British Medical Bulletin, 2002, 62, 149-162; Espinoza-Delgado I., "Cancer Vaccines," The Oncologist, 2002, 7(suppl3):20-33; Davis, I.D. et al., "Rational approaches to human cancer immunotherapy," Journal of Leukocyte Biology, 2003, 23: 3-29; Van den Eynde B, et al., "New tumor antigens recognized by T cells," Curr. Opin. Immunol., 1995, 7:674-81; Rosenberg SA, "Cancer vaccines based on the identification of genes encoding cancer regression antigens, Immunol. Today, 1997, 18:175-82; Offringa R et al., "Design and evaluation of antigen-specific vaccination strategies against cancer," Current Opin. Immunol., 2000, 2:576-582; Rosenberg SA, "A new era for cancer immunotherapy based on the genes that encode cancer antigens," Immunity, 1999, 10:281-7; Sahin U et al., "Serological identification of human tumor antigens," Curr. Opin. Immunol., 1997, 9:709-16; Old LJ et al., "New paths in human cancer serology," J. Exp. Med., 1998, 187:1163-7; Chaux P, et al., "Identification of MAGE-3 epitopes presented by HLA-DR molecules to CD4(+) T lymphocytes," J. Exp. Med., 1999, 189:767-78; Gold P, et al., "Specific carcinoembryonic antigens of the human digestive system," J. Exp. Med., 1965, 122:467-8; Livingston PO, et al., Carbohydrate vaccines that induce antibodies against cancer: Rationale," Cancer Immunol. Immunother., 1997, 45:1-6; Livingston PO, et al., Carbohydrate vaccines that induce antibodies against cancer: Previous experience and future plans," Cancer Immunol. Immunother., 1997, 45:10-9; Taylor-Papadimitriou J, "Biology, biochemistry and immunology of carcinoma-associated mucins," Immunol. Today, 1997, 18:105-7; Zhao X-J et al., "GD2 oligosaccharide: target for cytotoxic T lymphocytes," J. Exp. Med., 1995, 182:67-74; Theobald M, et al., "Targeting p53 as a general tumor antigen," Proc. Natl. Acad. Sci. USA, 1995, 92:11993-7; Gaudernack G, "T cell responses against mutant ras: a basis for novel cancer vaccines," Immunotechnology, 1996, 2:3-9; WO 91/02062; U.S. Patent No. 6,015,567; WO 01/08636; WO 96/30514; U.S. Patent No. 5,846,538; and U.S. Patent No. 5,869,445.

**Pediatric/Geriatric Antigens**

In one embodiment the compositions of the present invention are used in conjunction with an antigen for treatment of a pediatric population, as in a pediatric antigen. In a more particular embodiment the pediatric population is less than about 3 years old, or less than about 2 years, or less than about 1 years old. In another embodiment the pediatric antigen (in conjunction with the composition of the present invention) is administered multiple times over at least 1, 2, or 3 years.

In another embodiment the compositions of the present invention are used in conjunction with an antigen for treatment of a geriatric population, as in a geriatric antigen.

**Other Antigens**

Other antigens for use in conjunction with the compositions of the present include hospital acquired (nosocomial) associated antigens.

In another embodiment, parasitic antigens are contemplated in conjunction with the compositions of the present invention. Examples of parasitic antigens include those derived from organisms causing malaria and/or Lyme disease.

In another embodiment, the antigens in conjunction with the compositions of the present invention are associated with or effective against a mosquito born illness. In another embodiment, the antigens in conjunction with the compositions of the present invention are associated with or effective against encephalitis. In another embodiment the antigens in conjunction with the compositions of the present invention are associated with or effective against an infection of the nervous system.

In another embodiment, the antigens in conjunction with the compositions of the present invention are antigens transmissible through blood or body fluids.

**Antigen Formulations**

In other aspects of the invention, methods of producing microparticles having adsorbed antigens are provided. The methods comprise: (a) providing an emulsion by dispersing a mixture comprising (i) water, (ii) a detergent, (iii) an organic solvent, and (iv) a biodegradable polymer selected from the group consisting of a poly($\alpha$-hydroxy acid), a polyhydroxy butyric acid, a polycaprolactone, a polyorthoester, a polyanhydride, and a polycyanoacrylate. The polymer is typically present in the mixture at a concentration of about 1% to about 30% relative to the organic solvent, while the detergent is typically present in the mixture at a weight-to-weight detergent-to-polymer ratio of from about 0.00001:1 1

to about 0.1:1 (more typically about 0.0001:1 1 to about 0.1:1, about 0.001:1 1 to about 0.1:1, or about 0.005:1 to about 0.1:1); (b) removing the organic solvent from the emulsion; and (c) adsorbing an antigen on the surface of the microparticles. In certain embodiments, the biodegradable polymer is present at a concentration of about 3% to about 10% relative to the organic solvent.

Microparticles for use herein will be formed from materials that are sterilizable, non-toxic and biodegradable. Such materials include, without limitation, poly($\alpha$-hydroxy acid), polyhydroxybutyric acid, polycaprolactone, polyorthoester, polyanhydride, PACA, and polycyanoacrylate. Preferably, microparticles for use with the present invention are derived from a poly($\alpha$-hydroxy acid), in particular, from a poly(lactide) ("PLA") or a copolymer of D,L-lactide and glycolide or glycolic acid, such as a poly(D,L-lactide-co-glycolide) ("PLG" or "PLGA"), or a copolymer of D,L-lactide and caprolactone. The microparticles may be derived from any of various polymeric starting materials which have a variety of molecular weights and, in the case of the copolymers such as PLG, a variety of lactide:glycolide ratios, the selection of which will be largely a matter of choice, depending in part on the coadministered macromolecule. These parameters are discussed more fully below.

Further antigens may also include an outer membrane vesicle (OMV) preparation.

Additional formulation methods and antigens (especially tumor antigens) are provided in U.S. Patent Serial No. 09/581,772.

**Antigen References**

The following references include antigens useful in conjunction with the compositions and methods of the present invention:


1 International patent application WO99/24578
2 International patent application WO99/36544.
3 International patent application WO99/57280.
4 International patent application WO00/22430.
5 Tettelin et al. (2000) Science 287:1809-1815.
6 International patent application WO96/29412.
7 Pizza et al. (2000) Science 287:1816-1820.
8 PCT WO 01/52885.
9 Bjune et al. (1991) Lancet 338(8775).
10 Fuskasawa et al. (1999) Vaccine 17:2951-2958.
11 Rosenqist et al. (1998) Dev. Biol. Strand 92:323-333.
12 Constantino et al. (1992) Vaccine 10:691-698.
13 Constantino et al. (1999) Vaccine 17:1251-1263.
14 Watson (2000) Pediatr Infect Dis J 19:331-332.
15 Rubin (20000) Pediatr Clin North Am 47:269-285,v.
16 Jedrzejas (2001) Microbiol Mol Biol Rev 65:187-207.
17 International patent application filed on 3rd July 2001 claiming priority from GB-0016363.4;WO 02/02606; PCT IB/01/00166.
18 Kalman et al. (1999) Nature Genetics 21:385-389.
19 Read et al. (2000) Nucleic Acids Res 28:1397-406.
20 Shirai et al. (2000) J. Infect. Dis 181(Suppl 3):S524-S527.
21 International patent application WO99/27105.
22 International patent application WO00/27994.
23 International patent application WO00/37494.
24 International patent application WO99/28475.
25 Bell (2000) Pediatr Infect Dis J 19:1187-1188.
26 Iwarson (1995) APMIS 103:321-326.
27 Gerlich et al. (1990) Vaccine 8 Suppl:S63-68 & 79-80.
28 Hsu et al. (1999) Clin Liver Dis 3:901-915.
29 Gastofsson et al. (1996) N. Engl. J. Med. 334-:349-355.
30 Rappuoli et al. (1991) TIBTECH 9:232-238.
31 Vaccines (1988) eds. Plotkin & Mortimer. ISBN 0-7216-1946-0.

(continued)


32 Del Guidice et al. (1998) Molecular Aspects of Medicine 19:1-70.
33 International patent application WO93/018150.
34 International patent application WO99/53310.
35 International patent application WO98/04702.
36 Ross et al. (2001) Vaccine 19:135-142.
37 Sutter et al. (2000) Pediatr Clin North Am 47:287-308.
38 Zimmerman & Spann (1999) Am Fan Physician 59:113-118, 125-126.
39 Dreensen (1997) Vaccine 15 Suppl"S2-6.
40 MMWR Morb Mortal Wkly rep 1998 Jan 16:47(1):12, 9.
41 McMichael (2000) Vaccine19 Suppl 1:S101-107.
42 Schuchat (1999) Lancer 353(9146):51-6.
43 GB patent applications 0026333.5, 0028727.6 & 0105640.7.
44 Dale (1999) Infect Disclin North Am 13:227-43, viii.
45 Ferretti et al. (2001) PNAS USA 98: 4658-4663.
46 Kuroda et al. (2001) Lancet 357(9264):1225-1240; see also pages 1218-1219.
47 Ramsay et al. (2001) Lancet 357(9251):195-196.
48 Lindberg (1999) Vaccine 17 Suppl 2:S28-36.
49 Buttery & Moxon (2000) J R Coil Physicians Long 34:163-168.
50 Ahmad & Chapnick (1999) Infect Dis Clin North Am 13:113-133, vii.
51 Goldblatt (1998) J. Med. Microbiol. 47:663-567.
52 European patent 0 477 508.
53 U.S. Patent No. 5,306,492.
54 International patent application WO98/42721.
55 Conjugate Vaccines (eds. Cruse et al.) ISBN 3805549326, particularly vol. 10:48-114.
56 Hermanson (1996) Bioconjugate Techniques ISBN: 012323368 & 012342335X.
57 European patent application 0372501.
58 European patent application 0378881.
59 European patent application 0427347.
60 International patent application WO93/17712.
61 International patent application WO98/58668.
62 European patent application 0471177.
63 International patent application WO00/56360.
64 International patent application WO00/67161.


It is contemplated that the invention encompasses all possible combinations of the embodiments described herein.

## EXAMPLES

### Preparation of Quinazolines

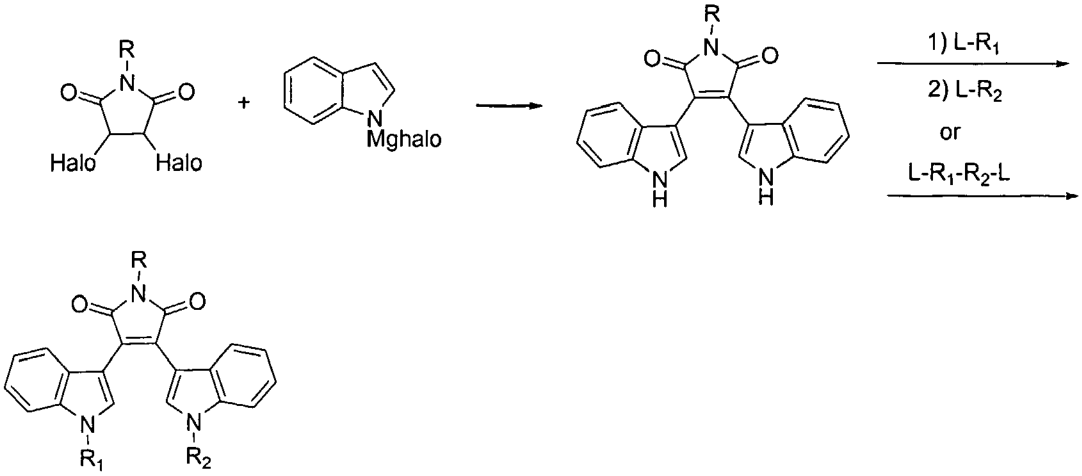


In the above reaction, halo is preferably chloro, bromo, or iodo. The reaction is a Grignard, carried out in an inert organic solvent, such as toluene, at a temperature between room temperature and the reflux temperature of the reaction mixture. Most significantly, the reaction is dependent on solvent conditions. When carried out in a Toluene: THF:ether solvent system, the reaction provides the product in high yield. The product is precipitated from the reaction mixture with ammonium chloride ($NH_4Cl$). The resulting bis-3,4(3'-indolyl)-1N-pyrrole-2,5-dione product, may be isolated by standard techniques.

In the next step, L is a good leaving group such as chloro, bromo, iodo, mesyl, tosyl, and the like. L may also be a hydroxy or other precursor that may be readily converted to a good leaving group by techniques known in the art. For example, the hydroxy may be readily converted to a sulfonic ester such as mesyl by reacting the hydroxy with methanesulfonyl chloride to produce the mesylate leaving group. The reaction is accomplished by any of the known methods of preparing N-substituted indoles. This reaction usually involves approximately equimolar amounts of the two reagents, although other ratios, especially those wherein the alkylating reagent is in excess, are operative. The reaction is best carried out in a polar aprotic solvent employing an alkali metal salt or other such alkylation conditions as are appreciated in the art. When the leaving group is bromo or chloro, a catalytic amount of iodide salt, such as potassium iodide may be added to speed the reaction. Reaction conditions include the following: Potassium hexamethyldisilazide in dimethylformamide or tetrahydrofuran, sodium hydride in dimethylformamide.

Preferably, the reaction is carried out under slow reverse addition with cesium carbonate in either acetonitrile, dimethylformamide (DMF), or tetrahydrofuran (THF). The temperature of the reaction is preferably from about ambient temperature to about the reflux temperature of the reaction mixture.

**Tabl 1:** 3,4-di(1H-indol-3-yl)-1H-pyrrole-2,5-dione compounds

| Example | Structure |
|---|---|
| 1 | 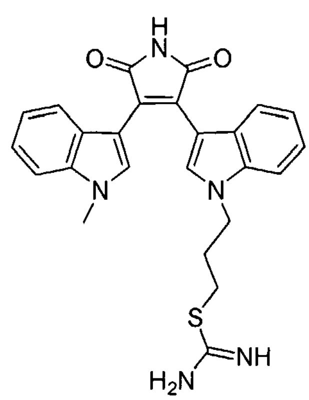 |

(continued)

| Example | Structure |
|---|---|
| 2 | 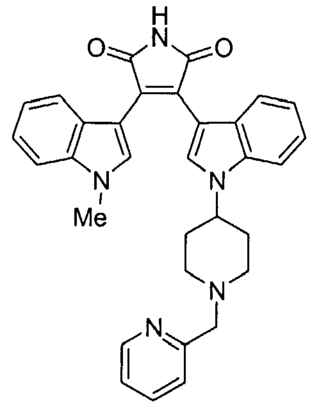 |
| 3 | 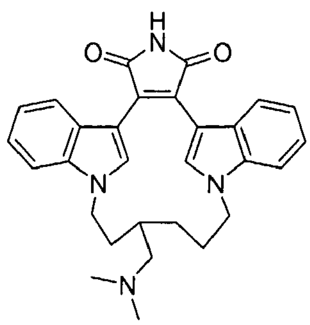 |
| 4 | 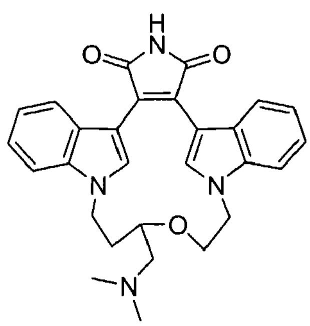 |

**Preparation of Indolinones**

Scheme 1:

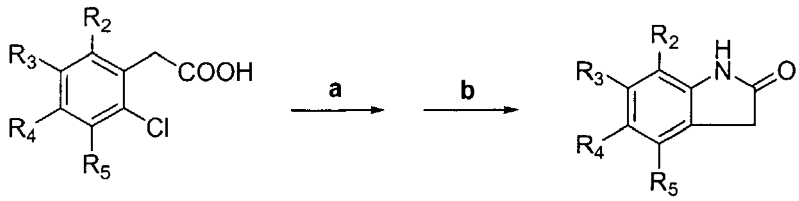


Scheme 1 is performed as a one pot procedure, with reagents in step a being $NH_4OH$, CuCl in $H_2O$. Followed by addition of aq. HCl in step b. $R_2$-$R_5$ are as defined herein.

Scheme 2:

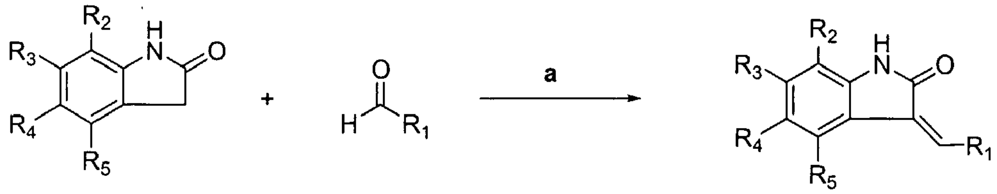


In Scheme 2, the reagents are stirred in EtOH in the presence of piperidine (a) to afford the final product. As will be apparent to a skilled artisan, the reaction may be heated to enhance the yield, depending on reactivity of the particular starting materials.

Scheme 3:

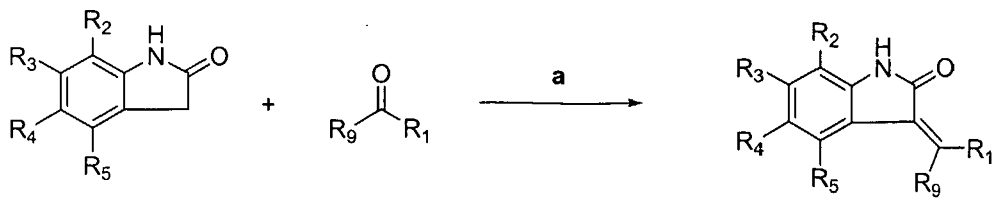


In Scheme 3, the reagents are refluxed in EtOH in the presence of NaOBu-t (a) to afford the final product. $R_9$ as shown in scheme 3 is H, -OH, -CN, alkyl, aryl, heterocyclyl, alkoxy, or $-NR_aR_b$ as defined herein. It is contemplated that the above structure may replace Formula I to allow substitution at $R_9$, whereby all other substituents are as defined herein.

Scheme 4 (Synthesis of Example 1):

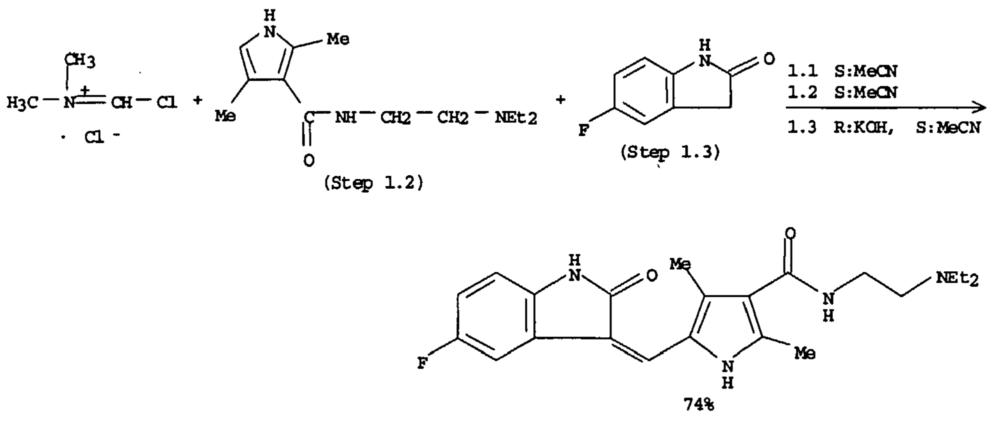

Table 1: Indolinone Compounds

| Example | Name | Structure | Reference |
|---|---|---|---|
| 1 | N-(2-(dimethylamino) ethyl)-5-((5-fluoro-2-oxoindolin-3-ylidene) methyl)-2,4-diethyl-1H-pyrrole-3-carboxamide | 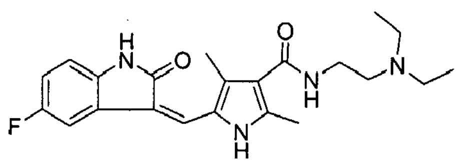 | WO 01/60814 |
| 2 | 3-((3,5-dimethyl-1H-pyrrol-2-yl)methylene) indolin-2-one | 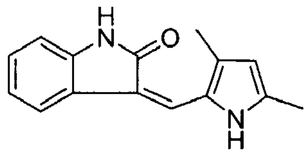 | US 5,883,113 WO 99/61422 |
| 3 | 3-(2,4-dimethyl-5-((2-oxoindolin-3-ylidene) methyl)-1H-pyrrol-3-yl) propanoic acid | 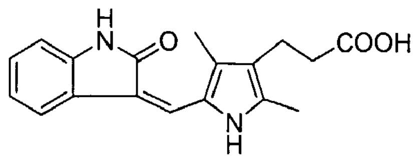 | US 5,883,113 WO 99/61422 |
| 4 | 3-(3,5-dibromo-4-hydroxybenzylidene)-5-iodoindolin-2-one | 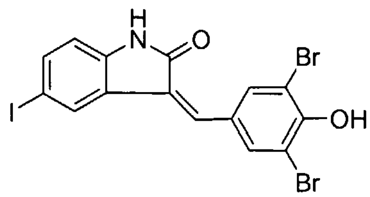 | Lackey, K., et al. 2000 *Bioorg. Med. Chem. Lett.* **10**, 223 |
| | | | |
| Example | Name | Structure | Reference |
| 5 | 3-((furan-2-yl)methylene) indolin-2-one | 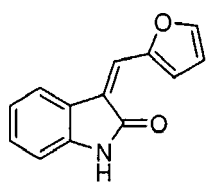 | 212.2 |
| 6 | 3-(2,4,6-trimethoxybenzylidene) indol in-2-one | 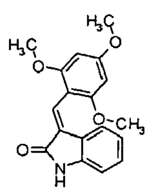 | 312.3 |
| 7 | 3-((5-morpholino-1H-benzo[d]imidazol-2-yl) methylene)indolin-2-one | 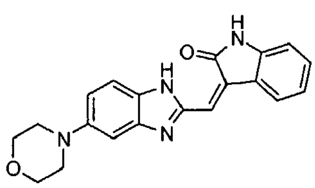 | 347.4 |

**Preparation of Example Chromen-4-ones**

**Scheme 1:** Synthesis of general flavones

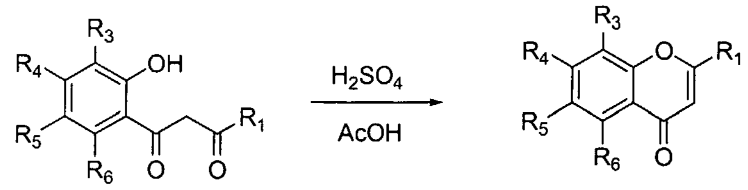


**Scheme 2:** Synthesis of general isoflavones

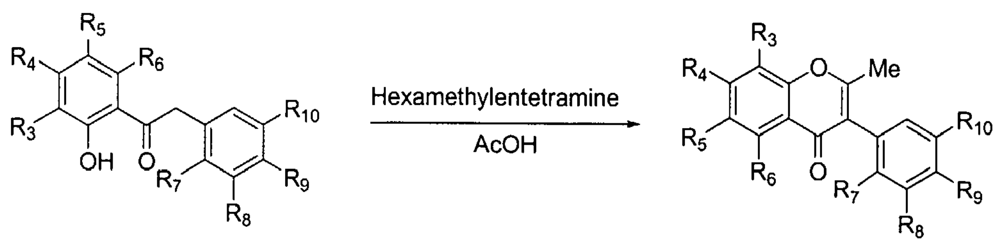


**Scheme 3:** Synthesis of 2-methyl-4-oxo-3-phenyl-4H-chromen-7-yl acetate

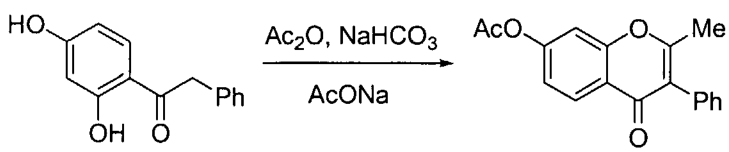


**Scheme 4:** Synthesis of 5,7-dihydroxy-2-(3,4-dihydroxyphenyl)-4H-chromen-4-one

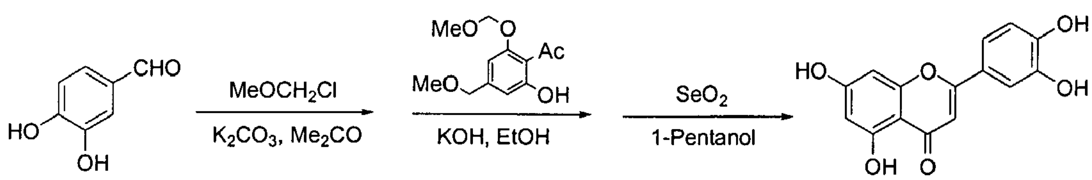


Further, it will become apparent to one skilled in the art that many chromen-4-ones, particularly flavones and isoflavones, are commercially available, including many of the Examples disclosed herein, as well as starting materials that can be functionalized to arrive at the desired final product.

**Table 1:** Chromen-4-one SMIPs

| Example | Structure | Name | Patent |
|---|---|---|---|
| 1 | 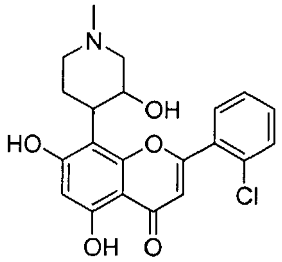 | 2-(2-chlorophenyl)-2,3-dihydro-5,7-dihydroxy-8-(3-hydroxy-1-methylpiperidin-4-yl)chromen-4-one | WO 97/42949 WO 98/13344 |
| 2 | 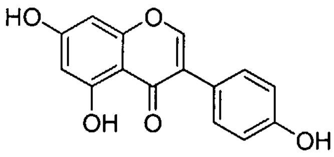 | 5,7-dihydroxy-3-(4-hydroxyphenyl)-4H-chromen-4-one | US 5,554,519 |
| 3 | 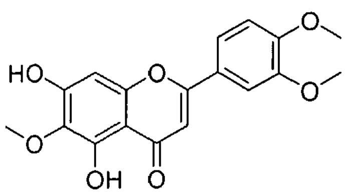 | 2,3-dihydro-5,7-dihydroxy-6-methoxy-2-(3,4-dimethoxyphenyl)chromen-4-one | WO 98/04541 US 6,025,387 |

**Table 2:** Examples 4-49 3-phenylchromen-4-one (Isoflavone) SMIPs

| Example | Structure | MH+ |
|---|---|---|
| 4 | 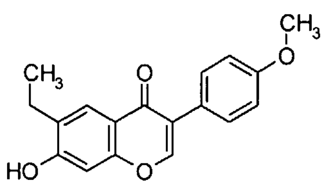 | 297.3 |
| 5 | 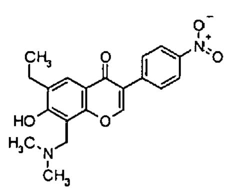 | 369.4 |
| 6 | 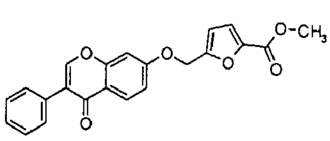 | 377.4 |
| 7 | 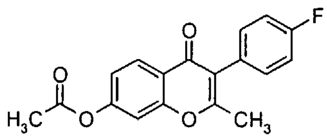 | 313.3 |

(continued)

| Example | Structure | MH+ |
|---|---|---|
| 8 | 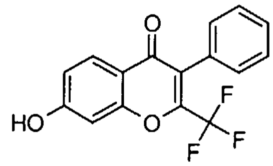 | 307.2 |
| 9 | 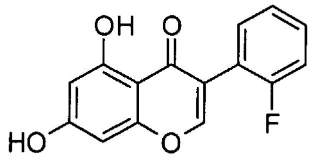 | 273.2 |
| 10 | 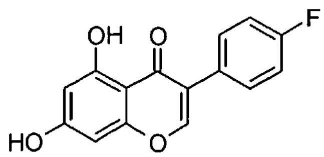 | 273.2 |
| 11 | 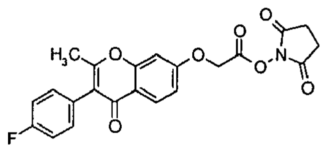 | 426.4 |
| 12 | 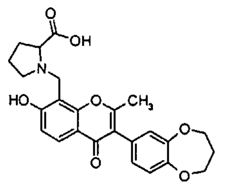 | 452.5 |
| 13 | 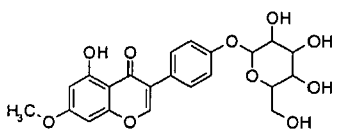 | 447.4 |
| 14 | 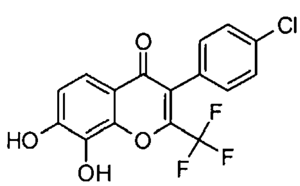 | 357.7 |
| 15 | 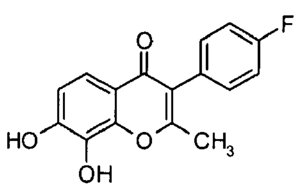 | 287.3 |

(continued)

| Example | Structure | MH+ |
|---|---|---|
| 16 | 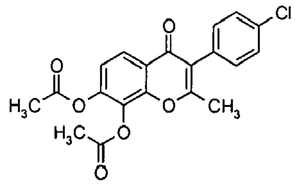 | 387.8 |
| 17 | 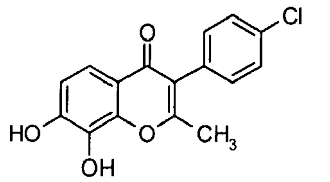 | 303.7 |
| 18 | 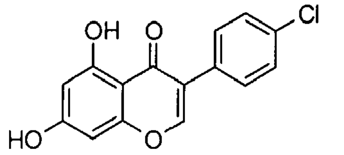 | 289.7 |
| 19 | 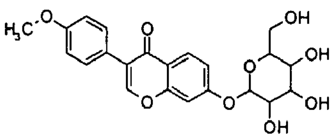 | 431.4 |
| 20 | 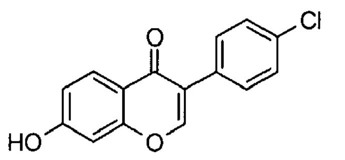 | 273.7 |
| 21 | 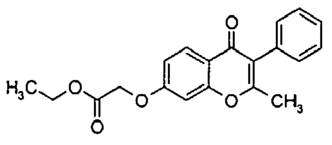 | 339.4 |
| 22 | 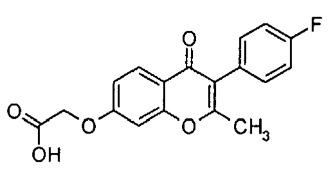 | 329.3 |
| 23 | 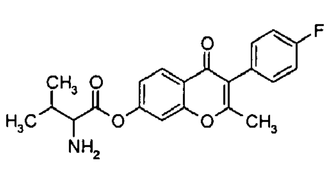 | 370.4 |

(continued)

| Example | Structure | MH+ |
|---|---|---|
| 24 | 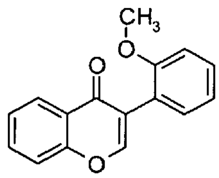 | 253.3 |
| 25 | 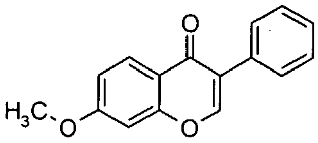 | 253.3 |
| 26 | 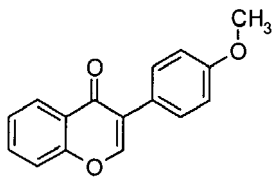 | 253.3 |
| 27 | 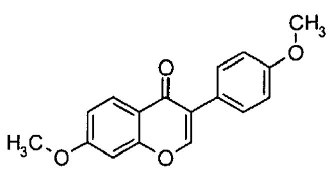 | 283.3 |
| 28 | 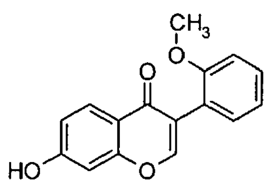 | 269.3 |
| 29 | 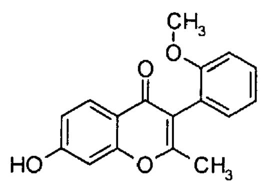 | 283.3 |
| 30 | 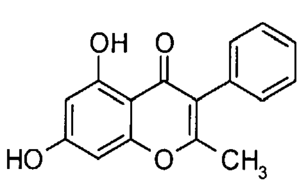 | 269.3 |
| 31 | 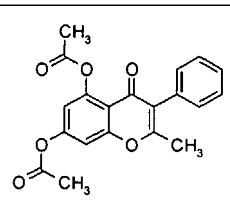 | 353.3 |

(continued)

| Example | Structure | MH+ |
|---|---|---|
| 32 | 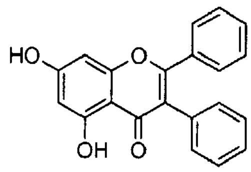 | 331.3 |
| 33 | 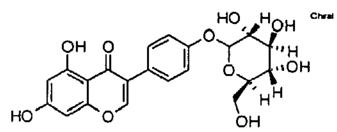 | 433.4 |
| 34 | 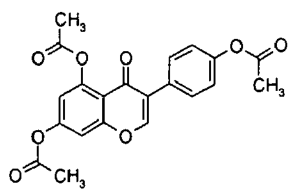 | 397.3 |
| 35 | 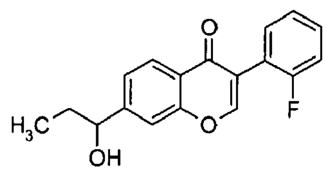 | 299.3 |
| 36 | 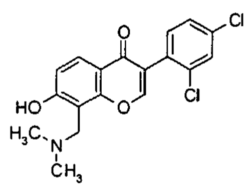 | 365.2 |
| 37 | 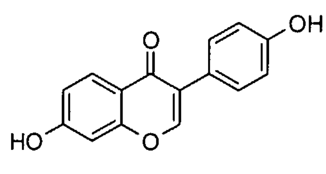 | 255.2 |
| 38 | 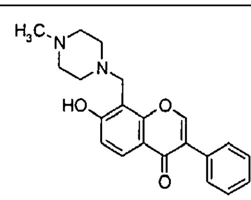 | 351.4 |
| 39 | 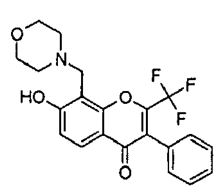 | 406.4 |

(continued)

| Example | Structure | MH+ |
|---|---|---|
| 40 | 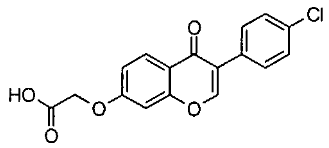 | 331.7 |
| 41 | 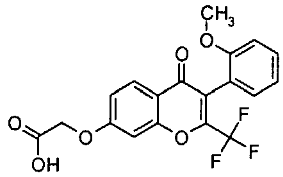 | 395.3 |
| 42 | 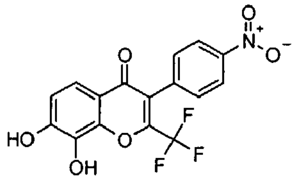 | 368.2 |
| 43 | 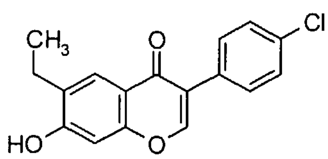 | 301.7 |
| 44 | 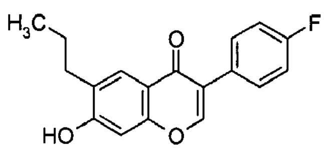 | 299.3 |
| 45 | 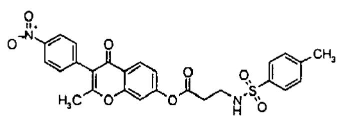 | 523.5 |
| 46 | 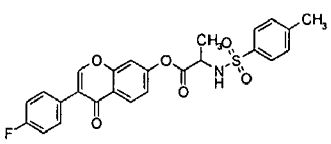 | 482.5 |
| 47 | 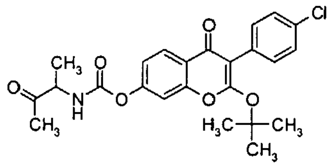 | 458.9 |

(continued)

| Example | Structure | MH+ |
|---|---|---|
| 48 | 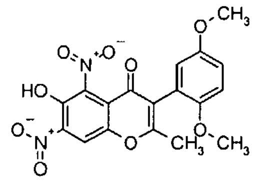 | 403.3 |
| 49 | 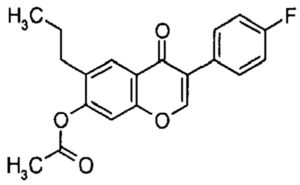 | 341.3 |

**Table 3:** Examples 50-100 2-Phenylchromen-4-one (Flavone) SMIPs

| Examples | Structure | MH+ |
|---|---|---|
| 50 | 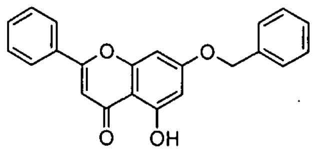 | 345.4 |
| 51 | 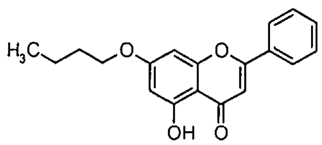 | 311.3 |
| 52 | 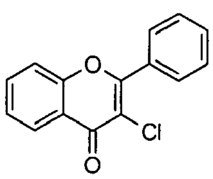 | 257.7 |
| 53 | 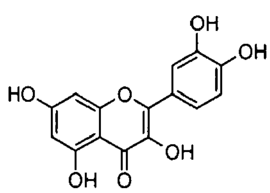 | 303.2 |
| 54 | 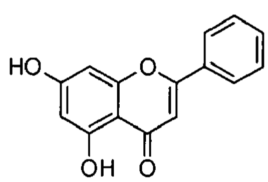 | 255.2 |

(continued)

| Examples | Structure | MH+ |
|---|---|---|
| 55 | 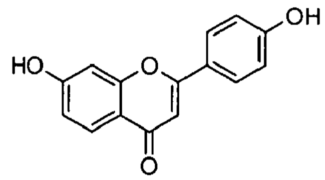 | 255.2 |
| 56 | 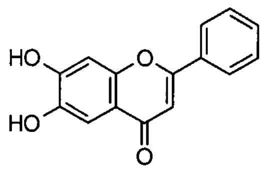 | 255.2 |
| 57 | 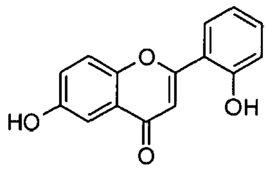 | 255.2 |
| 58 | 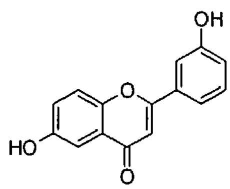 | 255.2 |
| 59 | 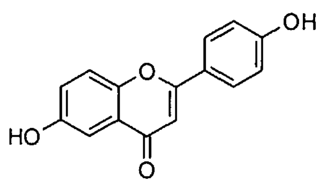 | 255.2 |
| 60 | 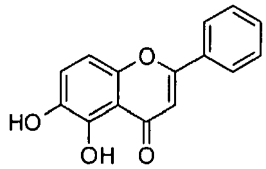 | 255.2 |
| 61 | 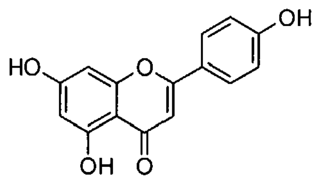 | 271.2 |
| 62 | 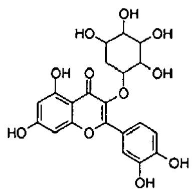 | 449.4 |

(continued)

| Examples | Structure | MH+ |
|---|---|---|
| 63 | 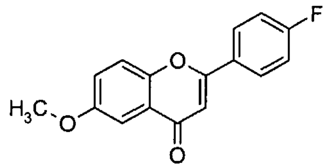 | 271.3 |
| 64 | 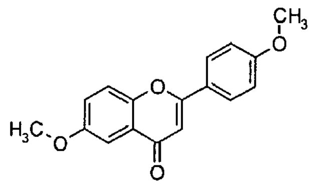 | 283.3 |
| 65 | 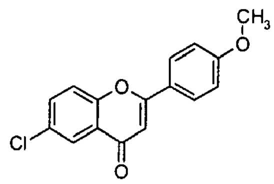 | 287.7 |
| 66 | 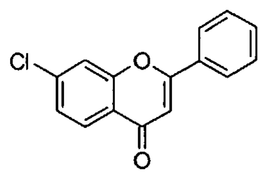 | 257.7 |
| 67 | 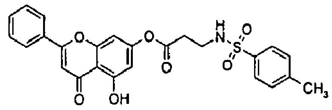 | 480.5 |
| 68 | 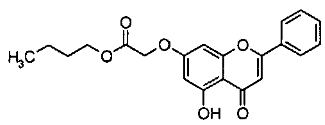 | 369.4 |
| 69 | 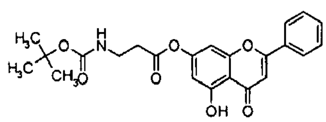 | 426.4 |
| 70 | 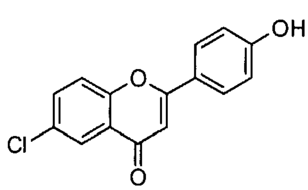 | 273.7 |

(continued)

| Examples | Structure | MH+ |
|---|---|---|
| 71 | 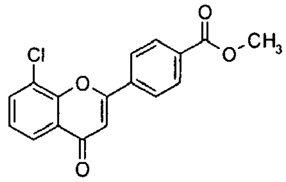 | 315.7 |
| 72 | 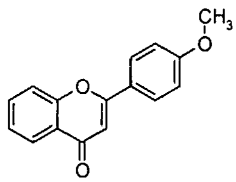 | 253.3 |
| 73 | 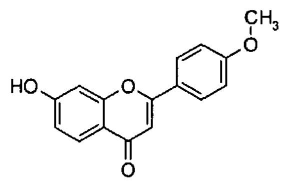 | 269.3 |
| 74 | 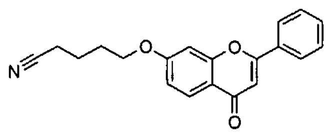 | 320.4 |
| 75 | 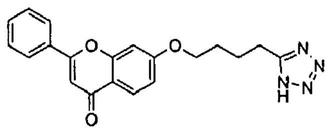 | 363.4 |
| 76 | 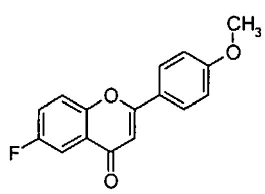 | 271.3 |
| 77 | 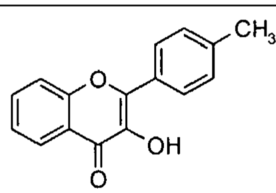 | 253.3 |
| 78 | 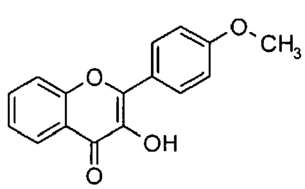 | 269.3 |

(continued)

| Examples | Structure | MH+ |
|---|---|---|
| 79 | 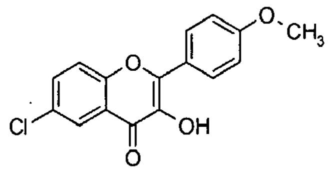 | 303.7 |
| 80 | 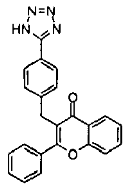 | 381.4 |
| 81 | 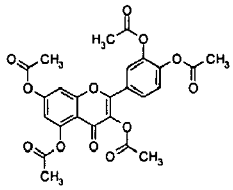 | 513.4 |
| 82 | 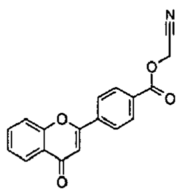 | 306.3 |
| 83 | 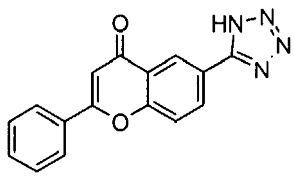 | 291.3 |
| 84 | 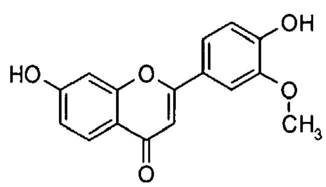 | 285.3 |
| 85 | 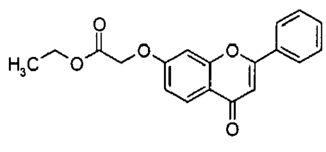 | 325.3 |
| 86 | 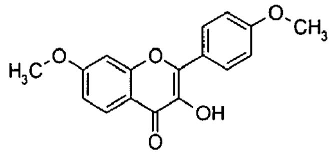 | 299.3 |

(continued)

| Examples | Structure | MH+ |
|---|---|---|
| 87 | 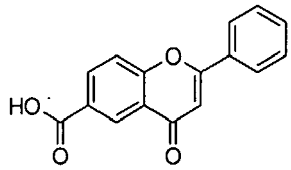 | 267.3 |
| 88 | 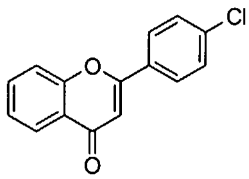 | 257.7 |
| 89 | 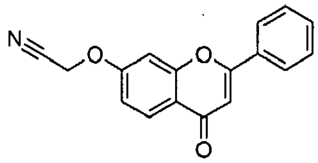 | 278.3 |
| 90 | 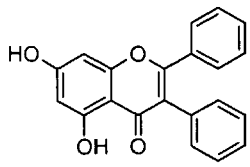 | 331.3 |
| 91 | 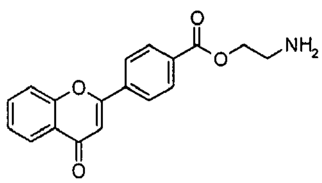 | 310.3 |
| 92 | 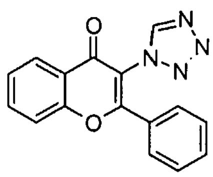 | 291.3 |
| 93 | 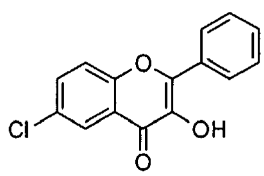 | 273.7 |
| 94 | 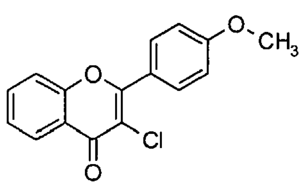 | 287.7 |

(continued)

| Examples | Structure | MH+ |
|---|---|---|
| 95 | 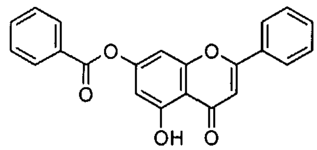 | 359.3 |
| 96 | 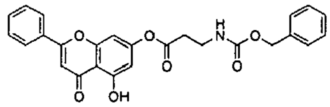 | 460.5 |
| 97 | 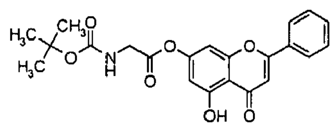 | 412.4 |
| 98 | 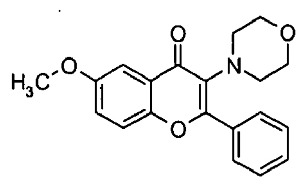 | 338.4 |
| 99 | 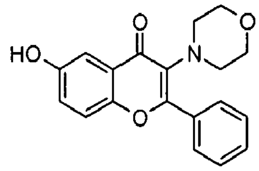 | 324.3 |
| 100 | 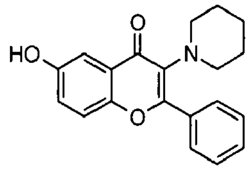 | 322.4 |

**Preparation of Derivatized Pyridazines**

Preparation of the following Examples 60-68 were described in US 6,258,812, which also includes other reaction schemes that may be helpful in synthesizing the compounds of the present invention.

Example 60:

1-(4-Chloroanilino)-4-(4-pyridylmethyl)phthalazine dihydrochloride

A mixture of 15.22 g (59.52 mmol) 1-chloro-4-(4-pyridylmethyl)phthalazine (for preparation see German Auslegeschriftno. 1 061 788 published Jul. 23, 1959]), 7.73 g (60.59 mmol) 4-chloroaniline and 200 ml 1-butanol is heated for 2 h under reflux. The crystallizate which is obtained when the mixture slowly cools to 5° C. is then filtered off and washed with 1-butanol and ether. The filter residue is dissolved in about 200 ml hot methanol, the solution is treated with 0.75 g activated carbon and filtered Via a Hyflo Super Cel, and the pH of the filtrate is adjusted to about 2.5 with 7 ml 3N methanolic HCl. The filtrate is evaporated to about half the original volume and ether added until slight turbidity occurs; cooling then leads to the precipitation of crystals. The crystallizate is filtered off, washed with a mixture of methanol/ether (1:2) as well as ether, dried for 8 h at 110° C. under HV, and equilibrated for 72 h at 20° C. and in room atmosphere. In this way, the title compound is obtained with a water content of 8.6%; m.p. >270° C. ; $^1$H NMR (DMSO-

d 6 ) 11.05-12.20 (br), 9.18-9.23 (m, 1H), 8. 88 (d, 2H), 8.35-8.40 (m, 1H), 8.18-8.29 (m, 2H), 8.02 (d, 2H), 7.73 (d, 2H), 7.61 (d, 2H), 5.02 (s, 2H); ESI-MS: $(M+H)^+$ =347.

Example 61:

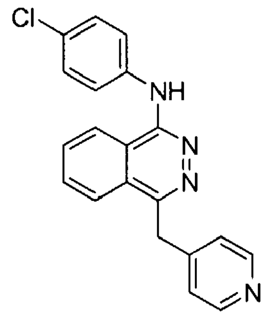

1-(4-Chloroanilino)-4-(4-pyridylmethyl)phthalazine hydrochloride

A mixture of 0.972 g (3.8 mmol) 1-chloro-4-(4- pyridylmethyl)phthalazine, 0.656 g (4 mmol) 4-chloroaniline hydrochloride (Research Organics, Inc., Cleveland, Ohio, USA) and 20 ml ethanol is heated for 2 h under reflux. The reaction mixture is cooled in an ice bath, filtered, and the crystallizate washed with a little ethanol and ether. After drying under HV for 8 h at 110° C. and for 10 h at 150° C., the title compound is obtained as a result of thermal removal of HCl; m.p. >270° C.; $^1$H NMR (DMSO-d 6 ) 9.80-11.40 (br), 8.89-8.94 (m, 1H), 8.67 (d, 2H), 8.25-8.30 (m, 1H), 8.06-8.17 (m, 2H), 7.87 (d, 2H), 7. 69 (d, 2H), 7.49 (d, 2H), 4.81 (s, 2H); ESI-MS: $(M+H)^+$ =347.

Example 62

1-(4-Chloroanilino)-4-(4-pyridylmethyl)phthalazine hydrochloride

A mixture of 1.28 g (5 mmol) 1-chloro-4-(4-pyridylmethyl)phthalazine, 0.67 g (5.25 mmol) 4-chloroaniline and 15 ml 1-butanol is heated for 0.5 h at 100 C while stirring in a nitrogen atmosphere. The mixture is then cooled to RT, filtered, and the filtrate washed with 1-butanol and ether. For purification, the crystallizate is dissolved in 40 ml of hot methanol, the solution treated with activated carbon, filtered via Hyflo Super Cel, and the filtrate evaporated to about half its original volume, resulting in the formation of a crystalline precipitate. After cooling to 0° C., filtration, washing of the filter residue with ether, and drying under HV for 8 h at 130° C., the title compound is obtained; m.p. >270° C.; $^1$H NMR (DMSO-d 6 ) 9.80-11.40 (br), 8.89-8.94 (m, 1H), 8.67 (d, 2H), 8.25-8.30 (m, 1H), 8.06-8.17 (m, 2H), 7.87 (d, 2H), 7.69 (d, 2H), 7. 49 (d, 2H), 4.81 (s, 2H); ESI-MS: $(M+H)^+$ =347.

Example 63

1-(4-Chloroanilino)-4-(4-pyridylmethyl)phthalazine

A mixture of 14.19 g (0.1 mol) phosphorus pentoxide, 13.77 g (0.1 mol) triethylamine hydrochloride and 12.76 g (0.1 mol) 4-chloroaniline is heated and stirred in a nitrogen atmosphere at 200° C. until a homogeneous melt has formed (about 20 min). To the melt, 5.93 g (0.025 mol) 4-(4-pyridylmethyl)-1 (2H)-phthalazinone (for preparation see German Auslegeschrift no. 1 061 788 [published 23.07.1959]) is added, and the reaction mixture is stirred for 3 h at 200° C. After the reaction mixture has cooled to about 100° C., 200 ml of water is added. Stirring is continued until the temperature reaches about 30° C., and then 20 ml conc. ammonia (30% aqueous ammonium hydroxide solution) and 900 ml chloroform are added consecutively. As soon as a diphasic mixture has formed, the organic phase is separated off, dried over anhydrous sodium sulfate, filtered, and the filtrate evaporated on a RE to a volume of about 50 ml, to which 100 ml acetate is then added, and the mixture is cooled in an ice bath. The crystallizate obtained is filtered off and washed with acetate and ether. After recrystallization from methanol and drying under HV for 8 h at 120° C., the title compound is obtained; m.p. 194-195° C.; ESI-MS: $(M+H)^+$ =347.

Example 64

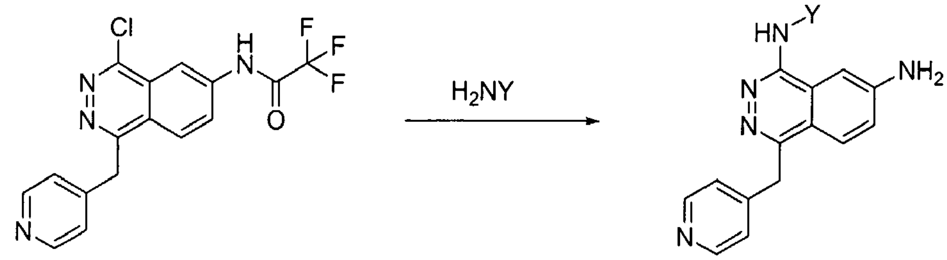


A: 7-Amino-1-(4-chloroanilino)-4-(4-pyridylmethyl)phthalazine hydrochloride

Under exclusion of air, 381 mg (0.77 mmol) 7-trifluoroacetamino-1- chloro-4-(4-pyridylmethyl)phthalazine is heated to 100° C for 5 h in 3.1 ml n-butanol with 295 mg (2.31 mmol) 4-chloroaniline. After cooling, the dark suspension is filtered off, washed with n-butanol and ethanol, and dried to obtain title compound: m.p.: >300° C.; HPLC: t Ret (Grad 5-40 )=12.9; FAB MS $(M+H)^+$ =362. With DIPE, further product may be precipitated out from the filtrate. The starting material is prepared as follows:

64A.1) 7-Trifluoroacetamino-4-(4-pyridylmethyl)-1(2H)-phthalazinone hydrochloride

A suspension of 500 mg (1.98 mmol) 7-amino-4-(4-pyridylmethyl)-1(2H)-phthalazinone [=1-oxo-4-[pyridyl-(4')-methylq-7-amino-1,2- dihydrophthalazine (for preparation see German Auslegeschrft no. 1061788 [published 23. 7.1959]) in 1.65 ml (11.88 mmol) trifluoroacetic acid anhydride is stirred over the weekend at RT. Addition of water and sonication yield a suspension which can be filtered and washed out with water. The crystals are suspended in 15 ml acetic acid. When 2.47 ml of a 2.4 M solution of HCl in dioxane is added, the suspension dissolves, and scraping eventually leads to renewed crystallization. Filtering and washing with ethyl acetate yield the title compound; HPLC: t Ret (Grad 5- 40 )=1 1.3; FAB MS $(M+H)^+$ =349.

64A.2) 7-Trifluoroacetamino-1-chloro-4-(4-pyridylmethyl)phthalazine

Under $N_2$ atmosphere, 552 mg (1.44 mmol) 7-trifluoroacetamino-4-(4-pyridylmethyl)-1 (2H)-phthalazinone hydrochloride is added to 4.2 ml acetonitrile 0.328 ml (3.58 mmol) phosphoroxychloride and heated for 4 h to 100° C. This is then cooled to 10° C., and 1.4 g $NaHCO_3$ in 7 ml water is added. After ethyl acetate is added, a reddish suspension forms which is filtered and washed out. Drying under HV yields the title compound: HPLC: t Ret (Grad 5-40 )=12.1; FAB MS $(M+H)^+$ =367.

The following compounds are prepared in the same manner:

| $H_2N$-Y | 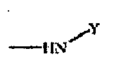 | HPLC: $t_{H52}$ | FAB MS $(M + H)^+$ |
|---|---|---|---|
| 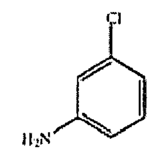 1 | 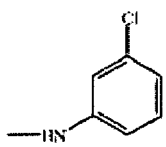 | 10.5 | 362 |
| 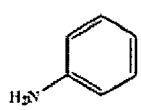 1,2 | 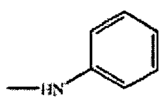 | 9.0 | 328 |
| 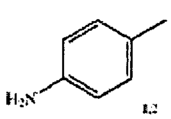 1,2 | 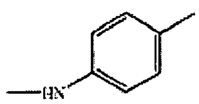 | 10.3 | 342 |
| 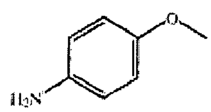 1 | 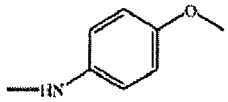 | 9.5 | 358 |

Example 65

5-(4-Chloroanilino)-8-(4-pyridylmethyl)pyrido[2.3-d]pyridazine

Under $N_2$ atmosphere, a mixture of 1.19 g (8.38 mmol) phosphorus pentoxide, 1.156 g (8.4 mmol) triethylamine hydrochloride, and 1.072 g (8. 4 mmol) 4-chloroaniline is heated for 5 min to 200° C. Then 0.50 g (2.1 mmol) 8-(4-pyridylmethyl)-.6H.-pyrido[2,3-.d.]pyridazin-5one is added to the melt, and this is stirred for 3 h at 200° C. After cooling, the melt is taken up in 25 ml dichloromethane, 10 ml water, and 5 ml sat. NH 3 solution, and the organic phase is separated off, dried (Na 2 SO 4 ), and concentrated by evaporation. Column chromatography ($SiO_2$; acetate/$CH_3OH$ 50:1→ 25:1) and crystallization from acetonitrile/methanol yields the title compound: m.p.: 220-222° C.; Anal. calc. C 65.61 %, H 4.06%, N 20.14%; found C 65.7%, H 4.1%, N 20.1%; FAB MS $(M+H)^+$ =348.

The starting material is prepared as follows:

65.1) 6-(Pyridin-4-yl)-1 pyridin-5,7-dione

To a suspension of 20.27 g (150 mmol) furo[3,4-b]pyridin-5(7h)-one (for preparation see Synthesis 1997,113) and 14.13 ml (150 mmol) 4-pyridinecarbaldehyde in 120 ml methanol and 75 ml ethyl propionate, 27.8 ml (150 mmol) of a 5.4M solution of sodium methylate in methanol is added dropwise under ice cooling (and $N_2$ atmosphere). The mixture is heated for 15 min to RT and then for 2 h to reflux temperature. The suspension temporarily goes into solution before a solid forms again. After cooling, 120 ml water is added, before stirring, filtering and washing the product with water. Further product is obtainable from the filtrate by acidification with acetic acid: FAB MS $(M+H)^+$ =225.

65.2) 8-(4-Pyridylmethyl)-.6H.-pyrido[2,3-.d.]pyridazin-5-one (A) and 5-(4-pyridylmethyl)-.7H.-pyrido[2,3-.d.] pyridazin-8-one (B)

A suspension of 8.7 g (38.8 mmol) 6-(pyridin-4-yl)-[11]pyridin-5,7- dione in 40 ml hydrazine hydrate is heated for 4 h to reflux. The suspension goes into solution temporarily, then once again a solid precipitates out, which is filtered off after cooling to RT, washed with water and ether, and dried. Fractionated crystallization from boiling methanol leads to mixtures of A and B. Column chromatography ($SiO_2$ ; ethyl acetate/$CH_3OH$ 19:1-7:3) and stirring in boiling methanol yields A followed by B. A: m.p.: 246-248° C.; $^1$H-NMR (DMSO-d 6 ) 12. 83 (s, HN), 9.13 (dd, 1H), 8.59 (dd, 1H), 8.43 (d, 2H), 7.85 (dd, 1H), 7. 29 (d, 2H), 4.38 (s, 2H; NOE on signal at 7.29 $^{pyridine}$ ); Anal. calc. ($C_{13}H_{10}N_4O$) C 65.54%, H 4.23%, N 23.52%; found C 65.2%, H 4.3%, N 23.5. B: m.p.: >260° C.; $^1$H-NMR (DMSO-d 6 ) 12.83 (s, HN), 9.04 (dd, 1H), 8.46 (d, 2H), 8.33 (dd, 1H), 7.86 (dd, 1H), 7.30 (d, 2H), 4.34 (s, 2H; NOE on signal at 7.29 $^{pyridine}$ and 8.33 $^{HC-4}$); Anal. calc. ($C_{13}H_{10}N_4O$) C 65.54%, H 4.23%, N 23. 52%; found C 65.2%, H 4.3%, N 23.5.

Example 66:

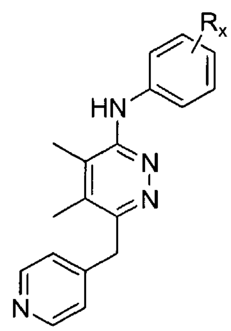

The following compounds are prepared as described below:

A: Rx = 4-Cl

B: Rx = 4-$CH_3$

C: Rx = 4-$OCH_3$

D: Rx = 3-Cl

E: Rx = 3-$CH_3$

Preparation of 82A=3-(4-chloroanilino)-4,5-dimethyl-6-(pyridin-4-yl)methylpyridazine (Rx = para-chloro):

A solution of 0.070 g 3-chloro-4,5-dimethyl-6-(pyridin-4-yl)methylpyridazine and 0.153 g para-choroaniline is heated in a sealed tube for 20h to 130° C. After cooling to RT, the solution is concentrated by evaporation, the residue diluted with 100 ml $CH_2Cl_2$ and then extracted with 100 ml sat. aqueous $NaHCO_3$ solution. The organic phase is dried over $MgSO_4$ , concentrated by evaporation, and the residue purified by flash chromatography (FC) on silica gel in $CH_2Cl_2$

/methanol 19/1. The title compound is obtained: m.p. 196-199° C. $^1$H-NMR (250 MHz, $CDCl_3$ ): δ=8.45 (s, wide, 2H); 7.55 (d, 2H); 7. 25 (d, 2H); 7.10 (d, 2H); 6.20 (s, wide, 1H); 4.25 (s, 2H); 2.15 (s, 3H); 2.10 (s, 3H). ES-MS 325, 327 (M+H for $^{35}$ Cl and $^{31}$ Cl).

Example 67

A: 1-(3-Phenoxyanilino)-4-(4-pyridylmethyl)phthalazine

A mixture of 256 mg (1.00 mmol) 1-chloro-4-(4- pyridylmethyl)phthalazine and 556 mg (3.00 mmol) 4-phenoxyaniline (Aldrich) is heated for 2 h at 90° C. The melt is cooled and stirred with 6 ml $NH_3$ solution (10% in water: or 10 ml sat. $NaHCO_3$ solution) and 15 ml dichloromethane/methanol 50:1 for 30 min. The aqueous phase is then separated off and extracted again with dichloromethane. The organic phase is dried ($Na_2SO_4$), concentrated by evaporation, and chromatographed ($SiO_2$ ; ethyl acetate→ ethyl acetate/$CH_3OH$ 19:1→ 10:1). Crystallization from acetonitrile yields the title compound: m.p.: 186-1 89° C.; Anal. calc. ($C_{26}H_{21}N_4O$) C 77.02%, H 5.22%, N 13.82%; found C 77.2%, H 4.9%, N 13.8%.

The starting material is prepared as follows:

67.A1) 1-Chloro-4-(4-pyridylmethyl)phthalazine

Under exclusion of air, 29 g (122 mmol) 4-(4-pyridylmethyl)-1 (2H)-phthalazinone [for preparation, see German Auslegeschrift no. 1061788 (published 23.7.1959)] in 450 ml acetonitrile is mixed with 61 ml HCl/dioxane 4N and 28 ml (306 mmol) phosphoryl chloride and stirred for 27 h at 50° C. To the white suspension, 119 g $NaHCO_3$ in 1.45 l water is then added dropwise under ice cooling, and the mixture is stirred and the title compound filtered off. Anal. calc. ($C_{14}H_{10}N_3Cl$) C 65.76%, H 3. 94%, N 16.43%, Cl 13.86%; found C 65.40%, H 4.12%, N 16.45%, Cl 13.66%; FAB MS $(M+H)^+$ =256.

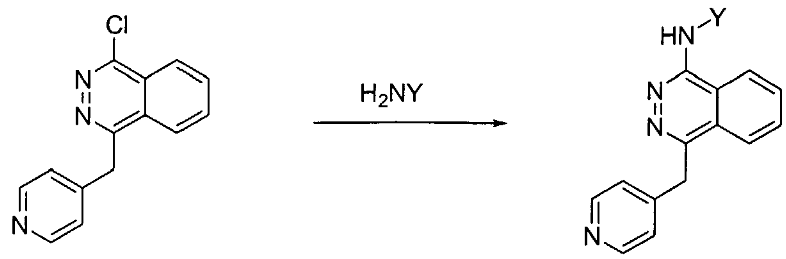


In the same manner, the following compounds are prepared by reaction in the melt:

| Example | $H_2N$-Y | 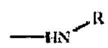 | m.p.[° C] | Anal.* | FAH-MS (M + H)$^+$ |
|---|---|---|---|---|---|
| 67B | 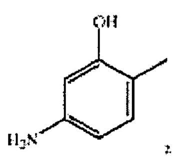 | 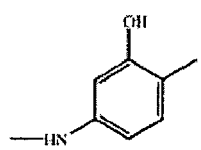 | 192-195 | CHN (1.5 $H_2O$) | λ<3 |
| 67C | 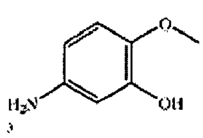 | 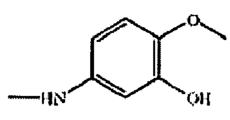 | 256-258 | CHN (0.23 $H_2O$) | 359 |
| 67D | 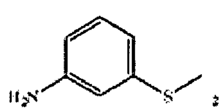 | 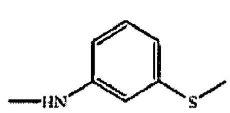 | 148-149 | CHN | 359 |

(continued)

| Example | $H_2N$-Y | 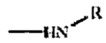 | m.p.[° C] | Anal.* | FAH-MS (M + H)$^+$ |
|---|---|---|---|---|---|
| 67E | 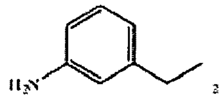 | 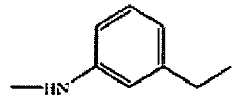 | 143-144 | CHN | 341 |
| 67F | 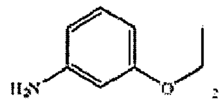 | 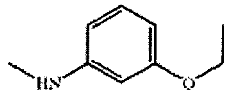 | 193-195 | CHN | 357 |
| 67G | 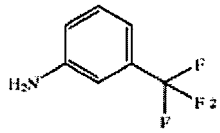 | 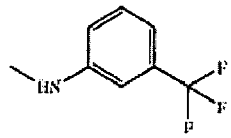 | 184-185 | CHN | 381 |
| 67H | 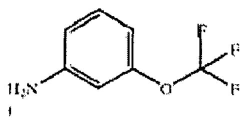 | 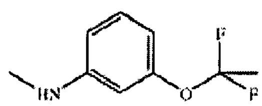 | 176-178 | CHN | 397 |
| 67I | 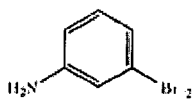 | 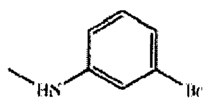 | | | 391/393 |
| 67J | 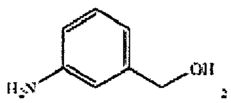 | 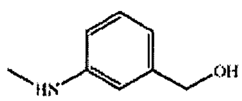 | 192-193 | CHN | 343 |
| 67K | 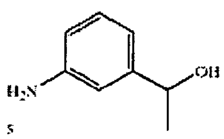 | 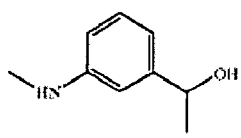 | 221-222 | CHN | 357 |
| 67L | 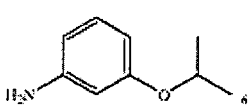 | 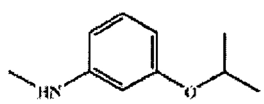 | 188-193 | CHN | 371 |
| 67M | 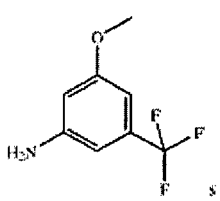 | 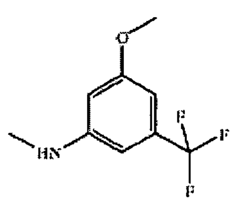 | 143-145 | CHN | 411 |

(continued)

| Example | $H_2N$-Y | 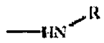 | m.p.[° C] | Anal.* | FAH-MS (M + H)$^+$ |
|---|---|---|---|---|---|
| 67N | 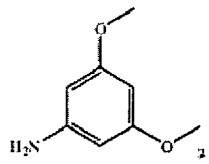 | 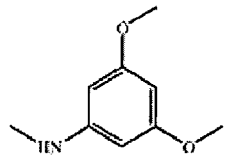 | 193-194 | CHN | 373 |
| 67O | 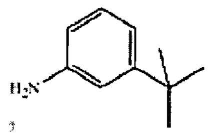 | 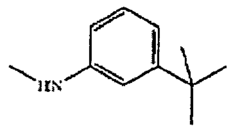 | | CHN (0.5 $H_2O$) | 369 |
| 67P | 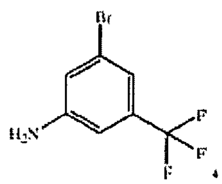 | 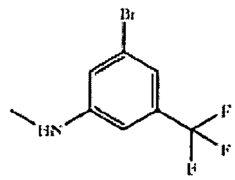 | 223-226 | CHN | 459/461 |
| 67O | 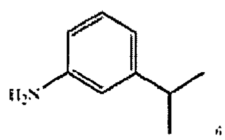 | 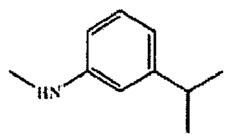 | | CHN (0.5 $H_2O$) | 355 |
| 67R | 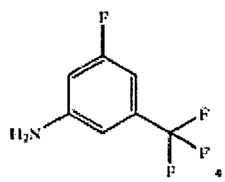 | 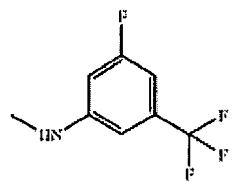 | 253-255 | CHN | 399 |
| 67S | 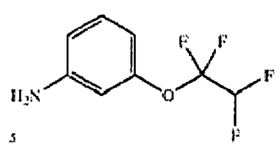 | 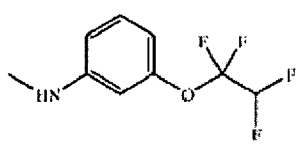 | 185-187 | CHN F (0.3 $H_2O$) | 429 |

(continued)

| Example | $H_2N$-Y | 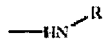 | m.p.[° C] | Anal.* | FAH-MS (M + H)$^+$ |
|---|---|---|---|---|---|
| 67T | 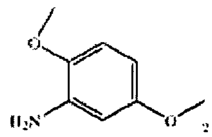 | 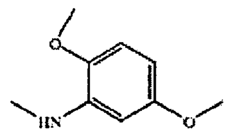 | 199-201 | CHN | 373 |

[1]Deviation ≥ 0.4%:
manucfaturer:
[2]Flokn;
[3]Laqcasier;
[4]IRD Fluotchemicals.
[5]Akhich:
[6]TCl;
[7]Maybaidge.

To 262 mg (1.05 mmol) decyloxyaniline (Salor) in 5 ml ethanol, 0.26 ml HCl/dioxane 4N is added, the mixture stirred for ≈3 min, and then 256 mg (1.00 mmol) 1-chloro-4-(4-pyridylmethyl)phthalazine (Example 67A.1) is added. After 2 h boiling under reflux, the mixture is cooled and concentrated by evaporation. The residue is stirred with 6 ml $NH_3$ solution (10% in water: or 10 ml sat. $NaHCO_3$ solution) and 15 ml dichloromethane/methanol SO: 1 for 30 min. The aqueous phase is then separated off and extracted again with dichloromethane. The organic phase is dried ($Na_2SO_4$) and concentrated by evaporation. Crystallization [possibly after chromatography on $SiO_2$ (ethyl acetate/$CH_3OH$ 19:1)] from acetonitrile (or methanol) yields the title compound: m.p.: 116-119° C.; Anal. calc. ($C_{30}H_{36}N_4O$) C 76.89%, H 7.74%, N 11.96%; found C 76. 7%, H 7.7%, N 11.9%; FAB MS (M+H) $^+$ = 469.

By the same manner, the following compounds are prepared in ethanol while heating:

| Example | $H_2N$-Y | 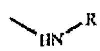 | m.p. [° C] Anal.[1] | | FAB MS (M + H)$^+$ |
|---|---|---|---|---|---|
| 68B | 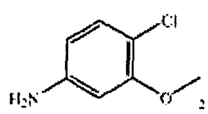 | 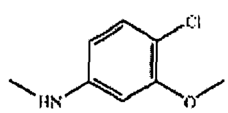 | 242-243 | CHN | 377 |
| 68C | 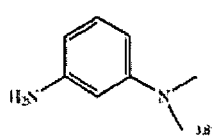 | 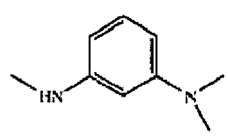 | 143-145 | CHN | 356 |
| 68D | 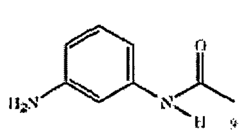 | 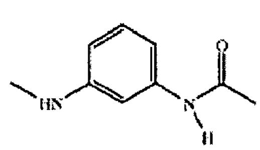 | 263-265 | CHN (0.22 $H_2O$) | 370 |
| 68E | 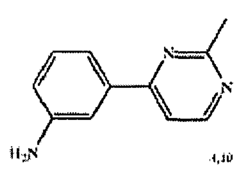 | 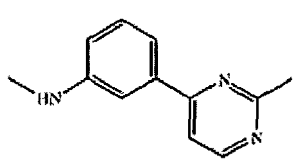 | 314-316 | CHN (0.13 $H_2O$) | 405 |

(continued)

| Example | $H_2N$-Y | 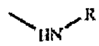 | m.p. [° C] | Anal.[1] | FABMS (M + H)$^+$ |
|---|---|---|---|---|---|
| 68F | 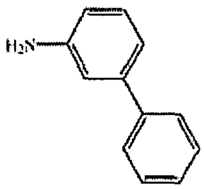 4,11 | 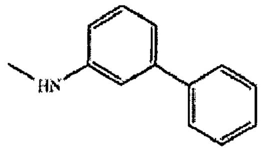 | | CNH (0.4 $CH_3CN$) | 389 |
| 68G | 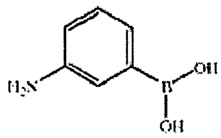 4,5,12,16 | 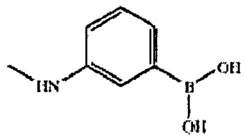 | | CHN (0.5 $H_2O$) | 357 |
| 68H | 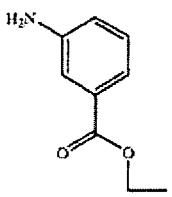 4,6,12 | 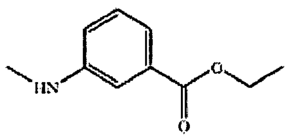 | 153-155 | CHN | 385 |
| 68I | 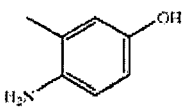 4,6,8 | 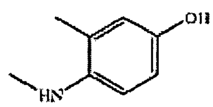 | | | 343 |
| 68J | 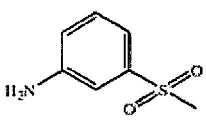 5,13 | 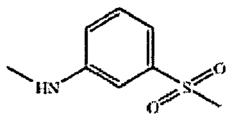 | 239-241 | CHN (0.2 $H_2O$) | 391 |
| 68K | 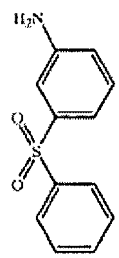 11 | 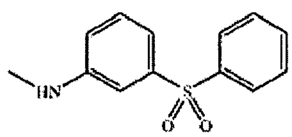 | 196-199 | CHNS (0.26 $H_2O$) | 453 |
| 68L | 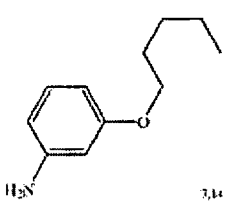 7,14 | 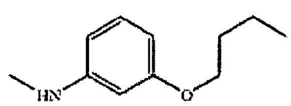 | | CHNCl(1.6 $H_2O$) | 399 |

(continued)

| Example | $H_2N$-Y | 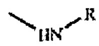 | m.p. [° C] Anal.[1] | | FAB MS (M + H)$^+$ |
|---|---|---|---|---|---|
| 68M | 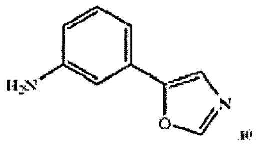 | 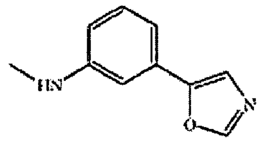 | 194-196 | CHN (0.2 $H_2O$) | 380 |
| 68N | 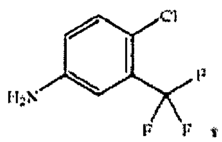 | 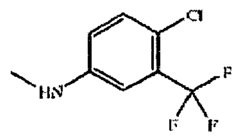 | 220-222 | CHNF | 415 |
| 68O | 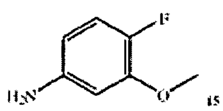 | 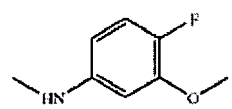 | 190-192 | CHNF | 361 |
| 68P | 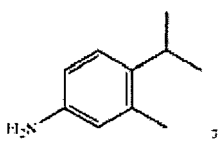 | 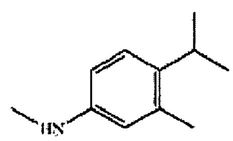 | 163-166 | CHN | 359 |

A: 1-(3-Chlorophenoxy)-4-(4-pyridylmethyl)phthalazine

Under exclusion of air, 200 mg (0.78 mmol) 1-chloro-4-(4-pyridylmethyl)phthalazine (Example 67A.1), 173 mg (1.25 mmol) $K_2CO_3$ , and 120 mg (0.94 mmol) 3-chlorophenol (Fluka) are heated in 2 ml DMSO for 3 h to 90° C. The reaction mixture is distributed between 20 ml water and 20 ml ethyl acetate, and the aqueous phase separated and extracted with 2 portions of ethyl acetate. The organic phase is washed with water and brine, dried ($MgSO_4$), and concentrated by evaporation. The residue is dissolved in -15 ml THF, precipitated with hexane, and filtered. Title compound is obtained from the evaporated filtrate after chromatography ($SiO_2$ ; ethyl acetate/$CH_3OH$ 4:1): m.p.: 143-145° C.; HPLC: t Ret (Grad 20-100 )=8.9; FAB MS (M+H) $^+$ =348.

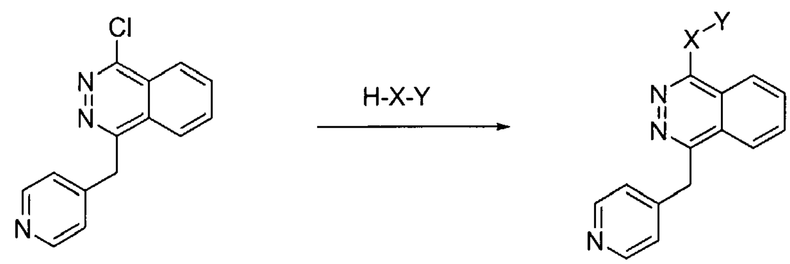


The following compounds are prepared in the same manner:

| Example | H-X-Y | 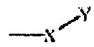 | m.p. [˚C.] | HPLC $I_{201}$ (Grad$_{33\text{-}Mok}$) | FAB MS $(M + H)^+$ |
|---|---|---|---|---|---|
| 72B | 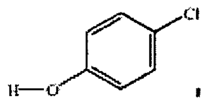 , | 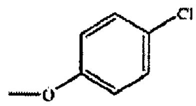 | 207-208 | 8.9 | 348 |
| 72C | 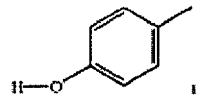 , | 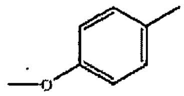 | 175-176 | 8.6 | 328 |
| 72D | 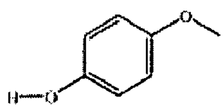 , | 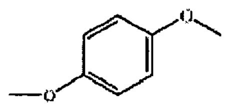 | 194-196 | 8.1 | 344 |
| 72E | 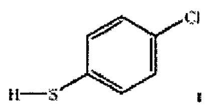 , | 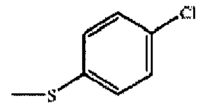 | 204-206 | 9.5 | 364 |

Mnmifromtion
[1]Floko

**Preparation of Staurosporine Analogs**

As will become apparent to a skilled artisan, many of the bridged epoxy diindolopyrrolo-hexahydrobenzodiazocines are commercially available as final compounds or modifiable intermediates. Staurosporine was originally isolated from the bacterium *Streptomyces staurosporeus.* (S.Omura *et al.* J.Antibiotics, **30**, 275 1977).

Synthesis of 9,12-epoxy staurosporine analogs:

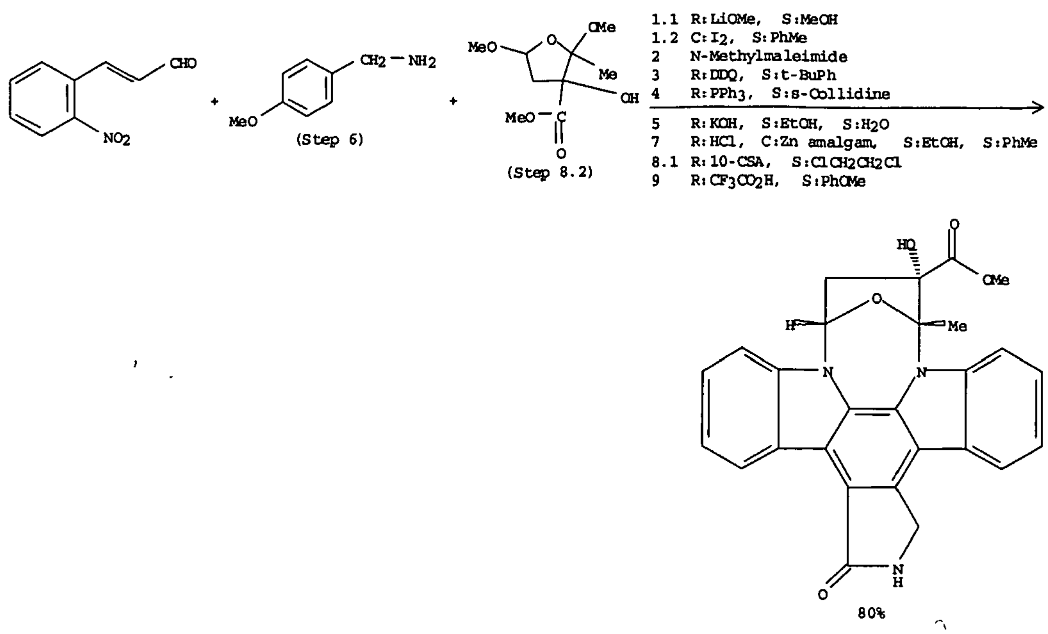


NOTE: 2) 120°, 6) 120°,
Reactants: 4, Reagents: 7, Catalysts: 2, Solvents: 8,
Steps: 9, Stages: 11, Most stages in any one step: 2

Greater detail is provided in Tetrahedron Letters, 36(46), 8383-6, 1995.

Alternative synthesis of 9,12-epoxy staurosporine analogs:

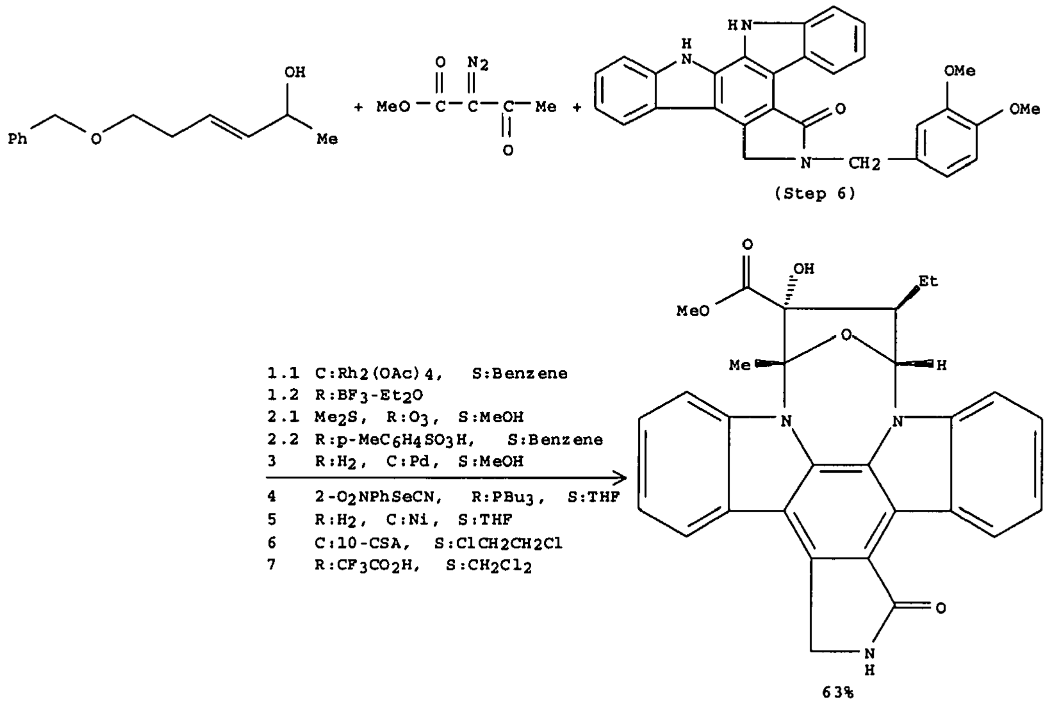


NOTE: 1) stereoselective, 5) Raney nickel present,
Reactants: 5, Reagents: 6, Catalysts: 4, Solvents: 5,
Steps: 7, Stages: 9, Most stages in any one step: 2

Greater detail is provided in Organic Letters, 3(11), 1689-1692; 2001.

Synthesis of 9,13-epoxy staurosporine analogs:

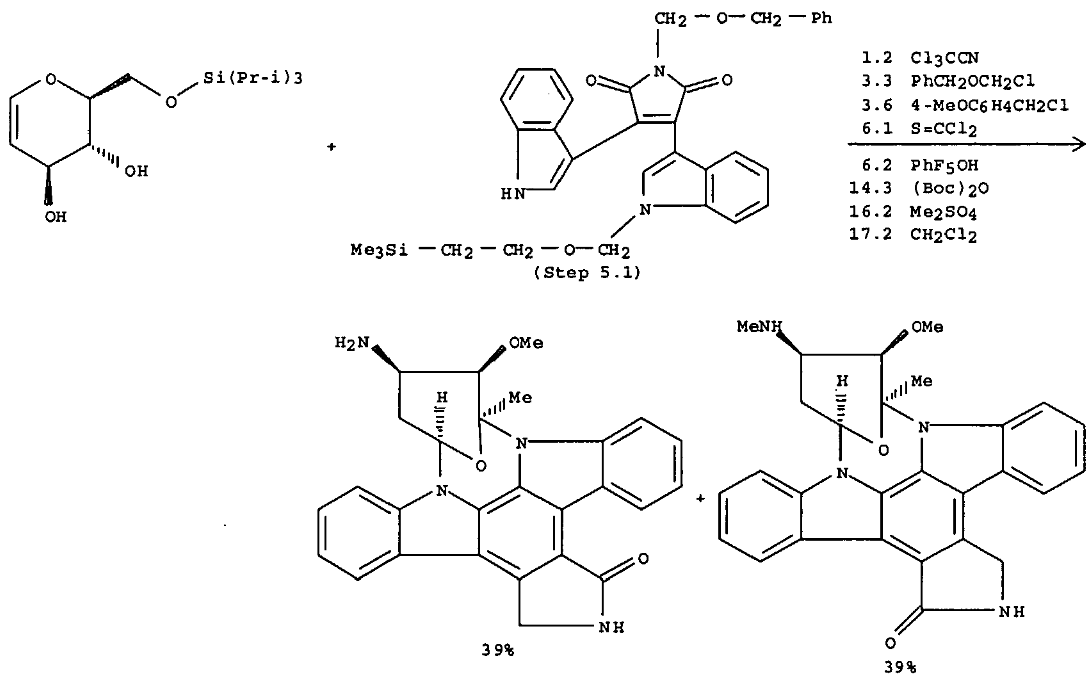


NOTE: 1) STEREOSELECTIVE, 3) (92%/65%/95%/92%), 4) 100% OVERALL (5.5:1, ALPHA:BETA), 5) STEREOSELECTIVE,KEY STEP, 8) (97%/91%), 9) PHOTOCHEM., 12) STEREOSELECTIVE KEY STEP, 14) (92%/81%/82%),
Reactants: 10, Reagents: 20, Catalysts: 5, Solvents: 9,
Steps: 17, Stages: 35, Most stages in any one step: 6

Whereas, a more thorough description of reagents, reaction conditions, and other pertinent syntheses are described Journal of the American Chemical Society, 117(1), 552-3; 1995. Additionally, syntheses on staurosporine and analogs thereof are described by S.J Danishefsky et al., J.Am.Chem.Soc., 118, 2825 1996 and J.L.Wood et al., J.Am.Chem.Soc., 118, 10656 1996.

Table 1: Staurosporine Analogs

| Example | Structure | Patent |
|---|---|---|
| 1 | 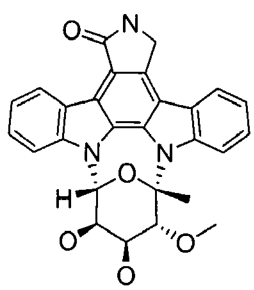 | WO 02/30941 WO 97/07081 |
| 2 | 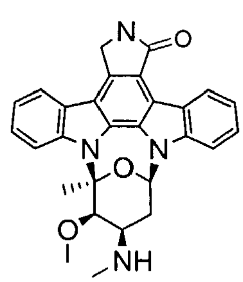 | WO 89/07105 |

(continued)

| Example | Structure | Patent |
|---|---|---|
| 3 | 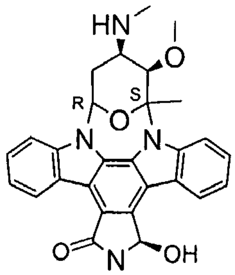 | WO 89/07105 |
| 4 | 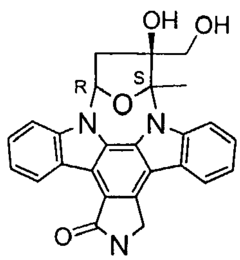 | US 5,621,100 |
| 5 | 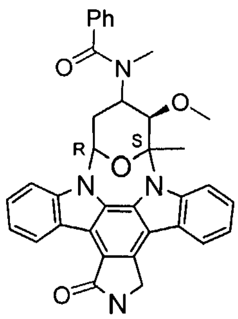 | WO 89/07105 |
| 6 | 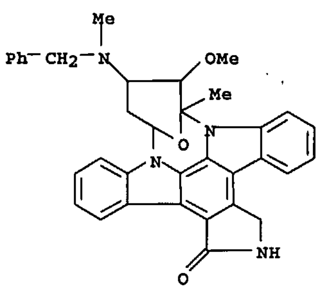 | WO 93/07153 |
| 7 | 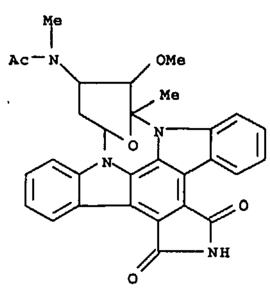 | WO 89/07105 |

(continued)

| Example | Structure | Patent |
|---|---|---|
| 8 | 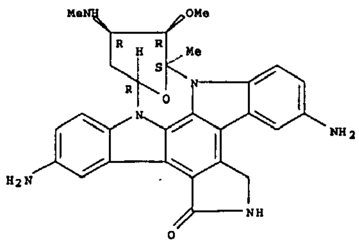 | WO 01/04125 |
| 9 | 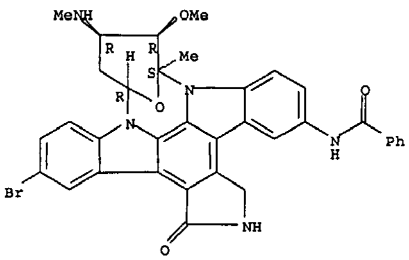 | WO 01/04125 |
| 10 | 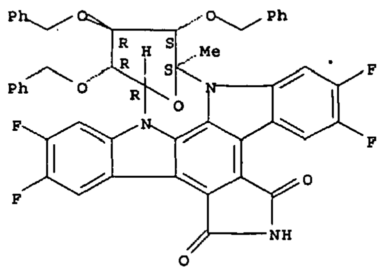 | WO 02/30941 |
| 11 | 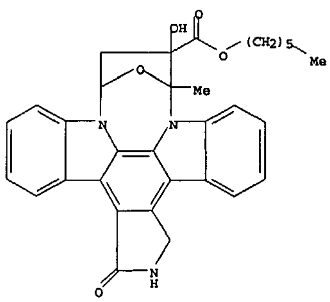 | WO 93/08809 WO 94/06799 WO 00/27422 |
| 12 | 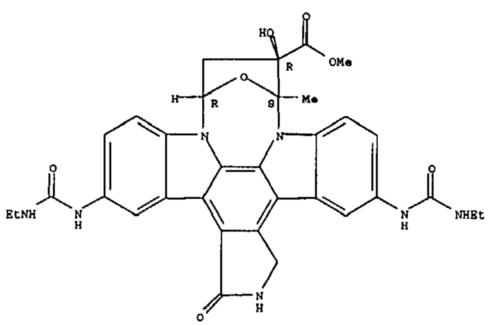 | WO 96/13506 |

(continued)

| Example | Structure | Patent |
|---|---|---|
| 13 | 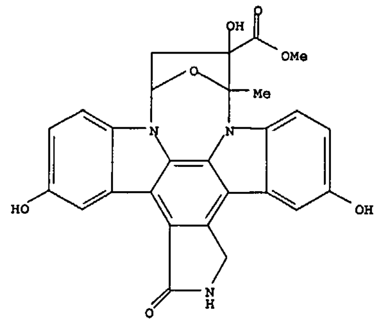 | WO 88/07045 |

**Preparation of Nucleoside Analogs**

Scheme 1a:

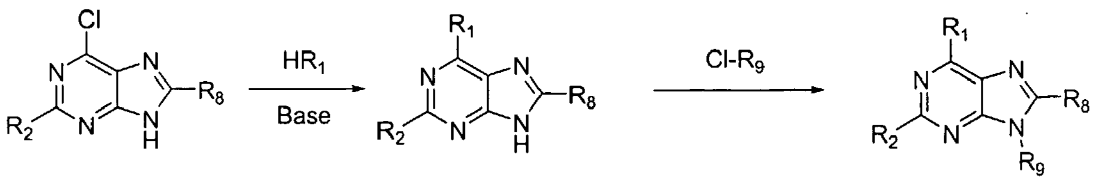


In addition to Cl other activating groups such as Br, I, triflate, or tosylate, and the like may be used in the above reaction scheme. It is preferred that $HR_1$ is a nucleophile, such as ammonia and the base is triethylamine or NaH.

Scheme 1b:

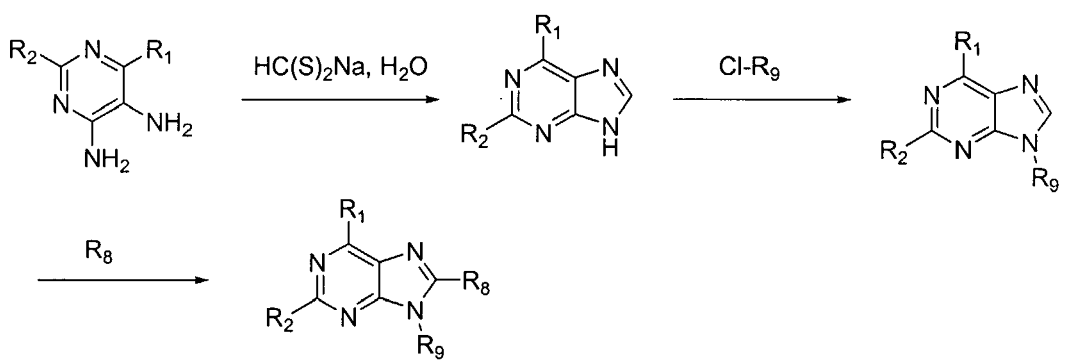


In addition to Cl other activating groups such as Br, I, triflate, or tosylate, and the like may be used in the above reaction scheme.

Scheme 2:

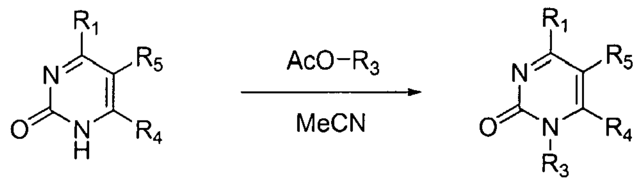


If $R_1$, $R_4$, or $R_5$ is nucleophilic (as in $-NH_2$) it may need to be protected (such as with a Bz group), which can subsequently be removed after addition of $R_3$.

Scheme 3:

Synthesis of analogs of 5'-modified derivatives of bredinin, the 5'-phosphate **2**, the 5'-deoxy derivative **3**, and the 5'-*O*-(3-aminopropyl)carbamate **4** (Fig. 1) were attempted.

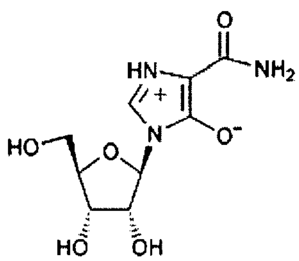

bredinin (1)

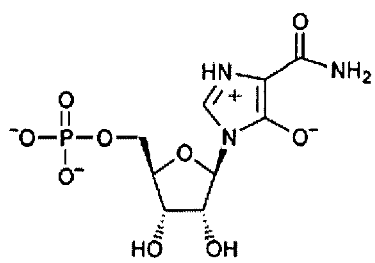

2

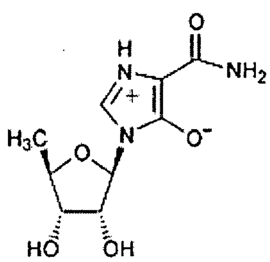

3

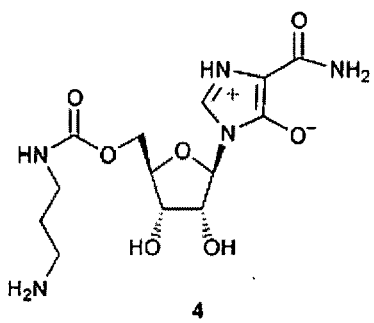

4

**Fig. 1**

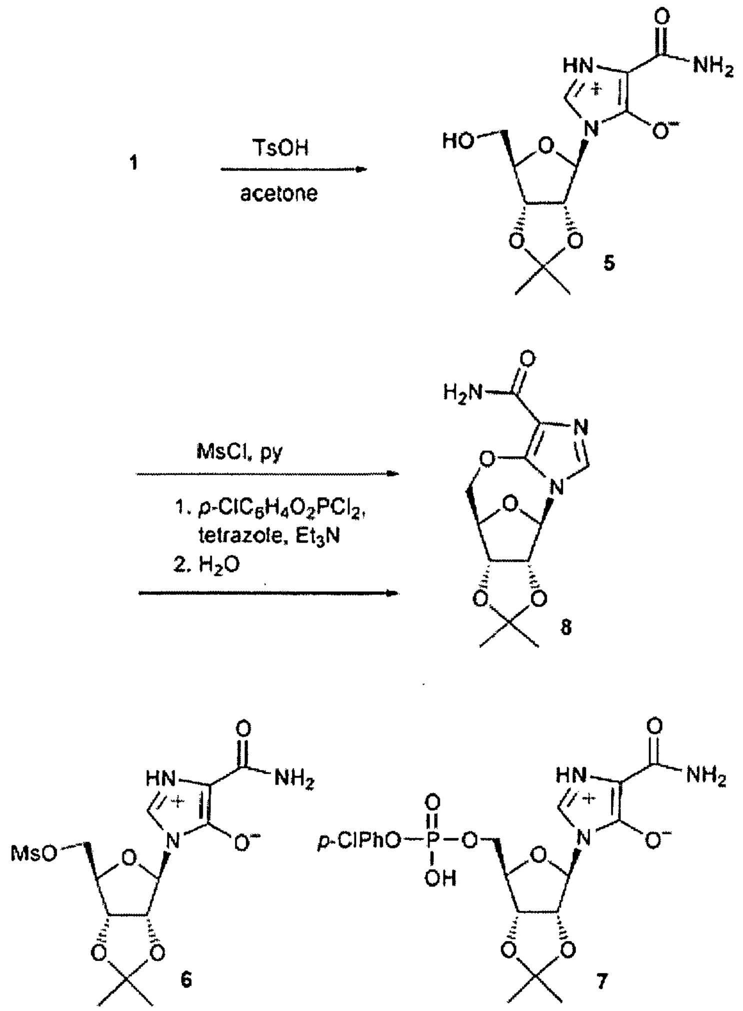


Scheme 3

A phosphoramidite method with o-xylylene *N,N*-diethylphosphoramidite (XEPA) 13 is effective in this system (Scheme 4). Treatment of **17** with XEPA and tetrazole in $CH_2Cl_2$, followed by oxidation with aq. $I_2$, gives the corresponding 5'-phosphotriester **19** in 70% yield. The isopropylidene and Boc groups of **19** are removed simultaneously with 90% aq. TFA, and the resulting product, without purification, is heated with $(EtO)_3CH$ in DMF at 90 °C to give the bredinin 5'-phosphate derivative **20** in 47% yield from **19.** Hydrogenation of **20** with Pd-carbon in MeOH furnished bredinin 5'-phosphate **2**, which is isolated as a disodium salt in 89% yield, after successive treatment with Dowex 50 ($H^+$) and Diaion WK-20 ($Na^+$) resins.

Scheme 4

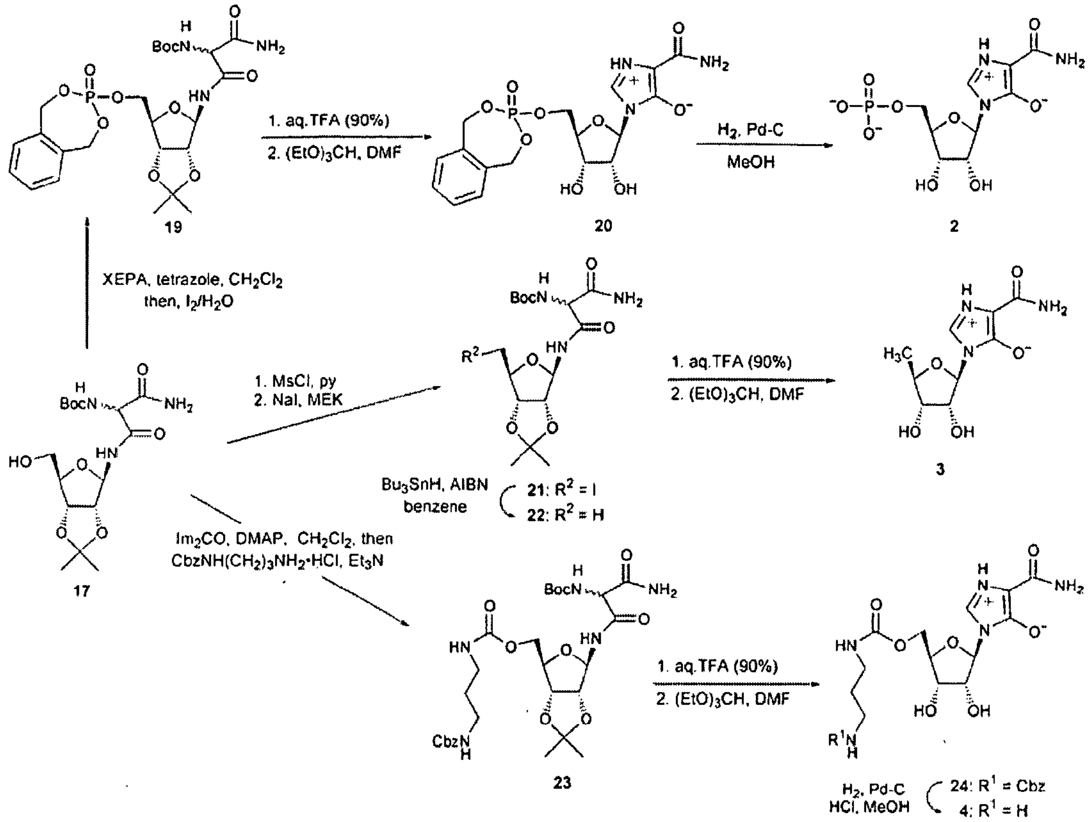


**Table 1:** Nucleoside Analogs

| Example | Structure | Name | Reference |
|---|---|---|---|
| 1 | 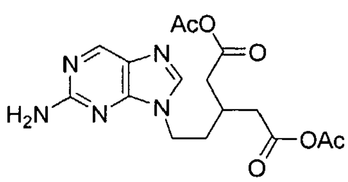 | Famciclovir | US 5,684,153 WO 95/28402 |
| 2 | 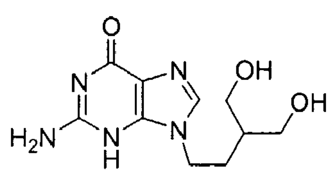 | Penciclovir | US 5,684,153 WO 95/28402 |
| 3 | 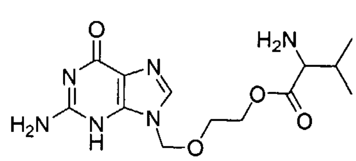 | Valacyclovir | WO 98/03553 WO 03/41647 |

(continued)

| Example | Structure | Name | Reference |
|---|---|---|---|
| 4 | 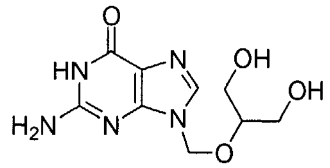 | Ganciclovir | US 4,355,032 |
| 5 | 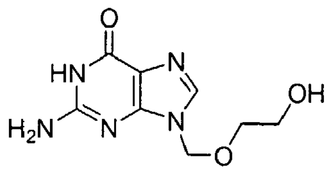 | Acyclovir | US 4,199,574 |
| 6 | 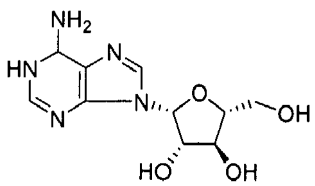 | Vidarabine | US 3,948,883 |
| 7 | 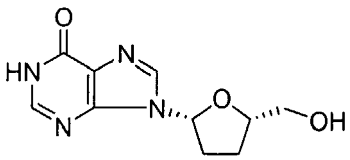 | Didanosine | US 4,970,148 |
| 8 | 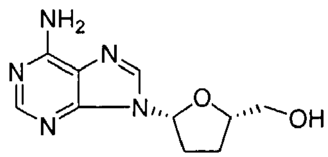 | Dideoxyadenosine | US 4,970,148 |
| 9 | 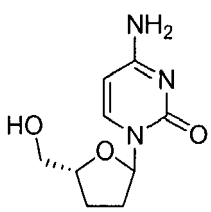 | Dideoxycytidine | US 4,970,148 |
| 10 | 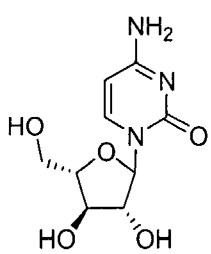 | Cytarabine | US 3,595,853 |

(continued)

| Example | Structure | Name | Reference |
|---|---|---|---|
| 11 | 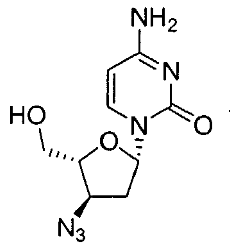 | Zidovudine | US 4,730,001 |
| 12 | 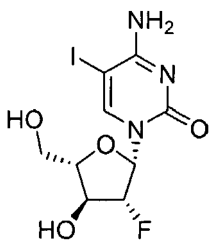 | Fiacitabine | US 4,594,339 US 5,753,789 |
| 13 | 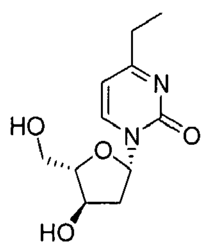 | Edoxudine | WO 03/53360 |
| 14 | 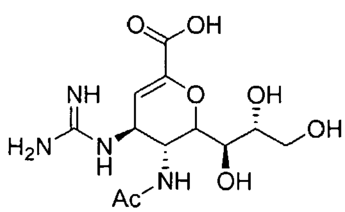 | Zanamivir | WO 91/16320 WO 94/07885 |
| | | | |
| **Example** | **Chiron ID** | **Structure** | **MH+** |
| 15 | 033288 | 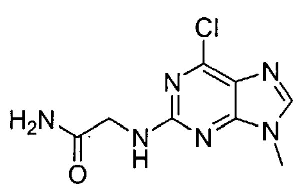 | 241.7 |
| 16 | 085361 | 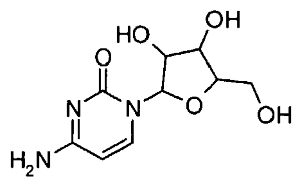 | 244.2 |

(continued)

| Example | Chiron ID | Structure | MH+ |
|---|---|---|---|
| 17 | 102410 | 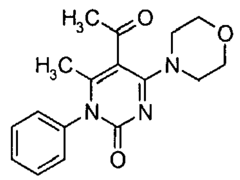 | 314.4 |
| 18 | 110529 | 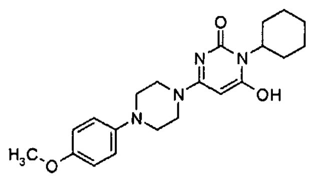 | 385.5 |
| 19 | 110681 | 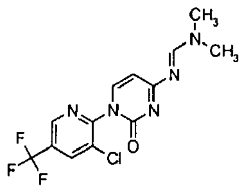 | 346.7 |
| 20 | 120907 | 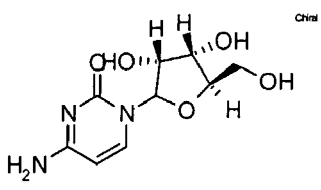 | 244.2 |
| 21 | 002432 | 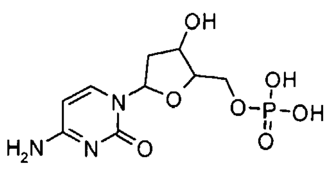 | 308.2 |
| 22 | 002466 | 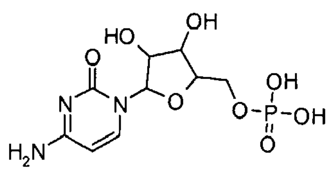 | 324.2 |
| 23 | 003266 | 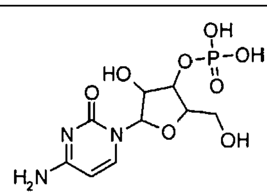 | 324.2 |
| 24 | 003268 | 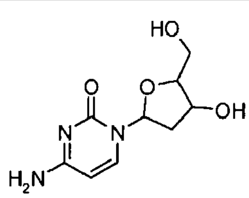 | 228.2 |

(continued)

| Example | Chiron ID | Structure | MH+ |
|---|---|---|---|
| 25 | 145854 | 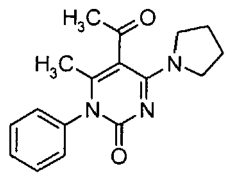 | 298.4 |
| 27 | 176431 | 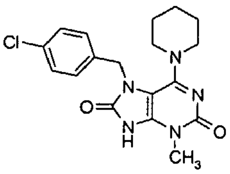 | 374.8 |
| 28 | 231323 | 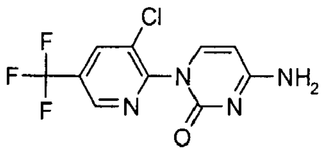 | 291.6 |
| 29 | 231324 | 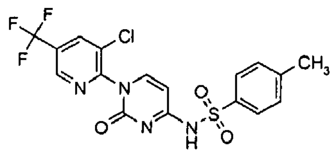 | 445.8 |
| 30 | 232148 | 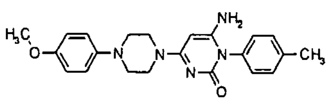 | 392.5 |
| 31 | 166141 | 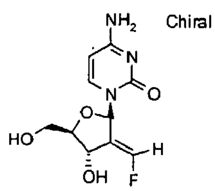 | 258.2 |
| 32 | 169218 | 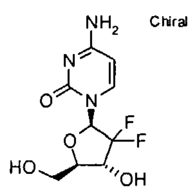 | 264.2 |
| 38 | 259624 | 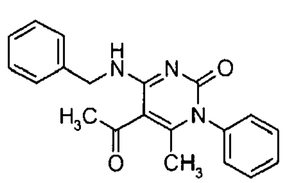 | 334.4 |

(continued)

| Example | Chiron ID | Structure | MH+ |
|---|---|---|---|
| 42 | 276564 | 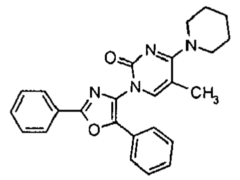 | 413.5 |
| 43 | 288440 | 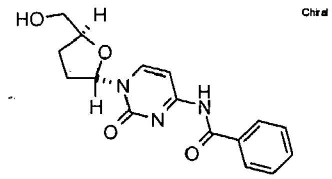 | 316.3 |
| 44 | 288441 | 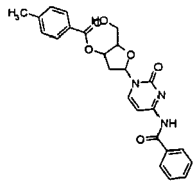 | 450.5 |
| 45 | 308679 | 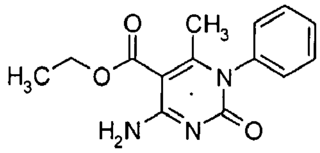 | 274.3 |
| 46 | 308702 | 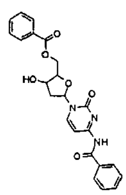 | 436.4 |
| 47 | 325289 | 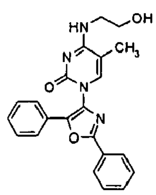 | 389.4 |
| 48 | 326212 | 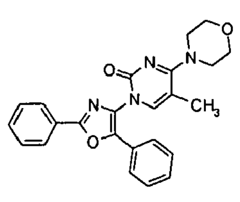 | 415.5 |
| 49 | 376367 | 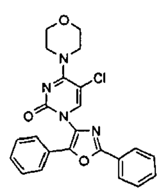 | 435.9 |

(continued)

| Example | Chiron ID | Structure | MH+ |
|---|---|---|---|
| 50 | 376632 | 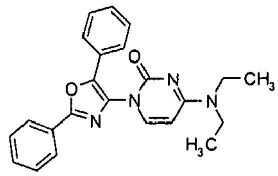 | 387.5 |
| 51 | 382680 | 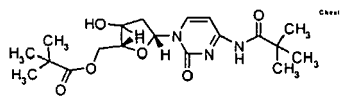 | 396.5 |
| Example | Chiron ID | Structure | MH+ |
| 52 | 085696 | 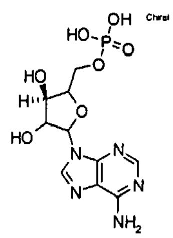 | 348.2 |
| 53 | 085743 | 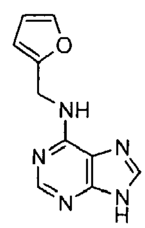 | 216.2 |
| 54 | 085773 | 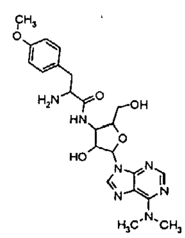 | 472.5 |
| 55 | 098445 | 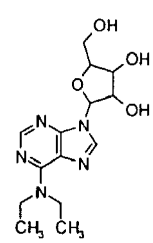 | 324.4 |

(continued)

| Example | Chiron ID | Structure | MH+ |
|---|---|---|---|
| 56 | 100013 | 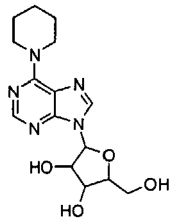 | 336.4 |
| 57 | 146680 | 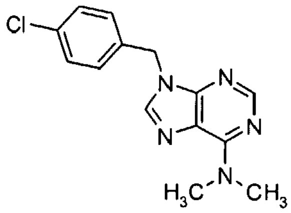 | 288.8 |
| 58 | 151489 | 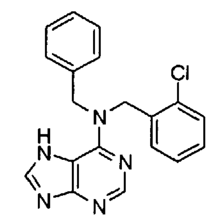 | 350.8 |
| 59 | 239459 | 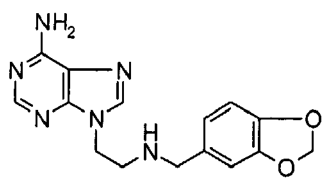 | 313.3 |
| 60 | 239462 | 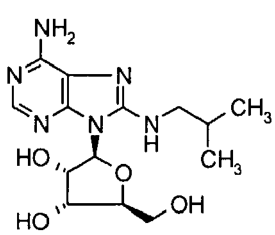 | 339.4 |
| 61 | 239491 | 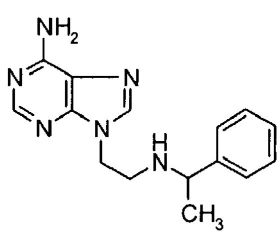 | 283.3 |

(continued)

| Example | Chiron ID | Structure | MH+ |
|---|---|---|---|
| 62 | 247177 | 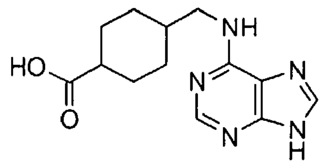 | 276.3 |
| 63 | 247445 | 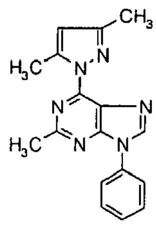 | 305.4 |
| 64 | 249027 | 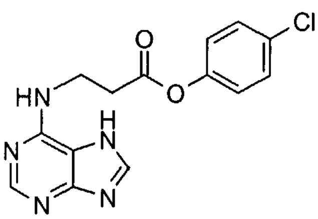 | 318.7 |
| 65 | 249028 | 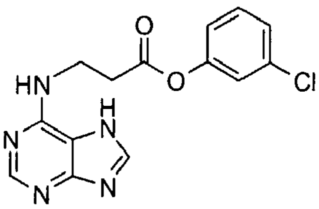 | 318.7 |
| 66 | 251666 | 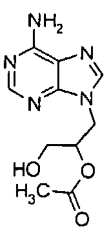 | 252.2 |
| 67 | 261664 | 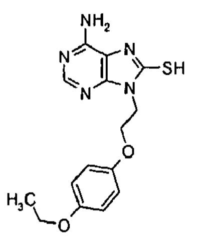 | 332.4 |

(continued)

| Example | Chiron ID | Structure | MH+ |
|---|---|---|---|
| 68 | 261688 | 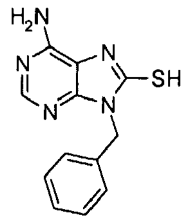 | 258.3 |
| 69 | 301772 | 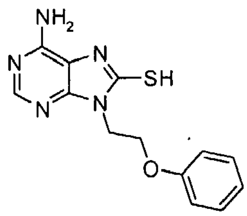 | 288.3 |
| 70 | 301812 | 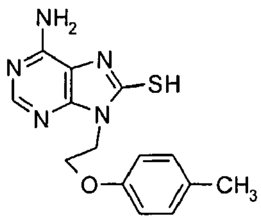 | 302.4 |
| 71 | 301814 | 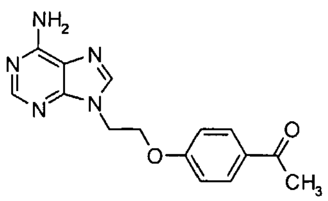 | 298.3 |
| 72 | 324616 | 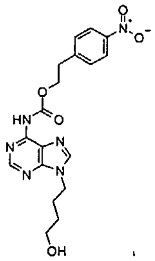 | 401.4 |
| 73 | 326056 | 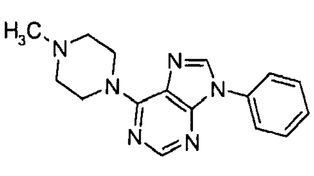 | 295.4 |

(continued)

| Example | Chiron ID | Structure | MH+ |
|---|---|---|---|
| 74 | 328065 | 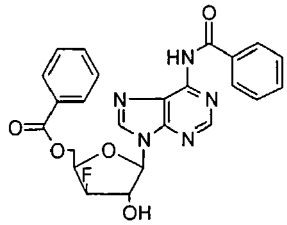 | 478.4 |
| 75 | 360367 | 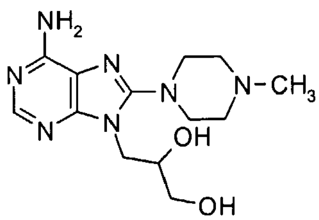 | 308.4 |
| 76 | 369765 | 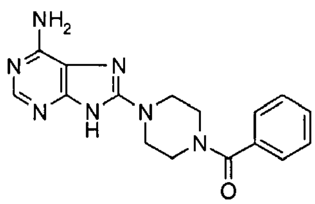 | 324.4 |
| 77 | 374495 | 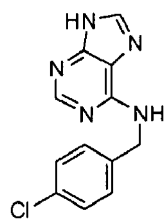 | 260.7 |
| 78 | 375497 | 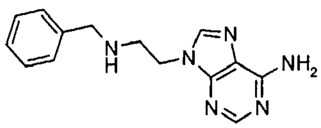 | 269.3 |
| 79 | 377272 | 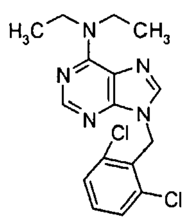 | 351.3 |

(continued)

| Example | Chiron ID | Structure | MH+ |
|---|---|---|---|
| 80 | 378008 | 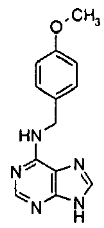 | 256.3 |
| 81 | 378010 | 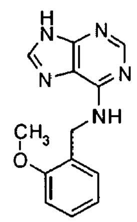 | 256.3 |
| 82 | 378011 | 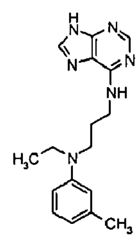 | 311.4 |
| 83 | 379980 | 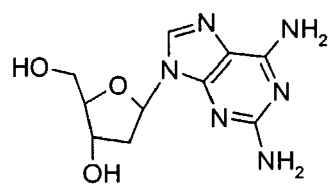 | 267.3 |
| 84 | 382512 | 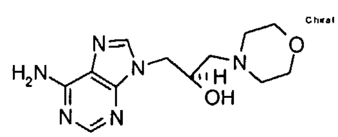 | 279.3 |
| 85 | 382621 | 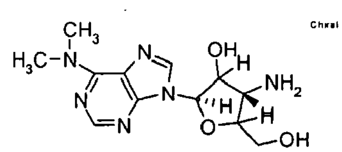 | 295.3 |
| 86 | 382681 | 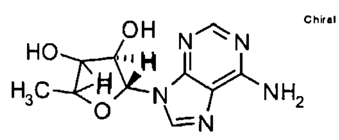 | 252.2 |

**Nucleoside analog example compounds** were screened in the assay described below for their ability to induce cytokines. Compounds of Formula I in the table showed activity with respect to production of TNF-α. Subsequently, each of these compounds is individually preferred and is preferred as a member of a group that includes any or all of the other compounds and each compound is preferred in methods of modulating an immune response and in methods of treating biological conditions associated therewith, for example to be used as an antiviral. Each of the compounds is also preferred for use in preparation of medicaments for immunopotentiation, treating microbial and viral infections, particularly HCV, HIV, and HSV, and in treating biological conditions mediated therefrom.

Some of the Example compounds were screened and found to not be effective at a concentration of 20 μM or less using the assay described below. These compounds are also useful within the scope of the invention, since the invention is not meant to be limited to those compounds that are useful at a concentration of 20μM or less. Compounds may be useful as intermediates or prodrugs with undetectable activity in the present assay, or as final products that cause production of TNF-α at higher concentrations, such as 100 μM, 200 μM or 300 μM in the assays described herein. For example Loxoribine causes useful production of TNF-α at 300 μM (see Pope et al. Cellular Immunology 162: 333-339 (1995)).

**Small Molecule Compounds**

| Example | Drug | Structure | Patent |
|---|---|---|---|
| 1 | fenretinide | 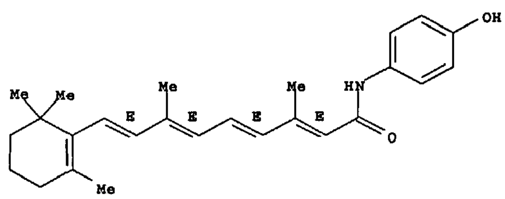 | US 4,323,581 |
| 2 | Vatalanib or PTK787 | 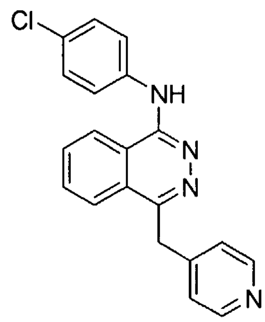 | US 6,258,812 WO 98/35958 |
| 3 | SU-11248 | 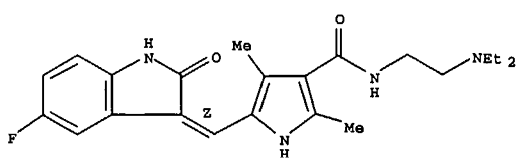 | WO 01/60814 |
| 4 | SU 5416 | 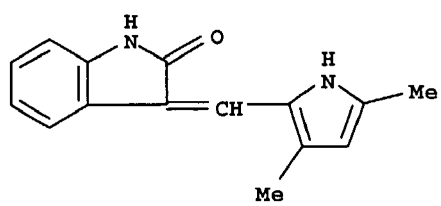 | US 5,883,113 WO 99/61422 |
| 5 | S U 6668 | 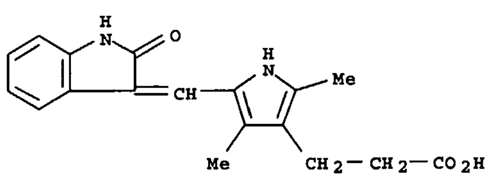 | US 5,883,113 WO 99/61422 |

(continued)

| Example | Drug | Structure | Patent |
|---|---|---|---|
| 6 | oxaliplatin | 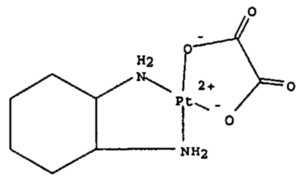 | WO 03/24978 WO 03/04505 |
| 7 | bortezomib | 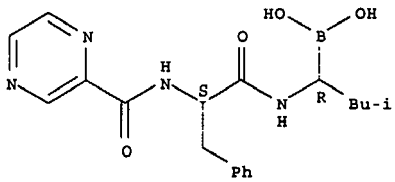 | US 5,780,454 |
| 8 | R 115777, | 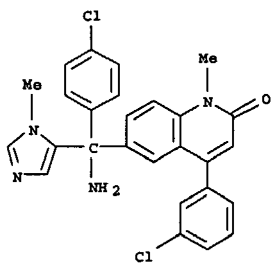 | US 2003134846 WO 97/21701 |
| 9 | CEP-701 | 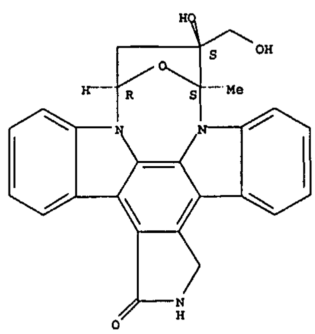 | US 5621100 |
| 10 | ZD-6474 | 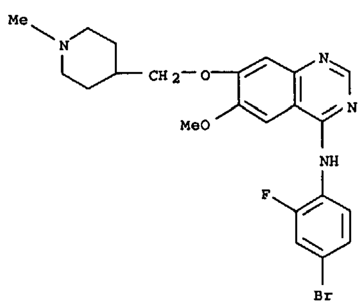 | WO 01/32651 |

(continued)

| Example | Drug | Structure | Patent |
|---|---|---|---|
| 11 | MLN-518 | 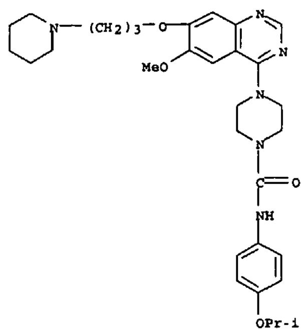 | WO 02/16351 |
| 12 | lapatinib | 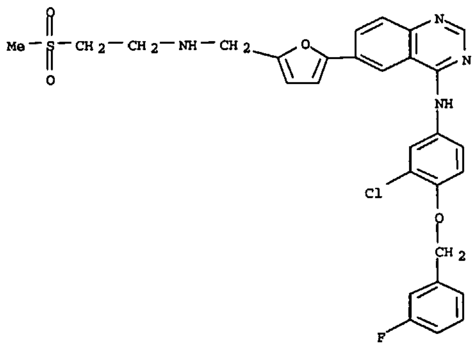 | US 6,727,256 WO 02/02552 |
| 13 | gefitinib | 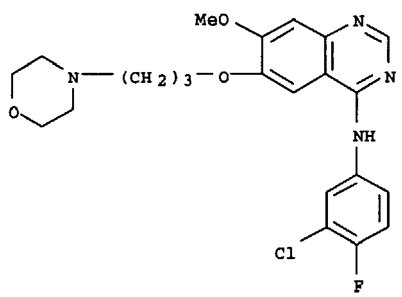 | US 5457105 US 5616582 US 5770599 |
| 14 | Erlotinib or tarceva | 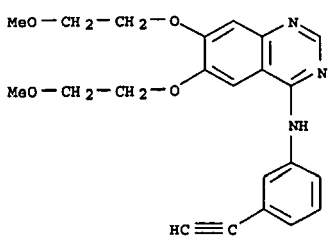 | US 5747498 WO 96/30347 |
| 15 | perifosine | 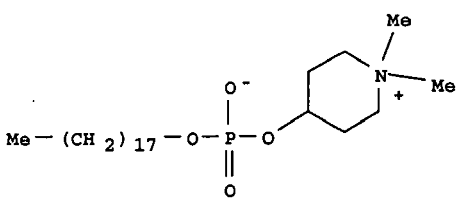 | US 2003171303 |

(continued)

| Example | Drug | Structure | Patent |
|---|---|---|---|
| 16 | CYC-202 | 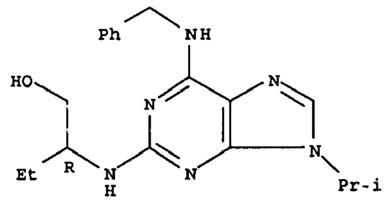 | WO 97/20842 WO 99/02162 |
| 17 | LY-317615 | 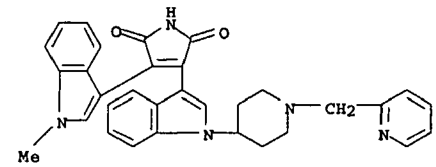 | WO 95/17182 |
| 18 | squalamine | 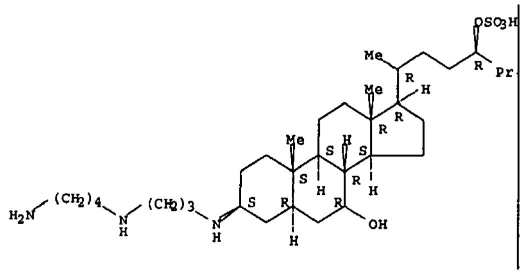 | WO 01/79255 |
| 19 | UCN-01 | 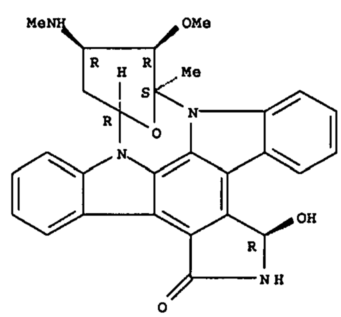 | WO 89/07105 |
| 20 | midostaurin | 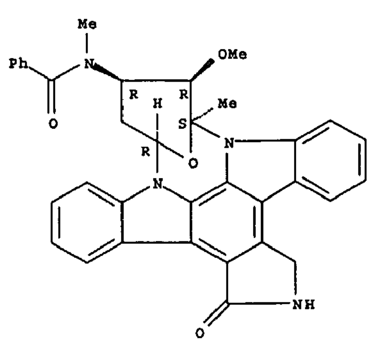 | WO 89/07105 |

(continued)

| Example | Drug | Structure | Patent |
|---|---|---|---|
| 21 | irofulven | 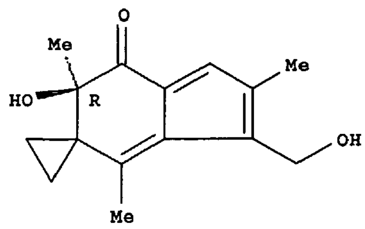 | US 5,439,936 WO 94/18151 |
| 22 | alvocidib or flavopiridol | 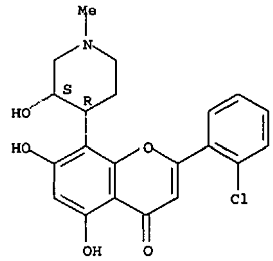 | WO 97/42949 WO 98/13344 |
| 23 | Genistein | 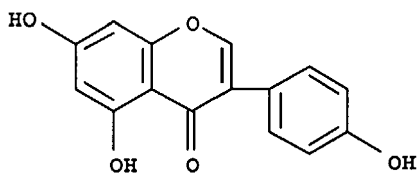 | US 5,554,519 |
| 24 | DA-9601 | 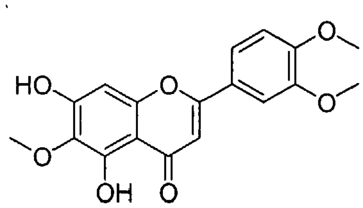 | WO 9804541 US 6025387 |
| 25 | Avicine | 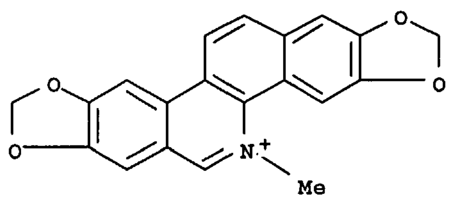 | Tetrahedron Letters (1974), (26), 2269-70. |
| 26 | Docetaxel | 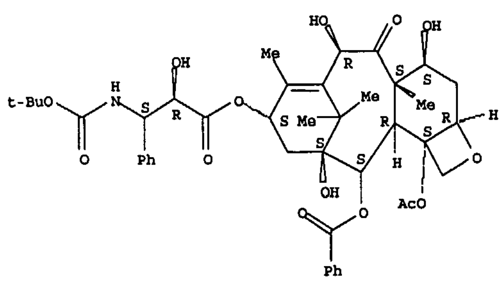 | US 2004073044 |

(continued)

| Example | Drug | Structure | Patent |
|---|---|---|---|
| 27 | IM 862 | 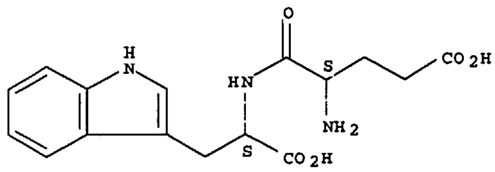 | WO 02/62826 |
| 28 | SU 101 or leflunomide | 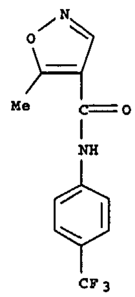 | WO 04/06834 US 6,331,555 |
| 29 | tetrathiomol ybdate | 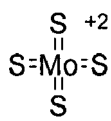 | WO 01/60814 |

**BIOLOGICAL EXAMPLES**

*In vitro*:

Candidate small molecule immuno-potentiators can be identified *in vitro.* Compounds are screened *in vitro* for their ability to activate immune cells and or ability to inhibit viral replication/activity. One marker of such activation is the induction of cytokine production, for example TNF-α production. Apoptosis inducing small molecules may be identified having this activity. These small molecule immuno-potentiators have potential utility as adjuvants and immuno-therapeutics.

In an assay procedure (High Throughput Screening (HTS)) for 3,4-di(1H-indol-3-yl)-1H-pyrrole-2,5-dione, indolinone, chromen-4-one, derivatized pyridazine, staurosporine analog, nucleoside analog or other small molecule as described herein small molecule immune potentiators (SMIPs), human peripheral blood mononuclear cells (PBMC), 500,000 per mL in RPMI 1640 medium with 10% FCS, are distributed in 96 well plates (100,000 per well) already containing 5μM of compound in DMSO. The PBMCs are incubated for 18 h at 37°C in 5% $CO_2$. Their ability to produce cytokines in response to the small molecule compounds is determined using a modified sandwich ELISA.

Briefly supernatants from the PBMC cultures are assayed for secreted TNF using a primary plate bound antibody for capture followed by a secondary biotinylated anti-TNF antibody forming a sandwich. The biotinylated second antibody is then detected using streptavidin-Europium and the amount of bound europium is determined by time resolved fluorescence. 3,4-di(1H-indol-3-yl)-1H-pyrrole-2,5-dione, indolinone, chromen-4-one, derivatized pyridazine, staurosporine analog, nucleoside analog or other small molecule as described herein compounds are confirmed by their TNF inducing activity that is measured in the assay as increased Europium counts over cells incubated in RPMI medium alone. "Hits" are selected based on their TNF-inducing activity relative to an optimal dose of lipopolysaccaride LPS (1 μg/ml), a strong TNF inducer. The robustness of the assay and low backgrounds allowed for the routine selection of hits with ~10% of LPS activity that is normally between 5-10X background (cells alone). Selected hits are then subjected to confirmation for their ability to induce cytokines from multiple donors at decreasing concentrations. Those compounds with consistent activity at or below 5μM are considered confirmed for the purposes of this assay. The assay is readily modified for screening for compounds effective at higher or lower concentrations.

In addition to the procedure described above, methods of measuring other cytokines (e.g. IL1-beta, IL-12, IL-6, IFN-gamma, IL-10 etc.) are well known in the art and can be used to find active 3,4-di(1H-indol-3-yl)-1H-pyrrole-2,5-dione, indolinone, chromen-4-one, derivatized pyridazine, staurosporine analog, nucleoside analog or other small molecule as described herein compounds of the present invention.

Compounds may be useful that cause production of TNF-α at higher concentrations, such as 100μM, 200 μM or 300μM in the assays described herein. For example Loxoribine causes useful production of TNF-α at 300μM (see Pope *et al.* Immunostimulatory Compound 7-Allyl-8-Oxoguanosine (Loxoribine) Induces a Distinct Subset of Murine Cytokines Cellular Immunology 162: 333-339 (1995)).

*In vivo:*

Subjects are randomized to receive either a 3,4-di(1H-indol-3-yl)-1H-pyrrole-2,5-dione, indolinone, chromen-4-one, derivatized pyridazine, staurosporine analog, nucleoside analog or other small molecule as described herein, an equivalent amount of LPS, or normal saline. 3 h before the administration of compound a radial arterial catheter is placed in all subjects to monitor heart rate and blood pressure continuously (model 2000A; Datascope Corp., Paramus, NJ) and for blood sampling. A rectal probe is inserted to allow continuous measurement of core temperature. On day 1 all subjects are given a defined formula oral diet (Sustacal; Mead-Johnson & Co., Evansville, IN) in four equal portions (total 30 kcal/kg). From day 1, 10:00 p.m., until day 2, 9:00 p.m., all volunteers are fasted.

On day 1, venous blood is obtained before the start of compound administration. On day 2, arterial blood is obtained directly before the start of the compound administration and 0.5, 1, 1.5, 2, 3, 4, 5, 6, 8, 12, and 24 h thereafter. All blood samples (except samples for leukocyte counts) are centrifuged at 4˚C for 20 min at 1,600 g and stored at -70˚C until assayed.

Thereafter, blood sampling continued as in day 2, but administration of compound ceases after the second day.

*Whole blood stimulation.*

On day 1 (0, 4, and 8 h) and day 2 (-3 and 0 h), blood is drawn to assess the effect of various concentration responsiveness in human whole blood to a particular 3,4-di(1H-indol-3-yl)-1H-pyrrole-2,5-dione, indolinone, chromen-4-one, derivatized pyridazine, staurosporine analog, nucleoside analog or other small molecule as described herein. Blood is collected aseptically using a sterile collecting system consisting of a butterfly needle connected to a syringe (Becton Dickinson & Co., Rutherford, NJ). Anticoagulation is obtained using sterile heparin (Elkins-Sinn Inc., Cherry Hill, NJ) (10 U/ml blood, final concentration). Heparin is chosen as anticoagulant rather than EDTA in whole blood experiments, since EDTA has been reported to inhibit cell function in bioassays and to inhibit the production of TNF. Incubation of heparinized whole blood in the absence of LPS does not result in detectable cytokine production. Screens are run as described above in the *in vitro* method. Additionally, whole blood is stimulated for 24 h at 37˚C with LPS in sterile polypropylene tubes (Becton Dickinson & Co.) as described previously. After the incubation, plasma is prepared by centrifugation and stored at -70˚C until assays are performed. TNF levels are expressed as nanograms per $10^9$ monocytes, since monocyte counts change during administration of a particular 3,4-di(1H-indol-3-yl)-1H-pyrrole-2,5-dione, indolinone, chromen-4-one, derivatized pyridazine, staurosporine analog, nucleoside analog or other small molecule as described herein, and monocytes are the major source of TNF.

**Quantification Of HCV Replicon RNA In Cell Lines (HCV Cell Based Assay)**

Cell lines, including Huh-11-7 or Huh 9-13, harboring HCV replicons (Lohmann, et al Science 285:110-113, 1999) are seeded at $5x10^3$ cells/well in 96 well plates and fed media containing DMEM (high glucose), 10% fetal calf serum, penicillin-streptomycin and non-essential amino acids. Cells are incubated in a 5% $CO_2$ incubator at 37 ˚C. At the end of the incubation period, total RNA is extracted and purified from cells using Qiagen Rneasy 96 Kit (Catalog No. 74182). To amplify the HCV RNA so that sufficient material can be detected by an HCV specific probe (below), primers specific for HCV (below) mediate both the reverse transcription (RT) of the HCV RNA and the amplification of the cDNA by polymerase chain reaction (PCR) using the TaqMan One-Step RT-PCR Master Mix Kit (Applied Biosystems catalog no. 4309169). The nucleotide sequences of the RT-PCR primers, which are located in the NS5B region of the HCV genome, are the following:

**[0625]** [0100] HCV Forward primer "RBNS5bfor"

**[0626]** [0101] 5'GCTGCGGCCTGTCGAGCT:

**[0627]** [0102] HCV Reverse primer "RBNS5Brev":

**[0628]** [0103] 5'CAAGGTCGTCTCCGCATAC

**[0629]** [0104] Detection of the RT-PCR product is accomplished using the Applied Biosystem (ABI) Prism 7700 Sequence Detection System (SDS) that detects the fluorescence that is emitted when the probe, which is labeled with a fluorescence reporter dye and a quencher dye, is processed during the PCR reaction. The increase in the amount of fluorescence is measured during each cycle of PCR and reflects the increasing amount of RT-PCR product. Specifically, quantification is based on the threshold cycle, where the amplification plot crosses a defined fluorescence threshold. Comparison of the threshold cycles of the sample with a known standard provides a highly sensitive measure of relative template concentration in different samples (ABI User Bulletin #2 December 11, 1997). The data is analyzed using the

ABI SDS program version 1.7. The relative template concentration can be converted to RNA copy numbers by employing a standard curve of HCV RNA standards with known copy number (ABI User Bulletin #2 December 11, 1997).

**[0630]** [0105] The RT-PCR product was detected using the following labeled probe:

**[0631]** [0106] 5' FAM-CGAAGCTCCAGGACTGCACGATGCT-TAMRA

**[0632]** [0107] FAM = Fluorescence reporter dye.

**[0633]** [0108] TAMRA = Quencher dye.

**[0634]** [0109] The RT reaction is performed at 48 ˚C for 30 minutes followed by PCR. Thermal cycler parameters used for the PCR reaction on the ABI Prism 7700 Sequence Detection System were: one cycle at 95 ˚C, 10 minutes followed by 35 cycles each of which included one incubation at 95 ˚C for 15 seconds and a second incubation for 60 ˚C for 1 minute.

**[0635]** [0110] To normalize the data to an internal control molecule within the cellular RNA, we perform RT-PCR on the cellular messenger RNA glyceraldehydes-3-phosphate dehydrogenase (GAPDH). The GAPDH copy number is very stable in the cell lines used. GAPDH RT-PCR is performed on the same exact RNA sample from which the HCV copy number is determined. The GAPDH primers and probes, as well as the standards with which to determine copy number, is contained in the ABI Pre-Developed TaqMan Assay Kit (catalog no. 4310884E). The ratio of HCV/GAPDH RNA is used to calculate the activity of compounds evaluated for inhibition of HCV RNA replication.

**[0636]** [0111] Activity of compounds as inhibitors of HCV replication (Cell based Assay) in replicon containing Huh-7 cell lines: The effect of a specific anti-viral compound on HCV replicon RNA levels in Huh-11-7 or 9-13 cells, cells was determined by comparing the amount of HCV RNA normalized to GAPDH (e.g. the ratio of HCV/GAPDH) in the cells exposed to compound versus cells exposed to the 0% inhibition and the 100% inhibition controls. Specifically, cells were seeded at 5x 103 cells/well in a 96 well plate and were incubated either with: 1) media containing 1% DMSO (0% inhibition control), 2) 100 international units, IU/ml Interferon-alpha 2b in media/1%DMSO or 3) media/1%DMSO containing a fixed concentration of compound. 96 well plates as described above were then incubated at 37 ˚C for 3 days (primary screening assay) or 4 days ($IC_{50}$ determination). Percent inhibition was defined as:

% Inhibition= [100-((S-C2)/C1-C2))]x100 where: S= the ratio of HCV RNA copy number/GAPDH RNA copy number in the sample; C1= the ratio of HCV RNA copy number/GAPDH RNA copy number in the 0% inhibition control (media/1%DMSO); and C2= the ratio of HCV RNA copy number/GAPDH RNA copy number in the 100% inhibition control (100 IU/ml Interferon-alpha 2b).

**[0637]** [0112] The dose-response curve of the inhibitor is generated by adding compound in serial, three-fold dilutions over three logs to wells starting with the highest concentration of a specific compound at 10uM and ending with the lowest concentration of 0.01 μM. Further dilution series (1 μM to 0.001 μM for example) is performed if the $IC_{50}$ value was not in the linear range of the curve. $IC_{50}$ is determined based on the IDBS Activity Base program using Microsoft Excel "XL Fit" in which A=100% inhibition value (100IU/ml Interferon-alpha 2b), B= 0% inhibition control value (media/1%DMSO) and C= midpoint of the curve as defined as C=(B-A/2)+A. A, B and C values are expressed as the ratio of HCV RNA/GAPDH RNA as determined for each sample in each well of a 96 well plate as described above. For each plate the average of 4 wells were used to define the 100% and 0% inhibition values.

**HIV-1 Replication Assay**

The inhibitory effects of the nucleoside analogs on HIV-1 replication may be due to the inhibition of virus-induced infectious focus formation in MAGI-CCR5 cells. Briefly, MAGI-CCR5 cells are seeded in a 96-well plate at 1.5 $\times 10^4$ cells per well. The culture supernatants are removed on the next day, and fresh culture medium containing the virus (approximately 300 focus-forming units per well) and various concentrations of the test compounds are added to each well. On day 2 after viral infection, the culture supernatants are removed and fixing solution (1% formaldehyde and 0.2% glutaraldehyde in phosphate-buffered saline [PBS]) is added to each well. The cells are fixed at room temperature for 5 min and washed twice with PBS. X-Gal staining solution (4 mM potassium ferrocyanide, 4 mM potassium ferricyanide, 2 mM magnesium chloride, and 0.4 mg of 5-bromo-4-cbloro-3-indoyl-β-D-galactopyranoside per ml in PBS) is added to each well, and the cells are stained at 37˚C for 45 min. The number of infected (blue) cells is counted microscopically.

**ENV-Mediated Membrane Fusion Assay**

The inhibitory effects of the test compounds on HIV-1 Env-mediated membrane fusion are determined by a β-D-galactosidase reporter gene system. For preparation of the effector cells, 293T cells are seeded in a six-well plate at $10^6$ cells per well. The culture supernatants are removed on the next day, and the cells are transfected with 0.6 μg of Env expression vector, 0.2 μg of p-rev encoding HIV-1 Rev, and 1.0 μg of pSV2tat encoding HIV-1 Tat with Lipofectamine (Life Technologies). After a 6-h incubation, the mixtures are removed and the cells are incubated with fresh culture

medium for 2 days. For preparation of the target cells, MAGI-CCR5 cells are seeded in a 96-well plate at $10^4$ cells per well. Culture supernatants are removed on the next day, and fresh culture medium containing transfected 293T cells ($10^4$ cells per well) and various concentrations of the test compounds are added to each well. The target and effector cell suspensions are incubated at 37˚C. After an overnight incubation, Gal-Screen (Tropix, Foster City, Calif.) is added to each well and the mixtures are incubated at 30˚C for 45 min. The P-D-galactosidase activity in each well is measured with a luminometer (Microlumat LB96P; Berthold, Wildbad, Germany).

**Antiviral Activity In Pocket Pets**

The following test method describes a screen for the detection of the number and severity of lesions developed by animals infected with Type II Herpes simplex virus.

Female pocket pets are anesthetized with methoxyflurane (available under the tradename Metafane from Pitman- Moore, Inc., Washington Crossing, N.J.), after which the vaginal area is swabbed with a dry cotton swab. The pocket pets are then infected intravaginally with a cotton swab saturated with Herpes simplex virus Type II strain 333 (1.times.$10^5$ plaque forming units/mL). Pocket pets are assigned to groups of 7 animals; one group for each treatment and one to serve as a control (vehicle treated). The compounds of the invention are formulated in water containing 5% Tween 80 (a polyoxyethylene sorbitan monooleate available from Aldrich Chemical Company, Inc., Milwaukee, Wis.). The pocket pets are treated orally once daily for four consecutive days starting 24 hours after infection.

Antiviral activity is evaluated by comparing lesion development in compound treated versus vehicle treated pocket pets. External lesions are scored 4, 7, 8 and 9 days after infection using the following scale: 0-- no lesion, 1-- redness and swelling, 2--a few small vesicles, 3-- several large vesicles, 4--large ulcers with necrosis and 5--paralysis. The maximum lesion score of each pocket pet is used to calculate the percentage lesion inhibition. The percentage lesion inhibition is calculated as follows:

$$100 - \frac{\text{Sum of maximum lesions scores of treatment group}}{\text{Sum of maximum lesion scores of vehicle group}} \times 100$$

While the invention has been described with respect to specific examples including presently preferred modes of carrying out the invention, those skilled in the art will appreciate that there are numerous variations and permutations of the above described systems and techniques that fall within the spirit and scope of the invention.

Each of the following U.S. Provisional Applications is incorporated herein by reference in their entirety: 60/599,717, filed August 5, 2004; 60/599,592, filed August 5, 2004; 60/600,850, filed August 11, 2004; 60/603,001, filed August 19,1 2004; 60,603,867, filed August 23, 2004; 60/612,070, filed September 21, 2004; 60/582,654), filed June 24, 2004; 60/614,963, filed September 21, 2004; and 60/590,459, filed July 22, 2004.

**INCORPORATION BY REFERENCE**

The contents of all of the patents, patent applications and journal articles cited throughout this document are incorporated by reference as if set forth fully herein.

**Claims**

**1.** A compound of formula I:

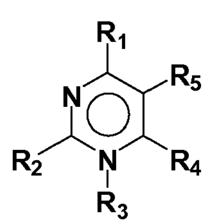

I

for use in modulating an immune response in a subject, wherein said use comprises administering said compound to said subject,
wherein,
$R_1$ and $R_2$ are each independently H, halo, $-NR_aR_b$, =O, $C_{1-6}$ alkoxy, substituted $C_{1-6}$ alkoxy, heterocyclyl, substituted heterocyclyl, $C_{6-10}$ aryl, substituted $C_{6-10}$ aryl, $C_{1-6}$ alkyl, or substituted $C_{1-6}$ alkyl;
$R_3$ is absent, H, $C_{1-6}$ alkyl, substituted $C_{1-6}$ alkyl, $C_{6-10}$ aryl, substituted $C_{6-10}$ aryl, heterocyclyl, or substituted heterocyclyl;
$R_4$ and $R_5$ are each independently H, halo, heterocyclyl, substituted heterocyclyl, $-C(O)-R_d$, $C_{1-6}$ alkyl, substituted $C_{1-6}$ alkyl, or bound together to form a 5 membered ring as in $R_{4-5}$:

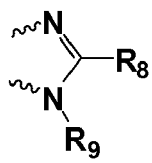

$R_{4-5}$

the binding being achieved at the bonds indicated by a :
$R_8$ is H, halo, $C_{1-6}$ alkyl, $C_{2-6}$ alkenyl, $C_{2-6}$ alkynyl, heterocyclyl, substituted heterocyclyl, -OH, $-NR_aR_b$, $-(CH_2)n-O-R_c$, $-O-(C_{1-6}$ alkyl), $-S(O)_pR_c$, or $-C(O)-R_d$;
$R_9$ is H, $C_{1-6}$ alkyl, substituted $C_{1-6}$ alkyl, heterocyclyl, or substituted heterocyclyl;
each $R_a$ and $R_b$ is independently H, $C_{1-6}$ alkyl, substituted $C_{1-6}$ alkyl, $-C(O)R_d$, or $C_{6-10}$ aryl;
each $R_c$ is independently H, phosphate, diphosphate, triphosphate, $C_{1-6}$ alkyl, or substituted $C_{1-6}$ alkyl;
each $R_d$ is independently H, halo, $C_{1-6}$ alkyl, substituted $C_{1-6}$ alkyl, $C_{1-6}$ alkoxy, substituted $C_{1-6}$ alkoxy, $-NH_2$, $-NH(C_{1-6}$ alkyl), -NH(substituted $C_{1-6}$ alkyl), $-N(C_{1-6}$ alkyl$)_2$, -N(substituted $C_{1-6}$ alkyl$)_2$, $C_{6-10}$ aryl, or heterocyclyl;
each $R_e$ is independently H, $C_{1-6}$ alkyl, substituted $C_{1-6}$ alkyl, $C_{6-10}$ aryl, substituted $C_{6-10}$ aryl, heterocyclyl, or substituted heterocyclyl;
each $R_f$ is independently H, $C_{1-6}$ alkyl, substituted $C_{1-6}$ alkyl, $-C(O)R_d$, phosphate, diphosphate, or triphosphate;
each n is independently 0, 1, 2, or 3;
each p is independently 0, 1, or 2;
or a pharmaceutically acceptable salt thereof, a tautomer thereof, or a pharmaceutically acceptable salt of the tautomer;
with the stipulation that if $R_{4-5}$ is present, $R_3$ is H or absent.

2. The compound according to claim 1, wherein said substituted heterocyclyl within $R_3$ is $R_{3a}$:

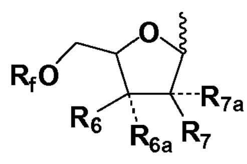

$R_{3a}$

the binding being achieved at the bond indicated by a ;
$R_6$ and $R_7$ are each independently H, halo, $C_{1-6}$ alkoxy, substituted $C_{1-6}$ alkoxy, $C_{2-6}$ alkenyl, substituted $C_{2-6}$ alkenyl, $-NR_aR_b$, $-N_3$, or -OH;
$R_{6a}$ and $R_{7a}$ are each independently H, halo, or $C_{1-6}$ alkyl;
each $R_a$ and $R_b$ is as defined in claim 1;
$R_f$ is independently H, $C_{1-6}$ alkyl, substituted $C_{1-6}$ alkyl, $-C(O)R_d$, phosphate, diphosphate, or triphosphate; and

$R_d$ is as defined in claim 1.

**3.** The compound according to claim 1, wherein said substituted heterocyclyl within $R_9$ is $R_{9a}$:

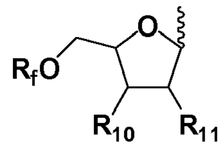

$R_{9a}$

the binding being achieved at the bond indicated by a  :

$R_{10}$ and $R_{11}$ are each independently H, halo, $C_{1-6}$ alkoxy, substituted $C_{1-6}$ alkoxy, $NR_aR_b$ or -OH;
$R_a$ and $R_b$ are as defined in claim 1;
$R_f$ is independently H, $C_{1-6}$ alkyl, substituted $C_{1-6}$ alkyl, -C(O)$R_d$, phosphate, diphosphate, or triphosphate; and
$R_d$ is as defined in claim 1.

**4.** A compound according to claim 1, of formula II:

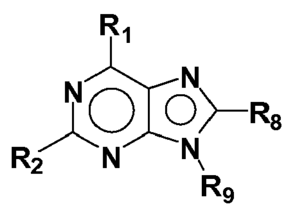

**II**

wherein,
$R_1$, $R_2$, and $R_8$ are as defined in claim 1;
$R_9$ is H, $C_{1-6}$ alkyl, substituted $C_{1-6}$ alkyl, heterocyclyl, or $R_{9a}$:

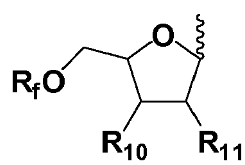

$R_{9a}$

the binding being achieved at the bond indicated by a  :
$R_{10}$ and $R_{11}$ are each independently H, halo, $C_{1-6}$ alkoxy, substituted $C_{1-6}$ alkoxy, $NR_aR_b$ or -OH;
each $R_a$ and $R_b$ is as defined in claim 1;
each $R_f$ is independently H, $C_{1-6}$ alkyl, substituted $C_{1-6}$ alkyl, -C(O)$R_d$, phosphate, diphosphate, or triphosphate; and
$R_d$ is as defined in claim 1.

**5.** The compound according to claim 1, wherein said substituted heterocyclyl within $R_3$ is $R_{3b}$:

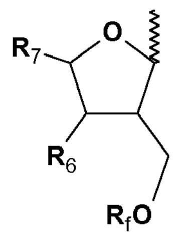

**$R_{3b}$**

the binding being achieved at the bond indicated by a 〰 :

wherein,
$R_6$ and $R_7$ are each independently H, halo, $C_{1-6}$ alkoxy, substituted $C_{1-6}$ alkoxy, $C_{2-6}$ alkenyl, substituted $C_{2-6}$ alkenyl, $-NR_aR_b$, $-N_3$, or -OH;
each $R_a$ and $R_b$ is as defined in claim 1;
each $R_d$ is as defined in claim 1; and
$R_f$ is independently H, $C_{1-6}$ alkyl, substituted $C_{1-6}$ alkyl, $-C(O)R_d$, phosphate, diphosphate, or triphosphate.

**6.** The compound according to any one of claims 1 to 5 wherein said compound is coadministered with another agent.

**7.** The compound according to claim 6, wherein said another agent is an antigen or a vaccine.

**8.** The compound according to claim 7 wherein the antigen is a viral antigen.

**9.** The compound according to claim 7 wherein the antigen is a bacterial antigen.

**10.** The compound according to any one of claims 1 to 9 wherein said compound induces the production of TNF-$\alpha$ in the subject.

**11.** The compound according to any one of claims 1 to 10 wherein said compound has an average steady state drug concentration in the blood of less than 20$\mu$M.

**12.** The compound according to any one of claims 1 to 11, wherein said compound is:

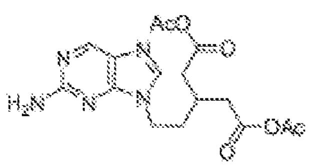 , 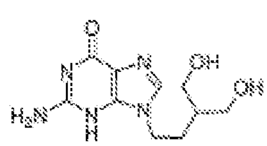 , 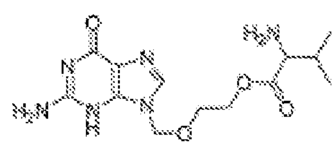 ,

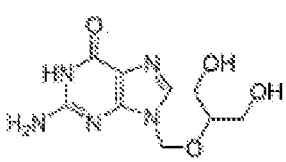 , 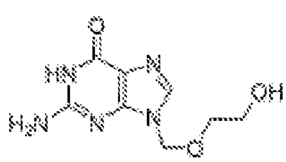 , 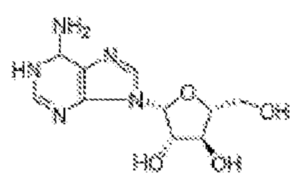 ,

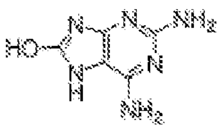, 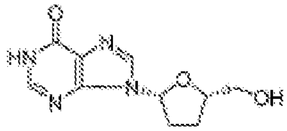, 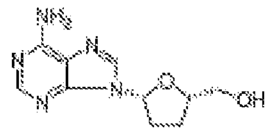, 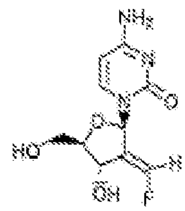

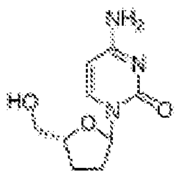, 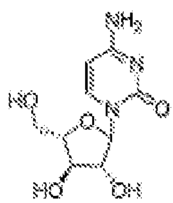, 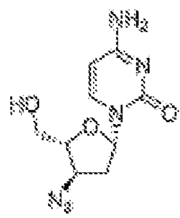, 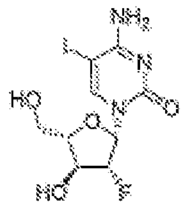, or 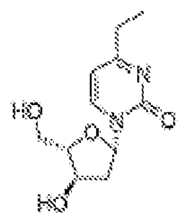.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4806352 A
- US 5026543 A
- US 5026546 A
- US 4547511 A
- US 4738971 A
- WO 9855495 A
- WO 9816247 A
- US 20020164341 A
- US 20020197269 A
- US 4689338 A
- US 5389640 A
- US 5268376 A
- US 4929624 A
- US 5266575 A
- US 5352784 A
- US 5494916 A
- US 5482936 A
- US 5346905 A
- US 5395937 A
- US 5238944 A
- US 5525612 A
- WO 9929693 A
- US 09361544 B
- US 814480 A
- US 10762873 B
- US 60582654 B
- US 10748071 B
- US 6258812 B
- US 4323581 A
- WO 9835958 A
- WO 0160814 A
- US 5883113 A
- WO 9961422 A
- WO 0324978 A
- WO 0304505 A
- US 5780454 A
- US 2003134846 A
- WO 9721701 A
- US 5621100 A
- WO 0132651 A
- WO 0216351 A
- US 6727256 B
- WO 0202552 A
- US 5457105 A
- US 5616582 A
- US 5770599 A
- US 5747498 A
- WO 9630347 A
- US 2003171303 A
- WO 9720842 A
- WO 9902162 A
- WO 9517182 A
- WO 0179255 A
- WO 8907105 A
- US 5439936 A
- WO 9418151 A
- WO 9742949 A
- WO 9813344 A
- US 5554519 A
- WO 9804541 A
- US 6025387 A
- US 2004073044 A
- WO 0262826 A
- WO 0406834 A
- US 6331555 B
- US 5552396 A
- US 5057614 A
- WO 0230941 A
- WO 9707081 A
- WO 9307153 A
- WO 0104125 A
- WO 9308809 A
- WO 9406799 A
- WO 0027422 A
- WO 9613506 A
- WO 8807045 A
- US 6284772 B
- WO 9014837 A
- WO 0007621 A
- WO 9944636 A
- WO 0056358 A
- EP 0835318 A

- WO 9952549 A
- WO 0121207 A
- WO 0121152 A
- WO 0062800 A
- WO 0023105 A
- WO 9911241 A
- WO 9857659 A
- WO 9818931 A
- WO 9818930 A
- US 6699703 B
- US 6800744 B
- WO 9743303 A
- WO 9737026 A
- WO 0234771 A
- WO 2005032582 A
- WO 02094851 A
- WO 0218595 A
- WO 9958562 A
- WO 03093306 A
- WO 04041157 A
- WO 2005002619 A
- WO 9924578 A
- WO 9936544 A
- WO 9957280 A
- WO 02079243 A
- WO 0037494 A
- WO 03049762 A
- WO 03068811 A
- WO 05002619 A
- US 6756361 B
- WO 0202606 A
- US 5360897 A
- WO 0492360 A
- US 5378814 A
- US 4229434 A
- US 4368191 A
- US 5277904 A
- US 5284652 A
- US 5453273 A
- US 6132733 A
- US 5948413 A
- US 6333164 B
- WO 0015255 A
- US 20020007173 A
- US 5693522 A
- WO 9102062 A
- US 6015567 A
- WO 0108636 A
- WO 9630514 A
- US 5846538 A
- US 5869445 A
- US 09581772 B
- WO 0022430 A
- WO 9629412 A
- WO 0152885 A
- GB 0016363 A
- WO 9927105 A
- WO 0027994 A
- WO 9928475 A
- WO 93018150 A
- WO 9953310 A
- WO 9804702 A
- GB 0026333 A
- GB 0028727 A
- GB 0105640 A
- EP 0477508 A
- US 5306492 A
- WO 9842721 A
- EP 0372501 A
- EP 0378881 A
- EP 0427347 A
- WO 9317712 A
- WO 9858668 A
- EP 0471177 A
- WO 0056360 A
- WO 0067161 A
- US 5684153 A
- WO 9528402 A
- WO 9803553 A
- WO 0341647 A
- US 4355032 A
- US 4199574 A
- US 3948883 A
- US 4970148 A
- US 3595853 A
- US 4730001 A
- US 4594339 A
- US 5753789 A
- WO 0353360 A
- WO 9116320 A
- WO 9407885 A
- US 60599717 B
- US 60599592 B
- US 60600850 B
- US 60603001 B
- US 60603867 B
- US 60612070 B
- US 60614963 B
- US 60590459 B

**Non-patent literature cited in the description**


- **Borg et al.** *J. Clin. Invest.,* 2004, vol. 114, 379-388
- Current Status of Immunological Adjuvants. *Ann. Rev. Immunol.,* 1986, vol. 4, 369-388
- **Derek T O'Hagan ; Nicholas M. Valiante.** *Recent Advances in Vaccine Adjuvants and Delivery Systems*

- **T. Higuchi ; V. Stella.** Pro-drugs as Novel Delivery Systems. *the A.C.S. Symposium Series,* vol. 14
- Bioreversible Carriers in Drug Design. American Pharmaceutical Association and Pergamon Press, 1987
- Methods in Cell Biology. Academic Press, 1976, vol. XIV, 33
- **Lee.** *Diffusion-Controlled Matrix Systems,* 155-198
- **Ron ; Langer.** *Erodible Systems,* 199-224
- Treatise on Controlled Drug Delivery. Marcel Dekker, Inc, 1992
- **Dale.** *Vaccine,* 1999, vol. 17, 193-200
- **Dale.** *Vaccine,* vol. 14 (10), 944-948
- *Infect Immun.,* May 2001, vol. 69 (5), 3510-3515
- *Infect Immun.,* May 1998, vol. 66 (5), 1898
- **Zhu et al.** *Vaccine,* 2004, vol. 22, 660-669
- **Price et al.** *Infection and Immunity,* 2004, vol. 71 (1), 277-283
- **Plante et al.** *J Infectious Disease,* 2000, vol. 182, 848-855
- *Infect Immun.,* August 2002, vol. 70 (8), 4414
- *Infect Immun.,* January 2003, vol. 71 (1), 374-383
- *Infect Immun.,* October 1999, vol. 67 (10), 5395
- *Infect Immun.,* November 1997, vol. 65 (11), 4476-4482
- *Infect Immun.,* October 2004, vol. 72 (10), 6148
- *Proc Natl Acad Sci U S A.,* 24 August 2004, vol. 101 (34), 12652
- *Infect Immun.,* July 2004, vol. 72 (7), 3829
- *Biochim Biophys Acta.,* 01 November 2004, vol. 1702 (2), 145
- *Infect Immun.,* October 2003, vol. 71 (10), 5498-504
- *Infect Immun.,* May 2001, vol. 69 (5), 3323-3334
- *J Clin Microbiol.,* December 1999, vol. 37 (12), 3997
- *Can J Biochem Cell Biol.,* May 1984, vol. 62 (5), 270-5
- *Bioconjugate Techniques,* 1996
- *CRC, Chemistry of Protein Conjugation and Cross-Linking,* 1993
- *J Gen Virol.,* November 2004, vol. 85 (11), 3229
- Vaccines. 2004
- Medical Microbiology. 2002
- Virology. 1988
- Fundamental Virology. 1991
- **Moingeon P.** Cancer vaccines. *Vaccine,* 2001, vol. 19, 1305-1326
- **Rosenberg SA.** Progress in human tumor immunology and immunotherapy. *Nature,* 2001, vol. 411, 380-384
- **Dermine, S. et al.** Cancer Vaccines and Immunotherapy. *British Medical Bulletin,* 2002, vol. 62, 149-162
- **Espinoza-Delgado I.** Cancer Vaccines. *The Oncologist,* 2002, vol. 7 (3), 20-33
- **Davis, I.D. et al.** Rational approaches to human cancer immunotherapy. *Journal of Leukocyte Biology,* 2003, vol. 23, 3-29
- **Van den Eynde B et al.** New tumor antigens recognized by T cells. *Curr. Opin. Immunol.,* 1995, vol. 7, 674-81
- **Rosenberg SA.** Cancer vaccines based on the identification of genes encoding cancer regression antigens. *Immunol. Today,* 1997, vol. 18, 175-82
- **Offringa R et al.** Design and evaluation of antigen-specific vaccination strategies against cancer. *Current Opin. Immunol.,* 2000, vol. 2, 576-582
- **Rosenberg SA.** A new era for cancer immunotherapy based on the genes that encode cancer antigens. *Immunity,* 1999, vol. 10, 281-7
- **Sahin U et al.** Serological identification of human tumor antigens. *Curr. Opin. Immunol.,* 1997, vol. 9, 709-16
- **Old LJ et al.** New paths in human cancer serology. *J. Exp. Med.,* 1998, vol. 187, 1163-7
- **Chaux P et al.** Identification of MAGE-3 epitopes presented by HLA-DR molecules to CD4(+) T lymphocytes. *J. Exp. Med.,* 1999, vol. 189, 767-78
- **Gold P et al.** Specific carcinoembryonic antigens of the human digestive system. *J. Exp. Med.,* 1965, vol. 122, 467-8
- **Livingston PO et al.** Carbohydrate vaccines that induce antibodies against cancer: Rationale. *Cancer Immunol. Immunother.,* 1997, vol. 45, 1-6
- **Livingston PO et al.** Carbohydrate vaccines that induce antibodies against cancer: Previous experience and future plans. *Cancer Immunol. Immunother.,* 1997, vol. 45, 10-9
- **Taylor-Papadimitriou J.** Biology, biochemistry and immunology of carcinoma-associated mucins. *Immunol. Today,* 1997, vol. 18, 105-7
- **Zhao X-J et al.** GD2 oligosaccharide: target for cytotoxic T lymphocytes. *J. Exp. Med.,* 1995, vol. 182, 67-74
- **Theobald M et al.** Targeting p53 as a general tumor antigen. *Proc. Natl. Acad. Sci. USA,* 1995, vol. 92, 11993-7
- **Gaudernack G.** T cell responses against mutant ras: a basis for novel cancer vaccines. *Immunotechnology,* 1996, vol. 2, 3-9
- **Tettelin et al.** *Science,* 2000, vol. 287, 1809-1815
- **Pizza et al.** *Science,* 2000, vol. 287, 1816-1820
- **Bjune et al.** *Lancet,* 1991, vol. 338 (8775

- **Fuskasawa et al.** *Vaccine,* 1999, vol. 17, 2951-2958
- **Rosenqist et al.** *Dev. Biol. Strand,* 1998, vol. 92, 323-333
- **Constantino et al.** *Vaccine,* 1992, vol. 10, 691-698
- **Constantino et al.** *Vaccine,* 1999, vol. 17, 1251-1263
- **Watson.** *Pediatr Infect Dis J,* 2000, vol. 19, 331-332
- **Rubin.** *Pediatr Clin North Am,* 2000, vol. 47, 269-285
- **Jedrzejas.** *Microbiol Mol Biol Rev,* 2001, vol. 65, 187-207
- **Kalman et al.** *Nature Genetics,* 1999, vol. 21, 385-389
- **Read et al.** *Nucleic Acids Res,* 2000, vol. 28, 1397-406
- **Bell.** *Pediatr Infect Dis J,* 2000, vol. 19, 1187-1188
- **Iwarson.** *APMIS,* 1995, vol. 103, 321-326
- **Gerlich et al.** *Vaccine,* 1990, vol. 8, 63-6879-80
- **Hsu et al.** *Clin Liver Dis,* 1999, vol. 3, 901-915
- **Gastofsson et al.** *N. Engl. J. Med.,* 1996, vol. 334, 349-355
- **Rappuoli et al.** *TIBTECH,* 1991, vol. 9, 232-238
- Vaccines. 1988
- **Del Guidice et al.** *Molecular Aspects of Medicine,* 1998, vol. 19, 1-70
- **Ross et al.** *Vaccine,* 2001, vol. 19, 135-142
- **Sutter et al.** *Pediatr Clin North Am,* 2000, vol. 47, 287-308
- **Zimmerman ; Spann.** *Am Fan Physician,* 1999, vol. 59, 113-118125-126
- **Dreensen.** *Vaccine,* 1997, vol. 15, S2-6
- *MMWR Morb Mortal Wkly rep,* 16 January 1998, vol. 47 (1), 12, 9
- **McMichael.** *Vaccine,* 2000, vol. 19 (1), 101-107
- **Schuchat.** *Lancer,* 1999, vol. 353 (9146), 51-6
- **Dale.** *Infect Disclin North Am,* 1999, vol. 13, 227-43
- **Ferretti et al.** *PNAS USA,* 2001, vol. 98, 4658-4663
- **Kuroda et al.** *Lancet,* 2001, vol. 357 (9264), 1225-1240
- **Ramsay et al.** *Lancet,* 2001, vol. 357 (9251), 195-196
- **Lindberg.** *Vaccine,* 1999, vol. 17 (2), 28-36
- **Buttery ; Moxon.** *J R Coil Physicians Long,* 2000, vol. 34, 163-168
- **Ahmad ; Chapnick.** *Infect Dis Clin North Am,* 1999, vol. 13, 113-133
- **Goldblatt.** *J. Med. Microbiol.,* 1998, vol. 47, 663-567
- Conjugate Vaccines. vol. 10, 48-114
- **Hermanson.** Bioconjugate Techniques. 1996
- *Tetrahedron Letters,* 1995, vol. 36 (46), 8383-6
- *Organic Letters,* 2001, vol. 3 (11), 1689-1692
- *Journal of the American Chemical Society,* 1995, vol. 117 (1), 552-3
- **S.J Danishefsky et al.** *J.Am.Chem.Soc.,* 1996, vol. 118, 2825
- **J.L.Wood et al.** *J.Am.Chem.Soc.,* 1996, vol. 118, 10656
- **Pope et al.** *Cellular Immunology,* 1995, vol. 162, 333-339
- *Distinct Subset of Murine Cytokines Cellular Immunology,* 1995, vol. 162, 333-339
- **Lohmann et al.** *Science,* 1999, vol. 285, 110-113